# EUROPEAN PATENT APPLICATION

(11) **EP 4 656 629 A1**
(43) Date of publication of application: **03.12.2025**
(21) Application number: 24747440.6
(22) Date of filing: 24.01.2024
(51) Int. Cl.: C07D 209/08, A61K 31/404, A61K 31/4439, C07D 405/12, C07D 401/12

(54) **INDOLE DERIVATIVE WITH SUBSTITUENT AND PHARMACEUTICAL USE**

(30) Priority: 25.01.2023 KR 20230009549
(71) Applicant: MitoImmune Therapeutics Inc., Seoul 06123 (KR)
(72) Inventor: KIM, Soon Ha, Seoul 06123 (KR); CHANG, Hye Kyung, Seoul 06123 (KR); SEO, Mooyoung, Seoul 06123 (KR); YANG, Jeong-Hee, Seoul 06123 (KR); PARK, Hyunjun, Seoul 06123 (KR); PARK, Jeong Hyang, Seoul 06123 (KR); PARK, Yun Min, Seoul 06123 (KR); PARK, Jin Sung, Seoul 06123 (KR); KIM, Jae Young, Seoul 06123 (KR); YUN, Yewon, Seoul 06123 (KR)
(74) Representative: Plasseraud IP
(86) International application number: PCT/KR2024/001124
(87) International publication number: WO 2024/158205

(57) **Abstract**

The present invention relates to a compound of Chemical Formula 1, a pharmaceutical composition including the same as an active ingredient, and a use thereof. The compound of Chemical Formula 1 according to the present invention may exhibit a necrotic cell death inhibitory effect in various cells such as heart, kidney, nerve, retinal, liver, or lung cells, and thus can be effectively used in the prevention or treatment of necrotic cell death- or ferroptosis-related diseases.

## Description

### [Technical Field]

The present invention relates to a compound of Chemical Formula 1, an isomer thereof, a solvate thereof, a hydrate thereof or pharmaceutically acceptable salt thereof, a pharmaceutical composition including the same, and a use thereof.

### [Background Art]

Cell death contributes to the maintenance and modulation of cellular functions for the survival of an organism, or to the maintenance of cell numbers by balancing cell proliferation for embryonic development. In addition, it is employed in the immune system as an effective and rapid method to inform the organism's immune system of various danger signals arising from the outside, such as the invasion of viruses or bacteria, or localized injury.

However, cell death due to external danger or localized injury can cause local or systemic inflammation due to excessive activation of the immune system, and potentially harming the organism. Cell death that causes this phenomenon is mainly observed in the form of necrotic cell death (or necrosis), and researchers have conducted many studies to treat and improve diseases through the inhibition of necrotic cell death.

Necrotic cell death is generally known as a type of cell death characterized by oxygen depletion and energy depletion, and disruption of the cell membrane, caused by sudden physical or chemical shock to a cell. Necrotic cell death is known to involve the release of damage-associated molecular patterns (DAMPs), such as adenosine triphosphate (ATP), calcium ions (Ca²⁺), reactive oxygen species (ROS), and high mobility group box 1 (HMGB1) to the outside of the cell, which stimulate or damage surrounding cells, thereby inducing a cascade of inflammatory responses and subsequent cell death. Therefore, research on suppressing necrotic cell death for the prevention and treatment of diseases has been conducted for a long time.

As the characteristics of various types of necrotic cell death have been identified, the Nomenclature Committee on Cell Death (NCCD) has subdivided the classification of necrotic cell death. An initially known type of necrotic cell death is non-regulated cell death (NRCD) that occurs due to sudden causes such as physical or chemical damage (accidental cell death), and cannot regulate biological processes. On the other hand, recently known regulated necrotic cell death (RNCD) is caused by physiological causes via diverse mechanisms. Types of cell death classified as regulated necrotic cell death include necroptosis, which occurs via RIPK1, RIPK3, and MLKL mechanisms; pyroptosis, which is mediated by an inflammasome; and ferroptosis, which is induced by the accumulation of lipid peroxides and iron. Among them, ferroptosis has been reported a lot to be observed in various physiological or pathological situations.

Ferroptosis is cell death characterized by the accumulation of excessive lipid ROS and ferrous ions (Fe²⁺). Generally, cells maintain the concentration of glutathione by receiving cysteine via System Xc⁻, and activates an antioxidant system in which glutathione peroxidase 4 (GPX4) eliminates lipid peroxides within the cells. However, when the glutathione concentration is low or GPX4 is not activated, the excessive accumulation of lipid peroxides induces damage to lipids, proteins, and nucleic acids, leading to cell death. Another cause of the excessive accumulation of lipid peroxides is known as ROS produced by binding of ferrous ions to hydrogen peroxide in cells via the Fenton reaction. Cell death caused by lipid peroxides also exhibits necrosis-like features, such as the massive release of DAMPs to the outside of the cells, which triggers inflammation and death of neighboring cells.

Necrotic cell death, which is non-regulated cell death, and ferroptosis, which is regulated cell death, have been reported to be associated with various diseases, including ischemic diseases (e.g., myocardial infarction, stroke, and neuropathy,), neurodegenerative diseases, inflammatory diseases, aging, macular degeneration, lung diseases, and neuropathic pain (refer to Inhibition of ferroptosis-like cell death attenuates neuropathic pain reactions induced by peripheral nerve injury in rats. Eur J Pain. 2021 Jan; 25:1227-1240). Ferroptosis is involved in the development of neuropathic pain and allodynia. Mol Cell Biochem. 2021 Aug; 476(8):3149-3161; Iron Metabolism and Ferroptosis in Peripheral Nerve Injury. Oxid Med Cell Longev. 2022 Dec;5918218.). For this reason, the identification and development of substances that inhibit necrotic cell death and ferroptosis are also actively being performed in disease research and treatment.

Recently, various inhibitors of necrotic cell death and ferroptosis were identified and developed. IM-54 is a representative necrotic cell death inhibitor, and ferrostatin-1 (Fer-1), liproxstatin-1 (Liprox-1) and UAMC-3203 are representative ferroptosis inhibitors. However, these inhibitors have several problems that make it difficult to develop therapeutic agents. Therefore, for disease prevention and treatment, there is an urgent need to develop novel necrotic cell death and ferroptosis inhibitors.

Meanwhile, 4-((2-phenyl-7-((tetrahydro-2H-pyran-4-yl)amino)- 1H-indole-5-yl)methyl)thiomorpholine 1,1-dioxide is the compound disclosed in the International Patent Publication WO2009-025478, and a substance known to exhibit an effect of preventing or treating and improving necrotic cell death and related diseases. Indole derivatives such as this compound are very useful structures pharmaceutically, and much research is being conducted on these structures.

The inventors conducted research to confirm whether indole derivatives, such as the above-mentioned compound, exhibit pharmaceutically similar efficacy on necrotic cell death, which is non-regulated cell death, and ferroptosis, which is regulated cell death, and thus confirmed the effect. In addition, the inventors continuously conducted research on various compounds that exhibit effects in preventing or treating and improving necrotic cell death- and ferroptosis-related diseases, and thus synthesized a novel compound, verified its effect, and completed the present invention.

### [Related Art Document]

### [Patent Document]

International Patent Publication WO2009-025478

### [Detailed Description of Invention]

### [Technical Problem]

The present invention is directed to providing a novel compound of Chemical Formula 1, and an isomer, a solvate, a hydrate or pharmaceutically acceptable salt thereof.

The present invention is also directed to providing a pharmaceutical composition for preventing or treating a necrotic cell death- or ferroptosis-related disease, which includes a pharmaceutically acceptable carrier, and a compound of Chemical Formula 1, or an isomer thereof, a solvate thereof, a hydrate thereof or pharmaceutically acceptable salt thereof as an active ingredient.

### [Technical Solution]

The present invention provides a compound of Chemical Formula 1 below, or an isomer thereof, a solvate thereof, a hydrate thereof or pharmaceutically acceptable salt thereof.

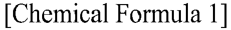

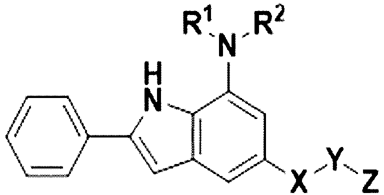

In Chemical Formula 1,
R¹ and R² are each independently hydrogen, -(CH₂)ₘ-C₃₋₁₀ cycloalkyl, -(CH₂)ₘ-C₃₋₁₀ heterocycloalkyl, -(CH₂)ₘ-aryl with 5 and 10 atoms, or -(CH₂)ₘ-heteroaryl with 5 to 10 atoms, wherein m is an integer from 0 to 4,
X is a direct bond, or -C(=O)-NX¹-, -C₁₋₈ alkylene-, C₁₋₈ alkylene-NX¹-, or -C₁₋₈ alkylene-O-,
Y is -(CH₂)ₙ-, -C₁₋₈ alkylene-O-, -C₁₋₈ alkylene-NX¹-, -C₁₋₈ alkynylene-, -C(=O)-, or - S(=O)₂-, wherein n is an integer from 0 to 4,
X¹ is hydrogen or -C₁₋₈ alkyl,
Z is -C₁₋₈ haloalkyl, -C₁₋₈ alkyl, a halogen, -CN, -NR³R⁴, -OH, -S(=O)₂-C₁₋₈ alkyl, -C₃₋₁₀ cycloalkyl, or -C₃₋₁₀ heterocycloalkyl, wherein the -C₁₋₈ alkyl, -C₃₋₁₀ cycloalkyl, or -C₃₋₁₀ heterocycloalkyl of Z is substituted with R³ and/or R⁴, or unsubstituted, and
R³ and R⁴ are each independently hydrogen, -OH, -(=O), -C₁₋₈ alkyl, -C₃₋₁₀ cycloalkyl, - C₁₋₈ alkoxy, or -aryl with 5 to 10 atoms.

However, among the compounds of Chemical Formula 1,
compounds in which -X-Y-Z is -C₁₋₈ alkylene-OH, -C₁₋₈ alkylene-O-C₁₋₈ alkyl, -C₁₋₈ alkylene-O-C₁₋₈ alkynylide alkyl, and -C₁₋₈ alkylene-O-C₁₋₈ alkynylide alkylene-O-C₁₋₈ alkyl are excluded.

In the definition of the substituents according to the present invention, the term "alkyl" refers to an aliphatic hydrocarbon radical. The alkyl may be a saturated alkyl that does not include an alkenyl or alkynyl moiety, or may be an unsaturated alkyl that includes at least one alkenyl or alkynyl moiety.

The term "alkynyl" refers to a group with at least one carbon-carbon triple bond. Alkenyl and alkynyl may mean a linear or branched non-cyclic hydrocarbon.

Unless defined otherwise, an alkyl group may have 1 to 20 carbon atoms. An alkyl group may be a medium-sized C₁₋₁₀ alkyl. An alkyl group may be a lower alkyl with 1 to 6 carbon atoms. Typical alkyl groups include methyl, ethyl, propyl, isopropyl, butyl, isobutyl, t-butyl, pentyl, hexyl, ethenyl, propenyl, and butenyl, but the present invention is not limited thereto. For example, a C₁₋₄ alkyl has 1 to 4 carbon atoms in the alkyl chain, and is selected from the group consisting of ethyl, ethyl, propyl, iso-propyl, n-butyl, iso-butyl, sec-butyl, and t-butyl.

The term "alkylene" refers to a bivalent hydrocarbon group in which a radical is additionally formed from the alkyl, and may include methylene, ethylene, propylene, butylene, and isobutylene, but the present invention is not limited thereto.

Unless defined otherwise, the term "alkoxy" refers to alkyloxy, and may include methoxy, ethoxy, and propoxy, but the present invention is not limited thereto.

The term "haloalkyl" may refer to -RX (X is one or more halogens (F, Cl, Br, or I)), and in other words, the "haloalkyl" may be the form of an alkyl substituted with one or more halogens. For example, a C₁₋₈ haloalkyl may include trifluoromethyl or difluoromethyl, but the present invention is not limited thereto.

The term "cycloalkyl" refers to a saturated or unsaturated aliphatic ring unless otherwise defined. In addition, the number of atoms that form a ring may be 3 to 12. Typical cycloalkyl groups include cyclopropyl, cyclobutyl, cyclopentyl, and cyclohexyl, but the present invention is not limited thereto.

Unless defined otherwise, the term "heterocycloalkyl" refers to the above-defined cycloalkyl having 1 to 3 hetero atoms selected from the group consisting of N, O and S. A heterocycloalkyl may be monocyclic, or polycyclic such as a spiro ring, a bridged ring, or a fused ring. Examples of heterocycloalkyls may include pyrrolidine, piperidine, tetrahydropyran, oxetane, thiopyran, and similar groups, but the present invention is not limited thereto.

The term "aryl" includes at least one ring with a delocalized π-electron system, and includes, for example, a monocyclic or fused polycyclic (i.e., rings sharing adjacent pairs of carbon atoms) group. In other words, unless defined otherwise, the aryl used herein refers to 4 to 10-, and preferably, 6 to 10-membered aromatic monocyclic or polycyclic rings, including phenyl and naphthyl.

Unless defined otherwise, the term "heteroaryl" refers to an aromatic, 3 to 10-, preferably, 4 to 8-, and more preferably, 5 or 6-membered ring, which has 1 to 3 hetero atoms selected from the group consisting of N, O and S and can be fused with benzo or C₃₋₈ cycloalkyl. Examples of monocyclic heteroaryl may include thiazole, oxazole, thiophene, furan, pyrrole, imidazole, isoxazole, isothiazole, pyrazole, triazole, triazine, thiadiazole, tetrazole, oxadiazole, pyridine, pyridazine, pyrimidine, pyrazine, and similar groups, but the present invention is not limited thereto. Examples of non-cyclic heteroaryl may include indole, indoline, benzothiophene, benzofuran, benzimidazole, benzoxazole, benzisoxazole, benzthiazole, benzthiadiazole, benztriazole, quinoline, isoquinoline, purine, furopyridine, and similar groups, but the present invention is not limited thereto.

Unless defined otherwise, other terms and abbreviations used herein shall be interpreted as having the meanings commonly understood by those skilled in the art to which the present invention pertains.

The "hydrate" used herein may refer to a compound of the present invention that includes a stoichiometric or non-stoichiometric amount of water bonded by a non-covalent intermolecular force, or a salt thereof. A hydrate of the compound represented by Chemical Formula 1 may include a stoichiometric or non-stoichiometric amount of water bonded by a non-covalent intermolecular force. The hydrate may contain 1 equivalent or more, and preferably, 1 to 5 equivalents of water. Such a hydrate may be prepared by crystallizing the compound represented by Chemical Formula 1, an isomer thereof, or a pharmaceutically acceptable salt thereof from water or a solvent containing water.

The "solvate" used herein may refer to a compound of the present invention that includes a stoichiometric or non-stoichiometric amount of solvent bound by a non-covalent intermolecular force, or a salt thereof. Preferred solvents for this solvate include those that are volatile, nontoxic, and/or suitable for administration to humans.

The "isomer" used herein may refer to a compound of the present invention that has the same chemical formula or molecular formula but is structurally or sterically different, or a salt thereof. Such isomers include structural isomers such as tautomers, stereoisomers such as R or S isomers with asymmetric carbon centers, geometric isomers (trans, cis), and optical isomers (enantiomers). All of the isomers and mixtures thereof are also be included in the scope of the present invention.

According to one embodiment of the compound of Chemical Formula 1, R¹ and R² are each independently hydrogen, -(CH₂)ₘ-C₃₋₆ cycloalkyl, -(CH₂)ₘ-C₃₋₆ heterocycloalkyl, -(CH₂)ₘ-aryl with 5 and 6 atoms, or -(CH₂)ₘ-5 to 6-membered heteroaryl, wherein m may be an integer from 0 to 2.

According to one embodiment of the compound of Chemical Formula 1, in R¹ and R², the heterocycloalkyl or heteroaryl may include one or more hetero atoms selected from the group consisting of N, O, and S.

According to one embodiment of the compound of Chemical Formula 1, X may be a direct bond, -C(=O)-NX¹-, -C₁₋₃ alkylene-, -C₁₋₃ alkylene-NX¹- or -C₁₋₃ alkylene-O-,
Y may be -(CH₂)ₙ-, -C₁₋₃ alkylene-O-, -C₁₋₃ alkylene-NX¹-, -C₁₋₆ alkynylene-, -C(=O)-, or -S(=O)₂-, wherein n is an integer from 0 to 4,
X¹ may be hydrogen or -C₁₋₃ alkyl,
Z may be -C₁₋₃ haloalkyl, -C₁₋₃ alkyl, -halogen, -CN, -NR³R⁴, -OH, -S(=O)₂-C₁₋₃ alkyl, - C₃₋₆ cycloalkyl, or -C₃₋₆ heterocycloalkyl, wherein the -C₁₋₃ alkyl, -C₃₋₆ cycloalkyl or -C₃₋₆ heterocycloalkyl of Z may be substituted with R³ and/or R⁴ or unsubstituted, and
R³ and R⁴ may each be independently hydrogen, -OH, (=O), -C₁₋₃ alkyl, -C₃₋₆ cycloalkyl, -C₁₋₃ alkoxy, or aryl with 5 and 6 atoms.

According to one embodiment of the compound of Chemical Formula 1, in Z, and R³ to R⁴, the heterocycloalkyl may include one or more hetero atoms selected from the group consisting of N, O, and S.

According to one embodiment of the compounds of Chemical Formula 1,
compounds in which -X-Y-Z is -C₁₋₈ alkylene-OH, -C₁₋₈ alkylene-O-C₁₋₈ alkyl, -C₁₋₈ alkylene-O-C₁₋₈ alkylene-O-C₁₋₈ alkyl, and -C₁₋₈ alkylene-O-C₁₋₈ alkylene-O-C₁₋₈ alkylene-O-C₁₋₈ alkyl may be excluded. The case in which the -X-Y-Z formed with X, Y and Z substituents having the above-described substituents is excluded from the compounds of Chemical Formula 1, and the case to which a different type of substituent (e.g., a hydroxyl group) is additionally attached may be included in the compounds of Chemical Formula 1.

According to one exemplary embodiment of the compound of Chemical Formula 1,
any one of R¹ and R² is hydrogen, and the other is -(CH₂)ₘ-C₃₋₁₀ cycloalkyl, -(CH₂)ₘ-C₃₋₁₀ heterocycloalkyl, -(CH₂)ₘ-aryl with 5 to 10 atoms, or -(CH₂)ₘ-heteroaryl with 5 to 10 atoms, wherein m may be an integer from 0 to 4.

According to one exemplary embodiment of the compound of Chemical Formula 1,
Y may be -(CH₂)ₙ-, -C₁₋₈ alkylene-NX¹- or -C₁₋₈ alkylene-O-, wherein n is an integer from 0 to 4,
X¹ may be hydrogen or -C₁₋₈ alkyl,
Z may be -C₁₋₈ haloalkyl, -C₁₋₈ alkyl, -CN, -OH, -C₃₋₁₀ cycloalkyl, or -C₃₋₁₀ heterocycloalkyl, wherein the -C₁₋₈ alkyl, -C₃₋₁₀ cycloalkyl or -C₃₋₁₀ heterocycloalkyl of Z may be substituted with R³ and/or R⁴ or unsubstituted, and
R³ and R⁴ may each be independently hydrogen, -OH, -(=O), -C₁₋₈ alkyl, -C₁₋₈ alkoxy, or -C₃₋₁₀ cycloalkyl.

According to one exemplary embodiment of the compound of Chemical Formula 1,
Y may be -(CH₂)ₙ-, -C₁₋₈ alkylene-O- or -C₁₋₈ alkynylene-, wherein n is an integer from 0 to 4,
Z may be -C₁₋₈ alkyl, -OH or -C₃₋₁₀ cycloalkyl, wherein the -C₁₋₈ alkyl or -C₃₋₁₀ cycloalkyl of Z is substituted with R³ or unsubstituted, and
R³ may be hydrogen, -OH or C₁₋₈ alkyl.

According to one exemplary embodiment of the compound of Chemical Formula 1,
Y may be -C(=O)- or -S(=O)₂-,
Z may be -NR³R⁴, and
R³ and R⁴ may each be independently hydrogen, -C₁₋₈ alkyl, -C₃₋₁₀ cycloalkyl, or aryl with 5 to 10 atoms.

According to one exemplary embodiment of the compound of Chemical Formula 1,
Y may be -C(=O)-,
Z may be -NR³R⁴, and
R³ and R⁴ may each be independently hydrogen, -C₁₋₈ alkyl, -C₃₋₁₀ cycloalkyl, or aryl with 5 to 10 atoms.

According to one exemplary embodiment of the compound of Chemical Formula 1,
Y may be -(CH₂)ₙ- or -C₁₋₈ alkylene-O-, wherein n is an integer from 0 to 4,
Z may be -halogen, -CN, -S(=O)₂-C₁₋₈ alkyl, -C₁₋₈ alkyl, -C₃₋₁₀ cycloalkyl, or -C₃₋₁₀ heterocycloalkyl, wherein the -C₁₋₈ alkyl, -C₃₋₁₀ cycloalkyl, or C₃₋₁₀ heterocycloalkyl of Z is substituted with R³ or unsubstituted, and
R³ may be hydrogen, -OH, -(=O), -C₁₋₈ alkyl, or -C₃₋₁₀ cycloalkyl.

According to one exemplary embodiment of the compound of Chemical Formula 1,
Z may be -S(=O)₂-C₁₋₈ alkyl, or C₃₋₁₀ heterocycloalkyl, wherein the C₃₋₁₀ heterocycloalkyl of Z is substituted with R³ or unsubstituted, and
R³ may be hydrogen, -(=O), or -C₁₋₈ alkyl.

According to one exemplary embodiment of the compound of Chemical Formula 1,
among the compounds of Chemical Formula 1, a compound in which X is -C₁₋₈ alkylene-, Y is -C(=O)-, and Z is -OH or -C₃₋₁₀ heterocycloalkyl, and a compound in which X is a direct bond or -C₁₋₈ alkylene-, Y is -(CH₂)ₙ-, n is an integer from 1 to 4, and Z is substituted with -(=O) or unsubstituted -C₃₋₁₀ heterocycloalkyl may be excluded.

The compound of Chemical Formula 1 according to the present invention may be any one selected from the group consisting of the following compounds: <1>7-(cyclopentylamino)-N,N-diethyl-2-phenyl-1H-indol-5-carboxamide; <2>N,N-diethyl-2-phenyl-7-((tetrahydro-2H-pyran-4-yl)amino)-1H-indol-5-carboxamide; <3>7-(benzylamino)-N,N-diethyl-2-phenyl-1H-indol-5-carboxamide; <4>N,N-diethyl-2-phenyl-7-((pyridin-4-ylmethyl)amino)-1H-indol-5-carboxamide; <5>7-(cyclopentylamino)-N-cyclopropyl-2-phenyl-1H-indol-5-carboxamide; <6>N-cyclopropyl-2-phenyl-7-((tetrahydro-2H-pyran-4-yl)amino)-1H-indol-5-carboxamide; <7>7-(benzylamino)-N-cyclopropyl-2-phenyl-1H-indol-5-carboxamide; <8>N-cyclopropyl-2-phenyl-7-((pyridin-4-ylmethyl)amino)-1H-indol-5-carboxamide; <9>N,2-diphenyl-7-((tetrahydro-2H-pyran-4-yl)amino)-1H-indol-5-carboxamide; <10>7-(benzylamino)-N,2-diphenyl-1H-indol-5-carboxamide; <11>2-(7-(cyclopentylamino)-2-phenyl-1H-indol-5-yl)-N,N-diethylacetamide; <12>N,N-diethyl-2-(2-phenyl-7-((tetrahydro-2H-pyran-4-yl)amino)-1H-indol-5-yl)acetamide; <13>N,N-diethyl-2-(2-phenyl-7-((pyridin-4-ylmethyl)amino)-1H-indol-5-yl)acetamide; <14>2-(7-(benzylamino)-2-phenyl-1H-indol-5-yl)-N,N-diethylacetamide; <15>N-methyl-2-(2-phenyl-7-((tetrahydro-2H-pyran-4-yl)amino)-1H-indol-5-yl)acetamide; <16>2-(7-(cyclopentylamino)-2-phenyl-1H-indol-5-yl)-N-methylacetamide; <17>2-(7-(benzylamino)-2-phenyl-1H-indol-5-yl)-N-cyclopropylacetamide; <18>2-(7-(benzylamino)-2-phenyl-1H-indol-5-yl)-N-phenylacetamide; <19>N-phenyl-2-(2-phenyl-7-((tetrahydro-2H-pyran-4-yl)amino)-1H-indol-5-yl)acetamide; <20>N-ethyl-2-phenyl-7-((tetrahydro-2H-pyran-4-yl)amino)-1H-indol-5-sulfonamide; <21>7-(benzylamino)-N-ethyl-2-phenyl-1H-indol-5-sulfonamide; <22>7-(cyclopentylamino)-N-ethyl-2-phenyl-1H-indol-5-sulfonamide; <23>2-(2-phenyl-7-((tetrahydro-2H-pyran-4-yl)amino)-1H-indol-5-yl)acetonitrile; <24>2-methyl-4-(2-phenyl-7-((tetrahydro-2H-pyran-4-yl)amino)-1H-indol-5-yl)but-3-yn-2-ol; <25>4-(7-cyclopentylamino)-2-phenyl-1H-indol-5-yl)-2-methylbut-3-yn-2-ol; <26>2-((7-(cyclopentylamino)-2-phenyl-1H-indol-5-yl)methoxy)ethan-1-ol; <27>2-((2-phenyl-7-((tetrahydro-2H-pyran-4-yl)amino)-1H-indol-5-yl)methoxy)ethan-1-ol; <28>2-(3-(2-phenyl-7-((tetrahydro-2H-pyran-4-yl)amino)-1H-indol-5-yl)propoxy)ethan-1-ol; <29>2-(2-((2-phenyl-7-((tetrahydro-2H-pyran-4-yl)amino)-1H-indol-5-yl)methoxy)ethoxy)ethan-1-ol; <30>2-methyl-1-((2-phenyl-7-((tetrahydro-2H-pyran-4-yl)amino)-1H-indol-5-yl)methoxy)propan-2-ol; <31>2-methyl-1-(2-((2-phenyl-7-((tetrahydro-2H-pyran-4-yl)amino)-1H-indol-5-yl)methoxy)ethoxy)propan-2-ol; <32>3-((2-((2-phenyl-7-((tetrahydro-2H-pyran-4-yl)amino)-1H-indol-5-yl)methoxy)ethoxy)methyl)pentan-3-ol; <33>2-(2-(2-phenyl-7-((tetrahydro-2H-pyran-4-yl)amino)-1H-indol-5-yl)ethoxy)ethan-1-ol; <34>3-(2-((2-phenyl-7-((tetrahydro-2H-pyran-4-yl)amino)-1H-indol-5-yl)methoxy)ethoxy)propan-1-ol; <35>4-((2-phenyl-7-((tetrahydro-2H-pyran-4-yl)amino)-1H-indol-5-yl)methoxy)cyclohexan-1-ol; <36>1-methyl-4-((2-phenyl-7-((tetrahydro-2H-pyran-4-yl)amino)-1H-indol-5-yl)methoxy)cyclohexan-1-ol; <37>2-phenyl-N-(tetrahydro-2H-pyran-4-yl)-5-(((tetrahydro-2H-pyran-4-yl)oxy)methyl)-1H-indol-7-amine; <38>4-(2-((2-phenyl-7-((tetrahydro-2H-pyran-4-yl)amino)-1H-indol-5-yl)methoxy)ethoxy)cyclohexan-1-ol; <39>5-((2-fluoroethoxy)methyl)-2-phenyl-N-(tetrahydro-2H-pyran-4-yl)-1H-indol-7-amine; <40>5-((2,2-difluoroethoxy)methyl)-2-phenyl-N-(tetrahydro-2H-pyran-4-yl)-1H-indol-7-amine; <41>N-cyclopentyl-5-((2-morpholinoethoxy)methyl)-2-phenyl-1H-indol-7-amine; <42>5-((2-morpholinoethoxy)methyl)-2-phenyl-N-(tetrahydro-2H-pyran-4-yl)-1H-indol-7-amine; <43>4-(2-((7-(cyclopentylamino)-2-phenyl-1H-indol-5-yl)methoxy)ethyl)thiomorpholine 1,1-dioxide; <44>N-cyclopentyl-5-((2-(methylsulfonyl)ethoxy)methyl)-2-phenyl-1H-indol-7-amine; <45>5-((2-(methylsulfonyl)ethoxy)methyl)-2-phenyl-N-(tetrahydro-2H-pyran-4-yl)-1H-indol-7-amine; <46>2-phenyl-N-(tetrahydro-2H-pyran-4-yl)-5-((2-thiomorpholinoethoxy)methyl)-1H-indol-7-amine; <47>4-(2-((2-phenyl-7-((tetrahydro-2H-pyran-4-yl)amino)-1H-indol-5-yl)methoxy)ethyl)thiomorpholine 1,1-dioxide; <48>2-((2-((2-phenyl-7-((tetrahydro-2H-pyran-4-yl)amino)-1H-indol-5-yl)methoxy)ethyl)amino)ethan-1-ol; <49>N-(2-methoxyethyl)-2-phenyl-7-((tetrahydro-2H-pyran-4-yl)amino)-1H-indol-5-carboxamide; <50>5-(((2-methoxyethyl)amino)methyl)-2-phenyl-N-(tetrahydro-2H-pyran-4-yl)-1H-indol-7-amine; <51>N-(2-methoxyethyl)-N-methyl-2-phenyl-7-((tetrahydro-2H-pyran-4-yl)amino)-1H-indol-5-carboxamide; <52>N-(2-(2-methoxyethoxy)ethyl)-N-methyl-2-phenyl-7-((tetrahydro-2H-pyran-4-yl)amino)-1H-indol-5-carboxamide; <53>2-(methyl(2-((2-phenyl-7-((tetrahydro-2H-pyran-4-yl)amino)-1H-indol-5-yl)methoxy)ethyl)amino)ethan-1-ol; <54>N-(2-(2-hydroxyethoxy)ethyl)-N-methyl-2-phenyl-7-((tetrahydro-2H-pyran-4-yl)amino)-1H-indol-5-carboxamide; <55>5-(((2-methoxyethyl)(methyl)amino)methyl)-2-phenyl-N-(tetrahydro-2H-pyran-4-yl)-1H-indol-7-amine; <56>N-(2-(2-methoxyethoxy)ethyl)-2-phenyl-7-((tetrahydro-2H-pyran-4-yl)amino)-1H-indol-5-carboxamide; <57>5-(((2-(2-methoxyethoxy)ethyl)amino)methyl)-2-phenyl-N-(tetrahydro-2H-pyran-4-yl)-1H-indol-7-amine; <58>5-(((2-(2-methoxyethoxy)ethyl)(methyl)amino)methyl)-2-phenyl-N-(tetrahydro-2H-pyran-4-yl)-1H-indol-7-amine; <59>N-(2-((2-phenyl-7-((tetrahydro-2H-pyran-4-yl)amino)-1H-indol-5-yl)methoxy)ethyl)methanesulfonamide; <60>5-((2-((2-methoxyethyl)amino)ethoxy)methyl)-2-phenyl-N-(tetrahydro-2H-pyran-4-yl)-1H-indol-7-amine, and <61>5-((2-((2-methoxyethyl)(methyl)amino)ethoxy)methyl)-2-phenyl-N-(tetrahydro-2H-pyran-4-yl)-1H-indol-7-amine.

The present invention also provides a pharmaceutical composition, which includes the compound of Chemical Formula 1, an isomer thereof, a solvate thereof, a hydrate thereof or pharmaceutically acceptable salt thereof; and a pharmaceutically acceptable carrier.

Specifically, the present invention provides a pharmaceutical composition for preventing or treating a necrotic cell death- or ferroptosis-related disease, which includes the compound of Chemical Formula 1, an isomer thereof, a solvate thereof, a hydrate thereof or pharmaceutically acceptable salt thereof; and a pharmaceutically acceptable carrier.

The necrotic cell death- or ferroptosis-related disease may be one or more selected from the group consisting of
an acute or chronic liver disease, such as hepatitis, fibrosis, or cirrhosis;
a degenerative nerve disease such as dementia including Alzheimer's disease and vascular dementia, Parkinson's disease, epilepsy, dementia with Lewy bodies, or Huntington's disease;
an ischemic disease such as ischemic heart disease, reperfusion injury, ischemic stroke, or ischemic injury;
pancreatitis, bacterial or viral sepsis, diabetes or diabetic complications, or a diabetic vascular disease;
necrotizing proctitis, cystic fibrosis, rheumatoid arthritis, degenerative arthritis, nephrotic syndrome, bacterial infections, viral infections such as SARS-CoV, multiple sclerosis, leukemia, lymphoma, neonatal respiratory distress syndrome, asphyxiation, tuberculosis, endometriosis, angiasthenia, frostbite, steroid injection course/complications, vibrio sepsis, tenderness, hemoglobinuria, burns, hyperthermia, celiac disease, compartment syndrome, spinal cord injury, glomerulonephritis, renal failure, metabolic genetic disorders, mycoplasma infections, anthrax, Fabry disease, congenital mitochondrial diseases, phenylketonuria, placental infarction, syphilis, and aseptic necrosis;
necrosis associated with exposure to drugs such as antibiotics, anticancer agents, doxorubicin, puromycin, bleomycin, non-steroidal anti-inflammatory drugs (NSAIDs), or cyclosporine, chemical toxins such as carbon tetrachloride, cyanide, methanol, or ethylene glycol, toxic gas, pesticides, or heavy metals such as lead, mercury, or cadmium, or administration thereof, or self-administration; and
damage caused by radiation or UV exposure and necrotic cell death associated therewith.

In addition, the compound of Chemical Formula 1 is expected to exhibit an effect of preventing or treating and improving acute or chronic kidney disease, traumatic brain injury, necrotizing enterocolitis, viral infections such as SARS-CoV, a skin disease such as psoriasis or allergic dermatitis; or organ preservation or organ transplantation (refer to Korean Patent Nos. 10-1098583 and 10-1941004).

In addition, the compound of Chemical Formula 1 may inhibit cell death through a Fenton reaction caused by ferroptosis-inducing substances such as RSL3, elastin or glutamate and ferroptosis due to lipid peroxide accumulation caused by the disruption of the GPX4 pathway.

Therefore, the pharmaceutical composition including the compound of Chemical Formula 1 may exhibit an effect of preventing or treating and improving a necrotic cell death- or ferroptosis-related disease. Related diseases are as follows:
inflammatory pulmonary diseases such as acute respiratory distress syndrome, acute pulmonary disease, pneumonia, tuberculosis, asthma, chronic obstructive pulmonary disease (COPD), idiopathic pulmonary fibrosis (IPF), and cystic fibrosis (Mitochondrial dysfunction in fibrotic diseases. Cell Death Discov. 2020 Sep 5;6:80., Mitochondrial dysfunction in lung aging and diseases. Eur Respir Rev. 2020 Oct 15;29(157):200165.; Korean Patent No. 10-1636563);
demyelinating diseases, such as demyelination and amyotrophic lateral sclerosis (ALS), hypertension including pulmonary hypertension, mental disorders such as stroke, prion disease, epilepsy, ataxia, migraine, memory and cognitive decline, seizures, tremors, or depression (Neuronal and glial calcium signaling in Alzheimer's disease. Cell Calcium. Oct-Nov 2003;34(4-5):385-97., Mitochondrial disorders: challenges in diagnosis & treatment. Indian J Med Res. 2015 Jan;141(1):13-26.);
spinocerebellar degeneration such as Friedreich's ataxia (Ferroptosis in Friedreich's Ataxia: A Metal-Induced Neurodegenerative Disease. Biomolecules. 2020 Nov 13;10(11):1551., The molecular and metabolic landscape of iron and ferroptosis in cardiovascular disease. Nat Rev Cardiol. 2023 Jan;20(1):7-23.);
insulin resistance, dyslipidemia such as hyperlipidemia, atherosclerosis, and inflammatory bowel disease (IBD) such as Crohn's disease and ulcerative colitis;
various types of cancer and cancer metastasis (Reticulum stress and oxidative stress in cell fate decision and human disease. Antioxid Redox Signal. 2014 Jul 20;21(3):396-413.);
visual impairment-related diseases such as macular degeneration, retinitis pigmentosa, cataracts, and glaucoma, anemia, cholestasis, hypoparathyroidism, pancytopenia, pancreatic disorder, lactic acidosis, lacticemia, hearing loss, short stature, intestinal obstruction, cardiac conduction defects including arrhythmia, cardiomyopathy, myocardial infarction, ischemia-reperfusion heart injury, heart failure, endometriosis, infertility, subfertility, and early menopause (Mitochondrial diseases: the contribution of organelle stress responses to pathology. Nat Rev Mol Cell Biol. 2018 Feb;19(2):77-92., Seminars in medicine of the Beth Israel Hospital, Boston. Mitochondrial DNA and disease. N Engl J Med. 1995 Sep 7;333(10):638-44., Mitochondrial injury and dysfunction in hypertension-induced cardiac damage. Eur Heart J. 2014 Dec 7; 35(46): 3258-3266.);
muscular atrophy such as limb-girdle muscular dystrophy (LGMD), Becker muscular dystrophy (BMD), and Duchenne muscular dystrophy (DMD) (Duchenne muscular dystrophy is associated with the inhibition of calcium uniport in mitochondria and an increased sensitivity of the organelles to the calcium-induced permeability transition. Biochim Biophys Acta Mol Basis Dis. 2020 May 1;1866(5):165674.);
aging and aging-related diseases (Interrelation between ROS and Ca2+ in aging and age-related diseases. Redox Biology. 2020;6:101678.);
neuropathic pain (Inhibition of ferroptosis-like cell death attenuates neuropathic pain reactions induced by peripheral nerve injury in rats. Eur J Pain. 2021 Jan; 25:1227-1240., Ferroptosis is involved in the development of neuropathic pain and allodynia. Mol Cell Biochem. 2021 Aug; 476(8):3149-3161., Iron Metabolism and Ferroptosis in Peripheral Nerve Injury. Oxid Med Cell Longev. 2022 Dec 2;2022:5918218.); and
mucositis, such as oral mucositis and gastrointestinal mucositis (Emerging role of mitochondrial DAMPs, aberrant mitochondrial dynamics and anomalous mitophagy in gut mucosal pathogenesis. Life Sci. 2022 Sep 15;305:120753., The Impacts of Iron Overload and Ferroptosis on Intestinal Mucosal Homeostasis and Inflammation, Int J Mol Sci. 2022 Nov 17;23(22):14195.).

In the present invention, the compound of Chemical Formula 1 may be used to prevent or treat heart diseases including ischemic heart disease, cardiac conduction defects, cardiomyopathy, myocardial infarction, and heart failure, degenerative brain diseases such as dementia, Parkinson's disease, and Lou Gehrig's disease, and the ischemia-reperfusion injury and fibrosis inhibition of the kidney, acute and chronic respiratory diseases, and macular degeneration.

The "treatment" used herein refers to stopping or delaying the progression of a disease when used in subjects exhibiting symptoms of a disease, and the "prevention" used herein refers to stopping or delaying the onset of disease when used in subjects that do not exhibit symptoms but are at high risk for such disease.

In the present invention, "administration" refers to the provision of the composition of the present invention to a subject by a suitable method.

In the present invention, the "pharmaceutical composition" may include the compound of the present invention and, if necessary, a pharmaceutically acceptable carrier.

The compound of Chemical Formula 1 according to the present invention may be administered in various oral and parenteral formulations in clinics, and in the preparation of the formulation, commonly used diluents or excipients such as fillers, thickening agents, binders, wetting agents, disintegrants, and surfactants are employed.

Solid preparations for oral administration include tablets, pills, powders, granules, and capsules, and such solid preparations are prepared by mixing at least one excipient, such as starch, calcium carbonate, sucrose, lactose, or gelatin with the composition. Aside from simple excipients, lubricants such as magnesium stearate, talc, etc. are further used. Liquid preparations for oral administration include suspensions, liquids for internal use, emulsions, and syrups, and may further include various types of excipients, for example, a wetting agent, a sweetener, a fragrance and a preservative, other than commonly-used simple diluents such as water or liquid paraffin.

Formulations for parenteral administration may include sterilized aqueous solutions, non-aqueous solvents, suspensions, emulsions, freeze-dried formulations, or suppositories. As the non-aqueous solvent or suspension, propylene glycol, polyethylene glycol, a vegetable oil such as olive oil, or an injectable ester such as ethyl oleate may be used. As a suppository base, Witepsol, Tween 61, cacao butter, laurin oil, glycerol, or gelatin may be used.

In addition, an effective dose of the compound of the present invention represented by Chemical Formula 1 for the human body may vary depending on a patient's age, body weight and sex, a dosage form, health status, and disease severity. The dose may be approximately 0.001 to 100 mg/kg/day, and preferably, 0.01-35 mg/kg/day. For an adult patient weighing 70 kg, the dose may be generally 0.07 to 7000 mg/day, and preferably, 0.7 to 2500 mg/day. The administration dose may be determined by the judgment of a physician or pharmacist, and may be applied once a day or in divided portions at regular intervals.

The pharmaceutical composition containing the compound of Chemical Formula 1, an isomer thereof, a hydrate thereof, a solvate thereof, or a pharmaceutically acceptable salt thereof as an active ingredient may be administered as a single therapeutic agent or in combination with another therapeutic agent currently in use.

Another aspect of the present invention provides a method of preventing or treating a necrotic cell death- or ferroptosis-related disease, which includes administering the compound of Chemical Formula 1, an isomer thereof, a hydrate thereof, a solvate thereof, or a pharmaceutically acceptable salt thereof to a subject in need thereof at a pharmaceutically effective amount.

In the present invention, the necrotic cell death- or ferroptosis-related disease may involve lipid peroxidation.

In the present invention, "lipid peroxidation" refers to oxidative degradation of lipids, oil, wax, sterols, or triglycerides, and lipid peroxidation is considered one of the main causes of various degenerative diseases.

In the present invention, the necrotic cell death- or ferroptosis-related disease may specifically be a degenerative neurological disease, liver disease, kidney disease, stroke, myocardial infarction, ocular disease, or lung disease.

The degenerative neurological disease may be one or more selected from Alzheimer's disease, Parkinson's disease, epilepsy, Huntington's disease, amyotrophic lateral sclerosis, Friedreich's ataxia, multiple sclerosis, Charcot-Marie-Tooth (CMT) disease, dementia with Lewy bodies, and traumatic brain injury.

Still another aspect of the present invention provides a method of inhibiting ferroptosis, which includes administering the compound of Chemical Formula 1, an isomer thereof, a hydrate thereof, a solvate thereof, or a pharmaceutically acceptable salt thereof to a subject in need thereof at a pharmaceutically effective amount.

In the present invention, the compound of Chemical Formula 1 may interfere with or inhibit ferroptosis by acting on a ferroptosis-inducing substance, such as elastin, glutamate, or RSL3, thereby suppressing cell death.

Yet another aspect of the present invention provides a method of reducing reactive oxygen species (ROS) in cells or mitochondria, which includes contacting the compound of Chemical Formula 1, an isomer thereof, a hydrate thereof, a solvate thereof, or a pharmaceutically acceptable salt thereof with cells or mitochondria, or administering it to a subject in need thereof in a pharmaceutically effective amount.

In the present invention, "reactive oxygen species (ROS)" refers to chemically active molecules, such as free radicals, containing oxygen, and examples of ROS may include oxygen ions and peroxides. Ferroptosis is characterized by rapid iron-dependent accumulation of reactive oxygen species, and the compound of Chemical Formula 1 can inhibit ferroptosis by inhibiting ROS.

In the present invention, "contact" may mean bringing the compound of the present invention and one or more optional additional therapeutic agents into proximity with a cell or mitochondria in need thereof. For example, the compound of the present invention and one or more optional additional therapeutic agents may be provided to cells or mitochondria in a subject by a conventional drug delivery method, or may be provided to cells or mitochondria in vitro by treatment with a culture medium containing cells or mitochondria.

In the present invention, a "subject" requiring administration may include both a mammal and a non-mammal. Here, examples of the mammals may include humans, non-human primates such as chimpanzees or monkey species, livestock animals such as cattle, horses, and sheep, but the present invention is not limited thereto.

Yet another aspect of the present invention provides a use of the compound of Chemical Formula 1, or an isomer, solvent or pharmaceutically acceptable salt thereof to prevent or treat a necrotic cell death- or ferroptosis-related disease.

The content of the pharmaceutical composition may be applied to the use and preventing or treating methods of the present invention.

### [Advantageous Effects]

A compound of Chemical Formula 1 according to the present invention can exhibit a necrotic cell death inhibitory effect in various cells such as heart, kidney, nerve, retinal, liver, or lung cells, and may efficiently inhibit, especially, ferroptosis.

In addition, the compound of Chemical Formula 1 according to the present invention can have an excellent pharmacokinetic value in plasma.

Therefore, the compound of Chemical Formula 1 according to the present invention can be useful for the prevention or treatment of the necrotic cell death- or ferroptosis-related diseases in various cells.

### [Description of Drawings]

FIG. 1 is a graph showing the effect of the compound of Example 29 on regulating lipid ROS in cells under RSL3 treatment conditions according to Experimental Example 4.
FIG. 2 is a graph showing the effect of the compound of Example 29 on regulating lipid ROS in mitochondria under RSL3 treatment conditions according to Experimental Example 5.
FIG. 3 is a graph showing the effect of the compound of Example 29 on regulating ROS in cells under RSL3 treatment conditions according to Experimental Example 6
FIG. 4 is a graph showing the effect of the compound of Example 29 on regulating ROS in mitochondria under RSL3 treatment conditions according to Experimental Example 7.
FIG. 5 is a graph showing the effect of the compound of Example 29 on regulating ferrous ions in cells under RSL3 treatment conditions according to Experimental Example 8.
FIG. 6 is a graph showing the effect of the compound of Example 29 on regulating ferrous ions in cells under RSL3 treatment conditions according to Experimental Example 9.

### [Best Mode]

Hereinafter, the advantages and features of the present invention and the methods of accomplishing the same will become apparent with reference to the following examples to be described in detail and the accompanying drawings. However, the present invention is not limited to the exemplary embodiments disclosed below, and may be embodied in many different forms. These exemplary embodiments are merely provided to complete the disclosure of the present invention and fully convey the scope of the present invention to those of ordinary skill in the art, and the present invention should be defined by only the accompanying claims.

### [Examples]

The following preparation examples more specifically explain the preparation of intermediates necessary for the synthesis of examples according to the present invention. Abbreviations used in the following preparation examples and examples are as follows:
Ac: acetyl
AcOH: acetic acid
ACN: acetonitrile
BOC: t-butoxycarbonyl
Bn: benzyl
Bu: butyl
DBU: 1,8-diazabicyclo (5,4,0)undec-7-ene
DCM: dichloromethane
DIBAL-H: diisobutylaluminum hydride
DIPEA: diisopropylethylamine
4-DMAP: 4-dimethylaminopyridine
DMF: N,N-dimethylformamide
DMSO: dimethyl sulfoxide
EA: ethyl aceate
EtOH: ethyl alcohol
Et: ethyl
EtI: ethyl iodiode
HATU: (1-[bis(dimethylamino)methylene]-1H-1,2,3-triazolo[4,5-b]pyridinium 3-oxide hexafluorophosphate
Hex: hexane
EDC: 1-ethyl-3-(3-dimethylaminopropyl)carbodiimide
LAH: lithium aluminum hydride
LCMS: liquid chromatography mass spectrometry
Me: methyl
MeOH: methyl alcohol
MsCl: methanesulfonyl chloride
NMM: N-methylmorpholine
NBS: N-bromosuccinimide
NIS: N-iodosuccinimide
PE: petroleum ether
Pd/C: palladium/charcoal
Pd(dppf)Cl₂: [1,1'-bis(diphenylphosphino)ferrocene]dichloropalladium (II)
Ph: phenyl
TBAF: tetrabutylammonium fluoride
TEA: triethylamine
TFA: trifluoroacetic acid
THF: tetrahydrofuran
TLC: thin layer chromatography
TMS: trimethylsilyl

### Preparation Example A1: N,N-diethyl-7-nitro-2-phenyl-1H-indol-5-carboxamide

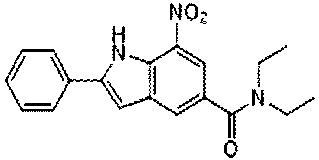

7-nitro-2-phenyl-1H-indol-5-carboxylic acid (200 mg, 708.59 µmol), HATU (323.31 mg, 850.31 µmol), DIPEA (274.73 mg, 2.13 mmol, 370.26 uL), and N-ethylethanamine (57.01 mg, 779.45 µmol, 80.29 uL) were added to DMF (4 mL) and allowed to react for 1 hour at room temperature. After confirming the termination of the reaction by LCMS, EA and H₂O were added and extracted, an organic layer was washed with a saturated NaCl solution, and the organic layer was dried with MgSO₄ and then filtered. The filtrate was concentrated under reduced pressure and purified through silica gel column chromatography, thereby obtaining the title compound (230 mg, 96.2%) as a yellow solid.
¹H NMR (400 MHz, DMSO-d₆) δ11.83 (br s, 1H), 8.07 (br s, 1H), 7.98 - 8.03 (m, 3H), 7.48 - 7.54 (m, 2H), 7.38 - 7.44 (m, 1H), 7.21 (s, 1H), 3.28 - 3.32 (m, 4H), 1.12 - 1.21 (m, 6H)

### Preparation Example A2: 7-amino-N,N-diethyl-2-phenyl-1H-indol-5-carboxamide

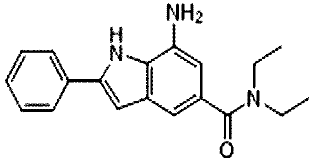

After the N,N-diethyl-7-nitro-2-phenyl-1H-indol-5-carboxamide (230 mg, 681.74 µmol) obtained in Preparation Example A1 was dissolved in THF (6 mL), Pd/C (100 mg) was added. After nitrogen purging, a hydrogen balloon was inserted and stirred at room temperature for 1 hour. After confirming the termination of the reaction by LCMS, the reaction solution was filtered through Celite and concentrated under reduced pressure, thereby obtaining the title compound (200 mg, 95.4%) as a yellow solid.

The following compounds of respective preparation examples in Table 1 were prepared in a similar manner to Preparation Examples A1 and A2. The chemical structure, compound name, NMR and LCMS analysis results of the compound of each preparation example are shown in Table 1.

**[Table 1]**

| **Prepar ation Exam ple No.** | **structure** | **compound name** | **¹H NMR; MS[M+H]+** |
|---|---|---|---|
| A3 | 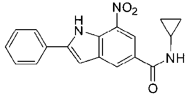 | N-cyclopropyl-7-nitro-2-phenyl-1H-indole-5-carboxamide | ¹H NMR (400 MHz, DMSO-d₆) δ 11.86 (br s, 1H), 8.74 (s, 1H), 8.58 (s, 1H), 8.54 (s, 1H), 8.03 (d, J=7.6Hz, 2H), 7.48 - 7.54 (m, 2H), 7.39 - 7.44 (m, 1H), 7.26 (s, 1H), 2.87 - 2.91 (m, 1H), 0.71 - 0.76 (m, 2H), 0.61 - 0.65 (m, 2H) |
| A4 | 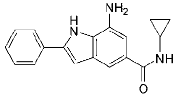 | 7-amino-N-cyclopropyl-2-phenyl-1H-indole-5-carboxamide | |
| A5 | 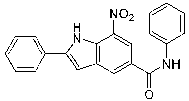 | 7-nitro-N,2-diphenyl-1H-indole-5-carboxamide | ¹H NMR (400 MHz, DMSO-d₆) δ11.96 (br s, 1H), 10.51 (br s, 1H), 8.72 (s, 2H), 8.06 (d, J=7.2Hz, 2H), 7.83 (d, J=8.0Hz, 2H), 7.48 - 7.53 (m, 2H), 7.36 - 7.39 (m, 1H), 7.33 - 7.35 (m, 3H), 7.11 - 7.15 (m, 1H) |
| A6 | 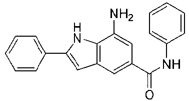 | 7-amino-N,2-diphenyl-1H-indole-5-carboxamide | |
| A12 | 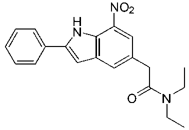 | N,N-diethyl-2-(7-nitro-2-phenyl-1H-indol-5-yl)acetamide | |
| A13 | 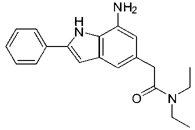 | 2-(7-amino-2-phenyl-1H-indol-5-yl)-N,N-diethylacetamide | |
| A14 | 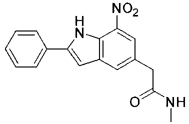 | N-methyl-2-(7-nitro-2-phenyl-1H-indol-5-yl)acetamide | ¹H NMR (400MHz, CDCl₃) δ 10.10 (br s, 1H), 8.05 (s, 1H), 7.90 (s, 1H), 7.76 (d, 2H), 7.54 (t, 2H), 7.44 (t, 1H), 6.93 (s, 1H), 5.47 (br, 1H), 3.74 (s, 2H), 2.82 (d, 3H); MS(ESI): m/z 310.11 [M+H]+ |
| A15 | 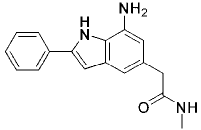 | 2-(7-amino-2-phenyl-1H-indol-5-yl)-N-methylacetamide | |
| A16 | 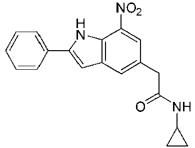 | N-cyclopropyl-2-(7-nitro-2-phenyl-1H-indol-5-yl)acetamide | |
| A17 | 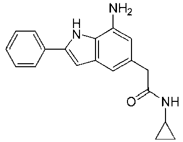 | 2-(7-amino-2-phenyl-1H-indol-5-yl)-N-cyclopropylacetamide | |
| A18 | 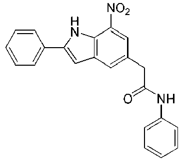 | 2-(7-nitro-2-phenyl-1H-indol-5-yl)-N-phenylacetamide | ¹H NMR (400 MHz, DMSO-*d₆*) δ11.61 (br s, 1H), 10.25 (br s, 1H), 8.10 (s, 1H), 7.98 - 8.02 (m, 3H), 7.60 (d, J=7.2Hz, 2H), 7.47 - 7.50 (m, 2H), 7.39 - 7.41 (m, 1H), 7.29 - 7.32 (m, 2H), 7.28 (s, 1H), 7.02 - 7.05 (m, 1H), 3.84 (s, 2H) |
| A19 | 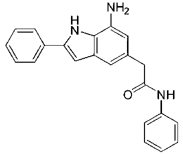 | 2-(7-amino-2-phenyl-1H-indol-5-yl)-N-phenylacetamide | |

### Preparation Example A7: {(7-nitro-2-phenyl-1H-indol-5-yl)methanol}

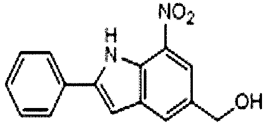

7-nitro-2-phenyl-1H-indol-5-carboxylic acid (1.0 g, 3.45 mmol) was added to THF (10 mL) and then cooled to 0 °C. After slowly adding 2M BH₃.SMe₂/THF (5.3 mL, 10.6 mmol) dropwise, the resulting solution was stirred for 30 minutes at the same temperature, heated to room temperature and stirred for 5 hours. The reaction solution was cooled to 0 °C, and a 1N NaOH aqueous solution (10 mL) was slowly added dropwise for 30 minutes. After additional stirring for 1 hour, extraction was performed twice with EA to collect an organic layer. The organic layer was washed with a saturated NaCl solution, dried over MgSO₄, and then filtrated. The filtrate was concentrated under reduced pressure, crystallized by adding ethanol, filtered, and then dried under vacuum, thereby obtaining the title compound (770 mg, 71%) as a yellow solid.

¹H NMR (400MHz, CDCl₃) δ 10.08 (br s, 1H), 8.17 (s, 1H), 7.97 (s, 1H), 7.76 (d, 2H), 7.51 (t, 2H), 7.49 (t, 1H), 6.94 (s, 1H), 4.87 (s, 2H), 1.81 (t, 1H; MS (ESI): m/z 268.0 [M+H]+)

### Preparation Example A8: 5-(bromomethyl)-7-nitro-2-phenyl-1H-indole

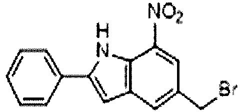

The (7-nitro-2-phenyl-1H-indol-5-yl)methanol (7.6 g, 28.33 mmol) obtained in Preparation Example A7 was added to THF (76 mL), and cooled to 0 °C. After slowing adding PBR2 (1.6 mL, 17.0 mmol) dropwise, the resulting solution was heated to room temperature and stirred for 2 hours. The reaction solution was poured into ice water to terminate the reaction, and EA was added for extraction. An organic layer was dried over MgSO₄ and then filtered. The filtrate was concentrated under reduced pressure, crystallized by adding diethyl ether, filtered and dried under vacuum, thereby obtaining the title compound (7.5 g, 80%) as a yellow solid.
¹H NMR (400MHz, CDCl₃) δ 10.04 (br s, 1H), 8.13 (s, 1H), 7.94 (s, 1H), 7.72 (d, 2H), 7.50 (t, 2H), 7.42 (t, 1H), 6.90 (s, 1H), 4.84 (s, 2H; MS (ESI): m/z 331.17 [M+H]+)

### Preparation Example A9: 2-(7-nitro-2-phenyl-1H-indol-5-yl)acetonitrile

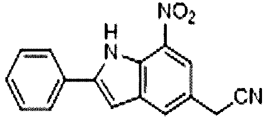

The 5-(bromomethyl)-7-nitro-2-phenyl-1H-indole (500 mg, 1.509 mmol) obtained in Preparation Example A8 was dissolved in methanol (8 mL) and water (2 mL), and KCN (127 mg, 1.962 mmol) was added dropwise and stirred overnight at room temperature. After extraction by adding EA and water to the reaction solution, an organic layer was washed with a saturated NaCl solution, dried over MgSO₄, filtered, and concentrated under reduced pressure. The concentrated residue was purified through silica gel column chromatography, thereby obtaining the title compound (206 mg, 49%) as a yellow solid.
¹H NMR (400MHz, CDCl₃) δ 10.09 (br s, 1H), 8.05 (s, 1H), 7.93 (s, 1H), 7.73 (d, 2H), 7.50 (t, 2H), 7.44 (t, 1H), 6.93 (s, 1H), 3.92 (s, 2H; MS (ESI): m/z 278.0 [M+H]+)

### Preparation Example A10: methyl 2-(7-nitro-2-phenyl-1H-indol-5-yl)acetate}

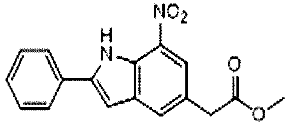

The 2-(7-nitro-2-phenyl-1H-indol-5-yl)acetonitrile (800 mg, 2.89 mmol) obtained in Preparation Example A9 was dissolved in methanol (10 mL), 4M HCl/MeOH (93.43 mmol, 20 mL) was added, and the resulting solution was stirred for 8 hours at 70 °C. After confirming the termination of the reaction by LCMS, the reaction solution was concentrated under reduced pressure and purified through silica gel column chromatography, thereby obtaining the title compound (800 mg, 89.36%) as a yellow solid.

### Preparation Example A11: 2-(7-nitro-2-phenyl-1H-indol-5-yl)acetic acid

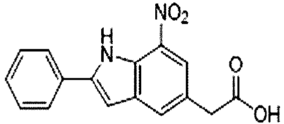

The methyl 2-(7-nitro-2-phenyl-1H-indol-5-yl)acetate (800 mg, 2.58 mmol) obtained in Preparation Example A10 was dissolved in tetrahydrofuran (10 mL) and water (10 mL), LiOH·H₂O (432.75 mg, 10.31 mmol) was added, and then the resulting solution was stirred for 12 hours at room temperature. After confirming the termination of the reaction by LCMS, the resulting solution was adjusted to pH of 5 to 6 using a 1N HCl aqueous solution. EA was added to the reaction solution for extraction, and the resulting extract was dried over MgSO₄, filtered and concentrated under reduced pressure, thereby obtaining the title compound (800 mg, crude) as a yellow solid.

### Preparation Example A20: N-ethyl-4-fluoro-3-nitrobenzenesulfonamide

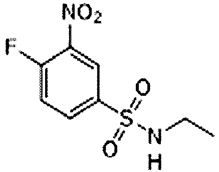

4-fluoro-3-nitrobenzylsulfonyl chloride (3 g, 12.52 mmol) was dissolved in THF (50 mL), an ethanamine (1.23 g, 15.02 mmol)/TEA (2.53 g, 25.04 mmol) solution was slowly added at - 50 °C, the resulting solution was warmed to 0 °C and stirred for 4 hours. After confirming the termination of the reaction by TLC, EA and water were added to the reaction solution for extraction, and an organic layer was washed with a saturated NaCl solution, dried over MgSO₄, filtered and concentrated under reduced pressure. The concentrated residue was purified through silica gel column chromatography, thereby obtaining the title compound (2 g, 64.35%) as a yellow solid.

### Preparation Example A21: 4-amino-N-ethyl-3-nitrobenzenesulfonamide

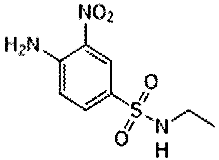

The N-ethyl-4-fluoro-3-nitrobenzenesulfonamide (2 g, 8.06 mmol) obtained in Preparation Example A20 was dissolved in dioxane (20 mL), NH₃.H₂O (22.59 g, 161.14 mmol) was added at 0 °C, and the resulting solution was stirred for 3 hours at 20 °C. After confirming the termination of the reaction by TLC, EA and water were added to the reaction solution for extraction, and an organic layer was washed with a saturated NaCl solution, dried over MgSO₄, filtered and concentrated under reduced pressure, thereby obtaining the title compound (2.1 g, crude) as a yellow solid. This compound was used in a subsequent reaction without purification.
¹H NMR (400MHz, CDCl₃) δ8.66 (s, 1H), 7.79 (d, J=7.2Hz, 1H), 6.92 (d, J=7.2Hz, 1H), 6.47 (br s, 1H), 4.35 (br s, 1H), 2.98 - 3.05 (m, 2H), 1.11 - 1.26 (m, 3H)

### Preparation Example A22: 4-amino-N-ethyl-3-iodo-5-nitrobenzenesulfonamide

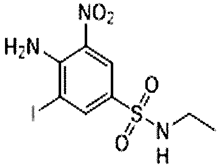

The 4-amino-N-ethyl-3-nitrobenzenesulfonamide (2 g, 8.15 mmol) obtained in Preparation Example A21 was dissolved in THF (40 mL), sulfuric acid (93.58 mg, 954.11 µmol) was added, and the resulting solution was stirred for 30 minutes at 20 °C. After adding NIS (2.75 g, 12.23 mmol) to the reaction solution, the resulting solution was stirred for 3 hours at 70 °C, and then the termination of the reaction was confirmed by TLC. EA and water were added to the reaction solution for extraction, and an organic layer was washed with a saturated NaCl solution, dried over MgSO₄, filtered and concentrated under reduced pressure. The concentrated residue was purified through silica gel column chromatography, thereby obtaining the title compound (1.5 g, 49.54%) as a yellow solid.
¹H NMR (400 MHz, DMSO-d₆) δ 8.38 (s, 1H), 8.25 (s, 1H), 2.47 - 2.79 (m, 2H), 0.95 - 1.18 (m, 3H)

### Preparation Example A23: 4-amino-N-ethyl-3-nitro-5-(phenylethynyl)benzenesulfonamide

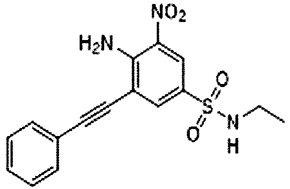

The 4-amino-N-ethyl-3-iodo-5-nitrobenzenesulfonamide (500 mg, 1.35 mmol) obtained in Preparation Example A22 was dissolved in dioxane (3 mL), and then ethynylbenzene (165.11 mg, 1.62 mmol) and TEA (408.95 mg, 4.04 mmol) were added and stirred for 30 minutes at 20 °C. CuI (2.57 mg, 13.47 µmol) and PdCl₂(PPh₃)₂ (9.46 mg, 13.47 µmol) were added to the reaction solution, and the resulting solution was stirred for 3 hours at 60 °C. After confirming the termination of the reaction by LCMS, it was used immediately in a subsequent reaction.

### Preparation Example A24: N-ethyl-7-nitro-2-phenyl-1H-indol-5-sulfonamide

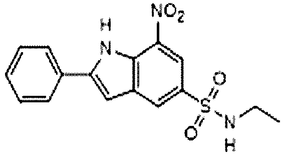

DBU (1.23 g, 8.08 mmol) was added to the reaction mixture of 4-amino-N-ethyl-3-nitro-5-(phenylethynyl)benzenesulfonamide (1.35 mmol) obtained in Preparation Example A23 and stirred for 2 hours at 110 °C. After confirming the termination of the reaction by LCMS, EA and water were added to the reaction solution for extraction, and an organic layer was washed with a saturated NaCl solution, dried over MgSO₄, filtered and concentrated under reduced pressure. The concentrated residue was purified through silica gel column chromatography, thereby obtaining the title compound (188 mg, 40.43%) as a yellow solid.
¹H NMR (400 MHz, DMSO-d₆) δ 12.09 (br s, 1H), 8.44 (s, 1H), 8.41 (s, 1H), 8.03 (d, J=7.6Hz, 2H), 7.68 - 7.72 (m, 1H, NH), 7.49 - 7.53 (m, 2H), 7.45 - 7.48 (m, 1H), 7.34 (s, 1H), 2.75 - 2.85 (m, 2H), 0.987 (t, J=7.2Hz, 3H)

### Preparation Example A25: 7-amino-N-ethyl-2-phenyl-1H-indol-5-sulfonamide

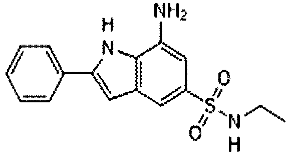

After the N-ethyl-7-nitro-2-phenyl-1H-indol-5-sulfonamide (188 mg, 544.34 µmol) obtained in Preparation Example A24 was dissolved in methanol (2 mL), THF (2 mL) and water (1 mL), Fe powder (364.78 mg, 6.53 mmol) and NH₄Cl (582.35 mg, 10.89 mmol) were added and stirred for 1 hour at 80 °C. After confirming the termination of the reaction by LCMS, EA and water were added to the reaction solution for extraction, and an organic layer was washed with a saturated NaCl solution, dried over MgSO₄, filtered and concentrated under reduced pressure, thereby obtaining the title compound (169 mg, crude) as a yellow solid.
¹H NMR (400 MHz, DMSO-d₆) δ 11.31 (br s, 1H), 7.84 (d, J=7.2Hz, 2H), 7.48 - 7.52 (m, 2H), 7.33 - 7.38 (m, 1H), 7.27 (s, 1H), 7.09 - 7.13 (m, 1H), 6.96 (s, 1H), 6.74 (s, 1H), 5.60 (br s, 2H), 2.68 - 2.78 (m, 2H), 0.95 (t, J=7.6Hz, 3H)

The A26, A30, A32, A41, A43, A48, andA52 compounds of the following preparation examples were prepared in similar manners to Preparation Example A25.

### Preparation Example A26: 2-(7-amino-2-phenyl-1H-indol-5-yl)acetonitrile

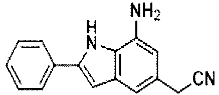

MS (ESI): m/z 248.1 [M+H]+

### Preparation Example A27: 4-bromo-2-iodo-6-nitroaniline

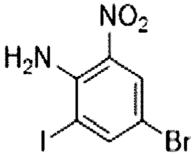

Commercially available 4-bromo-2-nitroaniline (7.4 g, 34.10 mmol) was treated in the same manner as in Preparation Example A22, thereby obtaining the title compound (8.6 g, 73%) as a brown solid; MS (ESI): m/z 343.9 [M+H]+)

### Preparation Example A28: 5-bromo-7-nitro-2-phenyl-1H-indole

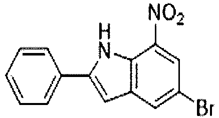

The 4-bromo-2-iodo-6-nitroaniline (2 g, 5.83 mmol) obtained in Preparation Example A27 was dissolved in dioxane (20 mL), ethynylbenzene (1.08 g, 10.6 mmol), TEA (1.77 g, 17.497 mmol), PdCl₂(PPh₃)₂ (41 mg, 0.0583 mmol), and CuI (11.1 mg, 0.0583 mmol) were added, and the resulting solution was heated to 50 °C and stirred for 4 hours. After cooling the resulting solution to room temperature and adding DBU (5.3 g, 35 mmol), the resulting solution was heated to 100 °C and refluxed overnight. After cooling to room temperature and filtration with Celite, EA and water were added to the filtrate to extract an organic layer twice, and the organic layer was washed with a saturated NaCl solution, dried over MgSO₄ and filtered. The filtrate was concentrated under reduced pressure and purified through silica gel column chromatography to obtain a yellow solid. The solid was crystallized by adding diethyl ether and then filtered, thereby obtaining the title compound (510 mg, 28%) as a yellow solid.
¹H NMR (400MHz, CDCl₃) δ 10.07 (br s, 1H), 8.23 (s, 1H), 8.04 (s, 1H), 7.72 (d, 2H), 7.50 (t, 2H), 7.44 (t, 1H), 6.88 (s, 1H; MS (ESI): m/z 318.1 [M+H]+)

### Preparation Example A29: 2-methyl-4-(7-nitro-2-phenyl-1H-indol-5-yl)but-3-yn-2-ol

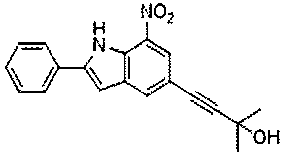

The 5-bromo-7-nitro-2-phenyl-1H-indole (100 mg, 0.135 mmol) obtained in Preparation Example A28, K₂CO₃ (435 mg, 3.15 mmol), and CuI (6 mg, 0.0315 mmol) were dissolved in anhydrous DMF (1.5 mL), 2-methyl but-3-yn-2-ol (0.046 mL, 0.473 mmol) and Pd(PPh₃)₄ (18 mg, 0.015 mmol) were added, and then the reactants were allowed to react for 1 hour at 120 °C in a microwave. After confirming the termination of the reaction, EA and water were added to the reaction solution to extract an organic layer twice, and then the organic solution was washed with a saturated NaCl solution, dried over MgSO₄ and filtered. The filtrate was concentrated under reduced pressure and purified through silica gel column chromatography, thereby obtaining the title compound (89 mg, 89%) as a yellow solid.
¹H NMR (400MHz, CDCl₃) δ 10.09 (br s, 1H), 8.18 (s, 1H), 7.98 (s, 1H), 7.72 (d, 2H), 7.50 (t, 2H), 7.44 (t, 1H), 6.89 (s, 1H), 1.64 (s, 3H), 1.53 (s, 3H; MS (ESI): m/z 321.3 [M+H]+)

### Preparation Example A30: 4-(7-amino-2-phenyl-1H-indol-5-yl)-2-methylbut-3-yn-2-ol

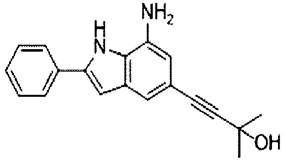

¹H NMR (400MHz, CDCl₃) δ 8.31 (br s, 1H), 7.67 (d, 2H), 7.44 (t, 2H), 7.34 (t, 1H), 7.30 (s, 1H), 6.76 (s, 1H), 6.64 (s, 1H), 1.63 (s, 6H; MS (ESI): m/z 291.2 [M+H]+)

### Preparation Example A31: 2-((7-nitro-2-phenyl-1H-indol-5-yl)methoxy)ethan-1-ol

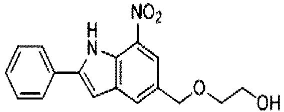

The 5-(bromomethyl)-7-nitro-2-phenyl-1H-indole (2 g, 6 mmol) obtained in Preparation Example A8 was dissolved in ethylene glycol (60 mL) and stirred overnight at 80 °. After confirming the termination of the reaction by TLC, the resulting solution was cooled to room temperature, extracted with EA twice to obtain an organic layer, the organic layer was washed with water and a saturated NaCl solution, dried over MgSO₄, and filtered. The concentrated residue was purified through silica gel column chromatography, thereby obtaining the title compound (1.7 g, 90%) as a yellow solid.
¹H NMR (400MHz, CDCl₃) δ 10.10 (br s, 1H), 8.15 (s, 1H), 7.96 (s, 1H), 7.75 (d, 2H), 7.53 (t, 2H), 7.44 (t, 1H), 6.95 (s, 1H), 4.74 (s, 2H), 3.83 (t, 2H), 3.69 (t, 2H), 1.61 (br, 1H; MS (ESI): m/z 313.3 [M+H]+)

### Preparation Example A32: 2-((7-amino-2-phenyl-1H-indol-5-yl)methoxy)ethan-1-ol

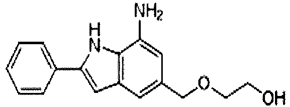

¹H NMR (400MHz, CDCl₃) δ 8.43 (br s, 1H), 7.72 (d, 2H), 7.47 (t, 2H), 7.33 (t, 1H), 7.14 (s, 1H), 6.80 (s, 1H), 6.59 (s, 1H), 4.58 (s, 2H), 3.79 (t, 2H), 3.65 (t, 2H), 2.07 (br, 2H), 1.28 (br, 1H; MS (ESI): m/z 283.3 [M+H]+)

### Preparation Example A33: 7-nitro-2-phenyl-1H-indol-5-carbaldehyde

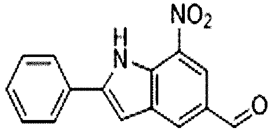

The (7-nitro-2-phenyl-1H-indol-5-yl)methanol (5 g, 18.6 mmol) obtained in Preparation Example A7 was dissolved in DCM (300 mL) and acetone (100 mL), pyridinium chlorochromate (5.63 g, 26.12 mmol) was added and then the resulting solution was heated and refluxed overnight. After confirming the termination of the reaction by TLC, the resulting solution was filtered through silica gel and concentrated, thereby obtaining the title compound (5 g, 100% crude) as a yellow solid.
¹H NMR (400MHz, CDCl₃) δ 10.34 (br s, 1H), 10.15 (s, 1H), 8.68 (s, 1H), 8.51 (s, 1H), 7.79 (d, 2H), 7.57 (t, 2H), 7.49 (t, 1H), 7.12 (s, 1H; MS (ESI): m/z 267.07 [M+H]+)

### Preparation Example A34: Tert-butyl 5-formyl-7-nitro-2-phenyl-1H-indol-1-carboxylate

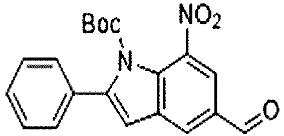

The 7-nitro-2-phenyl-1H-indol-carbaldehyde (5 g, 18.7 mmol) obtained in Preparation Example A33 was dissolved in DCM (250 mL), and then TEA (6.54 mL, 46.95 mmol), (Boc)₂O (8.19 g, 37.56 mmol), and 4-DMAP (0.229 g, 1.878 mmol) were added. The resulting solution was stirred for 2 days at room temperature. After confirming the termination of the reaction by TLC, the concentrated residue was purified through column chromatography, thereby obtaining the title compound (5.2 g, 75%).
¹H NMR (400MHz, CDCl₃) δ 10.12 (s, 1H), 8.38 (d, 2H), 7.49 (m, 5H), 6.77 (s, 1H), 1.31 (s, 9H; MS (ESI): m/z 367.12 [M+H]+)

### Preparation Example A35: 3-(1-(tert-butoxycarbonyl)-7-nitro-2-phenyl-1H-indol-5-yl)propanoic acid

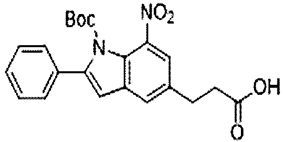

Formic acid (1.63 mL, 42.57 mmol), TEA (2.57 mL, 18.45 mmol), and 2,2-dimethyl-1,3-dioxane-4,6-dione (2.045 g, 14.19 mmol) were added to dimethylformamide (20 mL) at 10 °C. The tert-butyl 5-formyl-7-nitro-2-phenyl-1H-indol-1-carboxylate (5.2 g, 14.19 mmol) obtained in Preparation Example A34 was added and then the resulting solution was stirred overnight at 80 °C. After confirming the termination of the reaction by TLC, a saturated NH₄Cl solution was added to the reaction solution at 10 °C and the resulting solution was stirred for 30 minutes. After washing an organic layer with a saturated NH₄Cl solution three times, the resulting layer was washed with water and then with a saturated NaCl solution. After drying the resulting solution over anhydrous MgSO₄ and filtration, the filtrate was concentrated, thereby obtaining the title compound (6 g, Crude) as an orange oil.
¹H NMR (400MHz, CDCl₃) δ 7.85 (s, 1H), 7.78 (s, 1H), 7.51 (m, 2H), 7.46 (m, 2H), 6.61 (s, 1H), 3.84 (s, 2H), 1.30 (s, 9H; MS (ESI): m/z 410.15 [M+H]+)

### Preparation Example A36: Tert-butyl 5-(3-hydroxypropyl)-7-nitro-2-phenyl-1H-indol-1-carboxylate

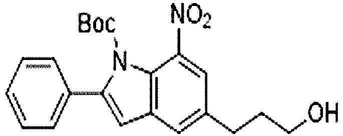

The 3-(1-(tert-butoxycarbonyl)-7-nitro-2-phenyl-1H-indol-5-yl)propionic acid (6 g, 14.76 mmol) obtained in Preparation Example A35 was dissolved in THF (120 mL) and cooled to 0 °C. BH₃.SMe₂ 2M in THF (22.05 mL, 44.28 mmol) was added, and the resulting solution was heated to room temperature and stirred for 5 hours. The completion of the reaction was confirmed by TLC, and then the temperature was lowered to 0 °C. A 1N NaOH aqueous solution was slowly added, and then the resulting solution was stirred for 1 hour. The resulting solution was extracted with EA twice, an organic layer was dried over MgSO₄, filtered and then concentrated under reduced pressure. The concentrated residue was purified through silica gel column chromatography, thereby obtaining the title compound (3.2 g, 55%) as a yellow oil.
¹H NMR (400MHz, CDCl₃) δ 7.76 (s, 1H), 7.67 (s, 1H), 7.54 (m, 2H), 7.44 (m, 3H), 6.58 (s, 1H), 3.75 (t, 2H), 2.91 (t, 2H), 1.99 (m, 2H), 1.30 (s, 9H; MS (ESI): m/z 397.17 [M+H]+)

### Preparation Example A37: Tert-butyl 5-(3-((methylsulfonyl)oxy)propyl)-7-nitro-2-phenyl-1H-indol-1-carboxylate

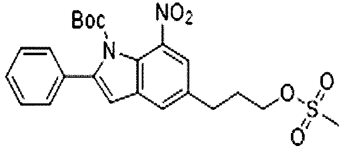

The tert-butyl 5-(3-hydroxypropyl)-7-nitro-2-phenyl-1H-indol-1-carboxylate (300 mg, 0.756 mmol) obtained in Preparation Example A36 was dissolved in DCM (6 mL), TEA (0.255 mL, 1.82 mmol) was added dropwise, and the resulting solution was cooled to 0 °C. MsCl (0.07 mL, 0.908 mmol) was added dropwise, and then the resulting solution was stirred for 2 hours at room temperature. After confirming the termination of the reaction by TLC, the resulting solution was extracted with EA twice, and then an organic layer was washed with a saturated NaHCO₃ solution. The organic layer was dried over MgSO₄, filtered and concentrated under reduced pressure. The concentrated residue was purified through silica gel column chromatography, thereby obtaining the title compound (342 mg, 95%) as a yellow oil.
¹H NMR (400MHz, CDCl₃) δ 7.74 (s, 1H), 7.70 (s, 1H), 7.54 (m, 2H), 7.46 (m, 3H), 6.59 (s, 1H), 4.29 (t, 2H), 3.06 (s, 3H), 2.96 (t, 2H), 2.20 (m, 2H), 1.30 (s, 9H; MS (ESI): m/z 475.5 [M+H]+)

### Preparation Example A38: 2-(3-(7-nitro-2-phenyl-1H-indol-5-yl)propoxy)ethan-1-ol

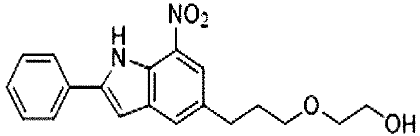

The tert-butyl 5-(3-((methylsulfonyl)oxy)propyl)-7-nitro-2-phenyl-1H-indol-1-carboxylate (342 mg, 0.72 mmol) obtained in Preparation Example A37 was dissolved in ethylene glycol (5 mL), potassium iodide (12 mg, 0.075 mmol) was added, and then the resulting solution was heated and refluxed overnight. The completion of the reaction was confirmed by TLC and then the temperature was lowered to room temperature. After extraction with EA twice and washing with a saturated NaHCO₃ solution, an organic layer was washed with a saturated NaCl solution. The organic solution was dried over MgSO₄, filtered and concentrated under reduced pressure. The concentrated residue was purified through silica gel column chromatography, thereby obtaining the title compound (170 mg, 66%) as a yellow solid.
¹H NMR (400MHz, CDCl₃) δ 10.04 (br s, 1H), 8.02 (s, 1H), 7.80 (s, 1H), 7.76 (s, 2H), 7.53 (t, 2H), 7.41 (t, 1H), 6.91 (s, 1H), 3.80 (m, 2H), 3.58 (m, 4H), 2.91 (t, 2H), 2.03 (m, 2H; MS (ESI): m/z 341.38 [M+H]+)

### Preparation Example A39: 2-(3-(7-amino-2-phenyl-1H-indol-5-yl)propoxy)ethan-1-ol

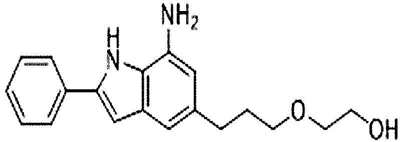

¹H NMR (400MHz, CDCl₃) δ 8.25 (br s, 1H), 7.69 (d, 2H), 7.46 (t, 2H), 7.32 (t, 1H), 7.00 (s, 1H), 6.77 (s, 1H), 6.49 (s, 1H), 3.78 (m, 2H), 3.56 (m, 4H), 2.74 (t, 2H), 1.97 (m, 2H; MS (ESI): m/z 311.1 [M+H]+)

### Preparation Example A40: 2-(2-((7-nitro-2-phenyl-1H-indol-5-yl)methoxy)ethoxy)ethan-1-ol

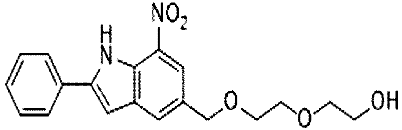

The 5-(bromomethyl)-7-nitro-2-phenyl-1H-indole (200 mg, 0.604 mmol) obtained in Preparation Example A8 was dissolved in 2,2'-oxybis(ethan-1-ol) (5 mL), and the resulting solution was heated to 80 °C and stirred overnight. After confirming the termination of the reaction by TLC, EA and water were added to the reaction solution and extracted twice, and then the resulting product was dried over MgSO₄, filtrated and concentrated under reduced pressure. The concentrated residue was purified through silica gel column chromatography, thereby obtaining the title compound (210 mg, 97%).
¹H NMR (400MHz, CDCl₃) δ 10.10 (br s, 1H), 8.18 (s, 1H), 7.97 (s, 1H), 7.78 (d, 2H), 7.53 (t, 2H), 7.42 (t, 1H), 6.95 (s, 1H), 4.75 (s, 2H), 3.81-3.74 (m, 6H), 3.68-3.66 (m, 2H), 2.25 (t, 1H; MS (ESI): m/z 357.1 [M+H]+)

### Preparation Example A41: 2-(2-((7-amino-2-phenyl-1H-indol-5-yl)methoxy)ethoxy)ethan-1-ol

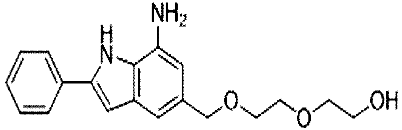

¹H NMR (400MHz, CDCl₃) δ 9.63 (br s, 1H), 7.79 (d, 2H), 7.46 (t, 2H), 7.34 (t, 1H), 7.04 (s, 1H), 6.77 (s, 1H), 6.05 (s, 1H), 4.43 (s, 2H), 3.87-3.83 (m, 4H), 3.73-3.67 (m, 4H; MS (ESI): m/z 327.1 [M+H]+)

### Preparation Example A42: 2-methyl-1-((7-nitro-2-phenyl-1H-indol-5-yl)methoxy)propan-2-ol

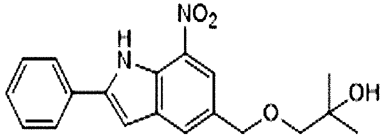

The 5-(bromomethyl)-7-nitro-2-phenyl-1H-indole (1 g, 3.02 mmol) obtained in Preparation Example A8 was dissolved in 2-methylpropane (25 mL), K₂CO₃ (417.33 mg, 3.02 mmol) was added, and the resulting solution was heated and refluxed for 1.5 hours at 80 °C. After confirming the termination of the reaction by TLC, EA and water were added to the reaction solution and extracted twice, and the resulting extract was dried over MgSO₄, filtered, and concentrated under reduced pressure. The concentrated residue was purified through silica gel column chromatography, thereby obtaining the title compound (427 mg, 41%) as a yellow solid.

### Preparation Example A43: 1-((7-amino-2-phenyl-1H-indol-5-yl)methoxy)-2-methylpropan-2-ol

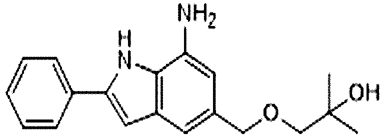

MS (ESI): m/z 311.1 [M+H]+)

### Preparation Example A44: Ethyl 2-(2-(benzyloxy)ethoxy)acetate

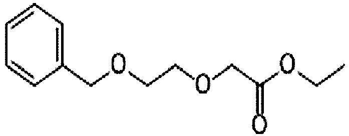

2-(benzyloxy)ethan-1-ol (5 g, 32.85 mmol) was dissolved in DMF (50 mL), NaH (1.58 g, 39.42 mmol, 60% purity) was added at 0 °C and the resulting solution was stirred for 1.5 hours at room temperature. A compound in which ethyl 2-bromoacetate (8.23 g, 49.28 mmol) was dissolved in DMF (20 mL) was added to the reaction solution and stirred for 2 hours at room temperature. After confirming the termination of the reaction by LCMS, EA and H₂O were added and extracted to obtain an organic layer, and the organic layer was washed with a saturated NaCl solution, dried over MgSO₄, and filtered. The filtrate was concentrated under reduced pressure and purified through silica gel column chromatography, thereby obtaining the title compound (5 g, 63%) as a yellow oil.
¹H NMR (400 MHz, DMSO-d₆) δ 7.30 - 7.37 (m, 5H), 4.49 (s, 2H), 4.08 - 4.17 (m, 4H), 3.62 - 3.66 (m, 2H), 3.57 - 3.59 (m, 2H), 1.71 - 1.22 (m, 3H)

### Preparation Example A45: 1-(2-(benzyloxy)ethoxy)-2-methylpropan-2-ol

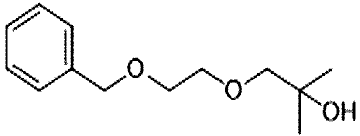

The ethyl 2-(2-(benzyloxy)ethoxy)acetate (5 g, 20.98 mmol) obtained in Preparation Example A44 was added to THF (70 mL) and purged with nitrogen at 0 °C, MeMgBr (3 M, 17.49 mL, 2.5 eq.) was added, and then the resulting solution was stirred for 2 hours at 0 °C. After confirming the termination of the reaction by TLC, EA and H₂O were added to perform extraction, and an organic layer was washed with a saturated NaCl solution, dried over MgSO₄, and filtered. The filtrate was concentrated under reduced pressure and purified through silica gel column chromatography, thereby obtaining the title compound (2.7 g, 57%) as a transparent oil.
¹H NMR (400 MHz, DMSO-d₆) δ 7.27 - 7.36 (m, 5H), 4.51 (s, 2H), 4.31 (br s, 1H), 3.56 - 3.60 (m, 4H), 3.18 (s, 2H), 1.07 (s, 6H)

### Preparation Example A46: 1-(2-hydroxyethoxy)-2-methylpropan-2-ol

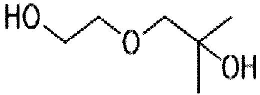

The 1-(2-(benzyloxy)ethoxy)-2-methylpropan-2-ol (2.7 g, 12.04 mmol) obtained in Preparation Example A45 was added to methanol (40 mL), Pd/C (1 g, 10 wt.%) was added, purged with nitrogen, and stirred for 12 hours at room temperature while inserting a hydrogen balloon. After confirming the termination of the reaction by TLC, the reaction solution was filtered through Celite and concentrated under reduced pressure, thereby obtaining the title compound (1.6 g, 99%) as a transparent oil.
¹H NMR (400 MHz, DMSO-d₆) δ 4.56 (t, J=5.2Hz, 1H), 4.29 (s, 1H), 3.44 - 3.49 (m, 2H), 3.37 - 3.42 (m, 2H), 3.16 (s, 2H), 1.06 (s, 6H)

### Preparation Example A47: 2-methyl-1-(2-((7-nitro-2-phenyl-1H-indol-5-yl)methoxy)ethoxy)propan-2-ol

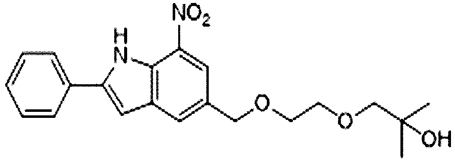

The 5-(bromomethyl)-7-nitro-2-phenyl-1H-indole (400 mg, 1.21 mmol) obtained in Preparation Example A8 and the 1-(2-hydroxyethoxy)-2-methylpropan-2-ol (1.62 g, 12.08 mmol) obtained in Preparation Example A46 were added to DMF (5 mL), K₂CO₃ (166.93 mg, 1.21 mmol) was added, and then the resulting solution was stirred for 5 hours at 80 °C. After confirming the termination of the reaction by LCMS, EA and H₂O were added to perform extraction, and an organic layer was washed with a saturated NaCl solution, dried over MgSO₄, and filtered. The filtrate was concentrated under reduced pressure and purified through silica gel column chromatography, thereby obtaining the title compound (170 mg, 36%) as a yellow solid.

The compound of Preparation Example A50 below was prepared in a similar manner to Preparation Example A46, and the compounds of Preparation Examples A51, A62, A69, and A73 were prepared in a similar manner to Preparation Example A47.

### Preparation Example A48: 1-(2-((7-amino-2-phenyl-1H-indol-5-yl)methoxy)ethoxy)-2-methylpropan-2-ol

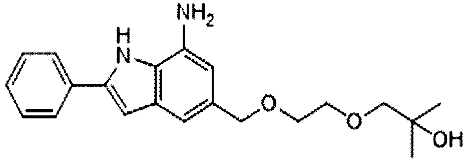

### Preparation Example A49: 3-((2-(benzyloxy)ethoxy)methyl)pentan-3-ol

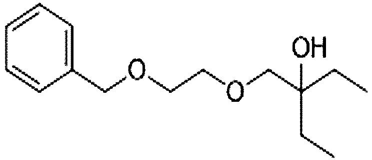

The ethyl 2-(2-(benzyloxy)ethoxy)acetate (1 g, 4.20 mmol) obtained in Preparation Example A44 and 2M EtMgBr/THF (2 M, 5.88 mL) were treated in the same manner as Preparation Example A45, thereby obtaining the title compound (619 mg, 58%) as a yellow oil.

### Preparation Example A50: 3-((2-hydroxyethoxy)methyl)pentan-3-ol

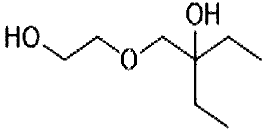

¹H NMR (400MHz, CDCl₃) δ 3.73 - 3.75 (m, 2H), 3.58 - 3.60 (m, 2H), 3.36 (s, 2H), 2.60 (br s, 2H), 1.49 - 1.56 (m, 4H), 0.87 (t, J=7.6Hz, 6H)

### Preparation Example A51: 3-((2-((7-nitro-2-phenyl-1H-indol-5-yl)methoxy)ethoxy)methyl)pentan-3-ol

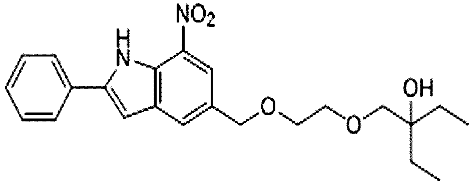

### Preparation Example A52: 3-((2-((7-amino-2-phenyl-1H-indol-5-yl)methoxy)ethoxy)methyl)pentan-3-ol

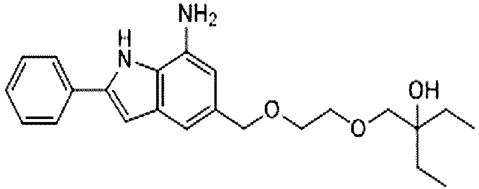

### Preparation Example A53: Tert-butyl 5-(2-methoxy-2-oxoethyl)-7-nitro-2-phenyl-1H-indol-1-carboxylate

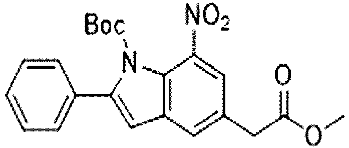

The methyl 2-(7-nitro-2-phenyl-1H-indol-5-yl)acetate (2.33 g, 7.058 mmol) obtained in Preparation Example A10 was treated in the same manner as in Preparation Example A34, thereby obtaining the title compound (2.53 g, 83%) as a yellow liquid.

### Preparation Example A54: 2-(1-(tert-butoxycarbonyl)-7-nitro-2-phenyl-1H-indol-5-yl)acetic acid

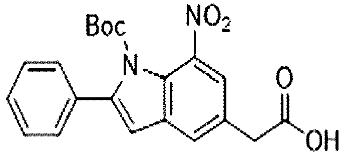

The tert-butyl 5-(2-methoxy-2-oxoethyl)-7-nitro-2-phenyl-1H-indol-1-carboxylate (2.53 g, 6.164 mmol) obtained in Preparation Example A53 were treated in the same manner as in Preparation Example A11, thereby obtaining the title compound (1.1 g, 47%) as a yellow solid.
¹H NMR (400MHz, CDCl₃) d 7.85 (s, 1H), 7.78 (s, 1H), 7.53-7.45 (m, 5H), 6.61 (s, 1H), 3.84 (s, 2H), 1.30 (s, 9H; MS (ESI): m/z 397 [M+H]+)

### Preparation Example A55: Tert-butyl 5-(2-hydroxyethyl)-7-nitro-2-phenyl-1H-indol-1-carboxylate

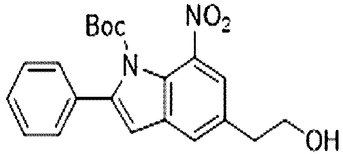

The 2-(1-(tert-butoxycarbonyl)-7-nitro-2-phenyl-1H-indol-5-yl)acetic acid (1.1 g, 2.78 mmol) obtained in Preparation Example A54 was treated in the same manner as in Preparation Example A11, thereby obtaining the title compound (990 mg, 93%) as a yellow solid.
¹H NMR (400MHz, CDCl₃) δ 10.06 (br, 1H), 7.80 (s, 1H), 7.72 (s, 1H), 7.54 (m, 2H), 7.46 (m, 3H), 6.60 (s, 1H), 3.98 (q, 2H), 3.05 (t, 2H), 1.44 (t, 1H), 1.30 (s, 9H; MS (ESI): m/z 383.4 [M+H]+)

### Preparation Example A56: 2-(7-nitro-2-phenyl-1H-indol-5-yl)ethan-1-ol

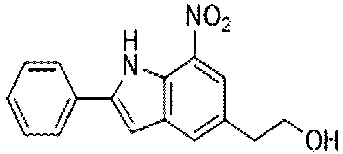

The tert-butyl 5-(2-hydroxyethyl)-7-nitro-2-phenyl-1H-indol-1-carboxylate (180 mg, 047 mmol) obtained in Preparation Example A55 was dissolved in DCM (10 mL), TFA (2 mL) was added dropwise, and the resulting solution was allowed to react for 16 hours at room temperature. After confirming the termination of the reaction by TLC, a saturated NaHCO₃ aqueous solution (20 mL) was added, and an organic layer was dried over MgSO₄ and filtered. The filtrate was concentrated under reduced pressure, and the concentrated residue was purified through silica gel column chromatography, thereby obtaining the title compound (30 mg, 22%) as a yellow solid.
¹H NMR (400MHz, CDCl₃) δ 10.04 (br s, 1H), 8.03 (s, 1H), 7.75 (s, 1H), 7.74 (d, 2H), 7.51(t, 2H), 7.43 (t, 1H), 6.90 (s, 1H), 3.97 (q, 2H), 3.04 (t, 2H), 1.46 (t, 1H; MS (ESI): m/z 283.11 [M+H]+)

### Preparation Example A57: 2-(7-nitro-2-phenyl-1H-indol-5-yl)ethyl methanesulfonate

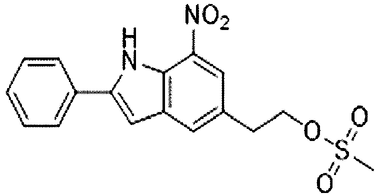

The 2-(7-nitro-2-phenyl-1H-indol-5-yl)ethan-1-ol (530 mg, 1.877 mmol) obtained in Preparation Example A56 and MsCl (322 mg, 2.816 mmol) were treated in the same manner as in Preparation Example A37, thereby obtaining the title compound (570 mg, 84%).
¹H NMR (400MHz, CDCl₃) δ 10.06 (br s, 1H), 8.02 (s, 1H), 7.84 (s, 1H), 7.74 (d, 2H), 7.52 (t, 2H), 7.47 (t, 1H), 6.91 (s, 1H), 4.50 (m, 2H), 3.24 (t, 2H), 2.95 (s, 3H; MS (ESI): m/z 361.08 [M+H]+)

### Preparation Example A58: 2-(2-(7-nitro-2-phenyl-1H-indol-5-yl)ethoxy)ethan-1-ol

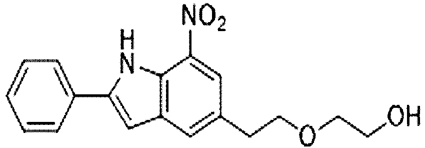

The 2-(7-nitro-2-phenyl-1H-indol-5-yl)ethyl methanesulfonate (222 mg, 0.616 mmol) obtained in Preparation Example A57 was treated in the same manner as in Preparation Example A38, thereby obtaining the title compound (110 mg, 55%).
¹H NMR (400MHz, CDCl₃) δ 10.03 (br s, 1H), 8.04 (s, 1H), 7.82 (s, 1H), 7.74 (d, 2H), 7.51 (t, 2H), 7.49 (t, 1H), 6.89 (s, 1H), 3.80 (t, 2H), 3.74 (br, 2H), 3.59 (br, 2H), 3.07 (t, 2H; MS (ESI): m/z 327.13 [M+H]+)

### Preparation Example A59: 2-(2-(7-amino-2-phenyl-1H-indol-5-yl)ethoxy)ethan-1-ol

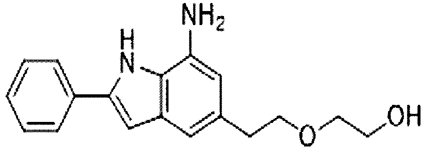

The 2-(2-(7-nitro-2-phenyl-1H-indol-5-yl)ethoxy)ethan-1-ol (100 mg, 0.306 mmol) obtained in Preparation Example A58 was treated in the same manner as in Preparation Example A2, thereby obtaining the title compound (70 mg, 77%; MS (ESI): m/z 297.16 [M+H]+).

The compounds of Preparation Examples A64, A66, A70, A74, A77, and A79 below were prepared in a similar manner to Preparation Example A59.

### Preparation Example A60: Methyl 3-(2-(benzyloxy)ethoxy)acrylate

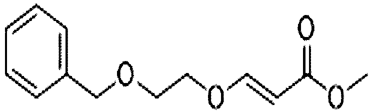

2-(benzyloxy)ethan-1-ol (3 g, 19.7 mmol), methyl propiolate (1.823 g, 21.68 mmol), and N-methylmorpholine (2.193 g, 21.68 mmol) were added to DCM (60 mL) and stirred at room temperature. After confirming the termination of the reaction by TLC, the resulting product was concentrated under reduced pressure and purified through silica gel column chromatography, thereby obtaining the title compound (4.60 g, 98%).
¹H NMR (400MHz, CDCl₃) δ 7.63 (d, 1H), 7.37-7.30 (m, 5H), 5.24 (d, 1H), 4.58 (s, 2H), 4.02 (br s, 2H), 3.73 (br s, 2H), 3.70 (s, 3H; MS (ESI): m/z 237.1 [M+H]+)

### Preparation Example A61: Methyl 3-(2-hydroxyethoxy)propanoate

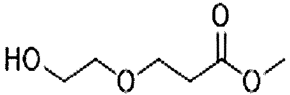

The methyl 3-(2-(benzyloxy)ethoxy)acrylate (4.6 g, 19.46 mmol) obtained in Preparation Example A60 was treated in the same manner as in Preparation Example A46, thereby obtaining the title compound (2.8 g, 97%).
¹H NMR (400MHz, CDCl₃) δ 3.78 (t, 2H), 3.73 (m, 2H), 3.71 (s, 3H), 3.59 (br s, 2H), 2.61 (t, 2H), 2.31 (br, 1H; MS (ESI): m/z 149.08 [M+H]+)

### Preparation Example A62: Methyl 3-(2-((7-nitro-2-phenyl-1H-indol-5-yl)methoxy)ethoxy)propanoate

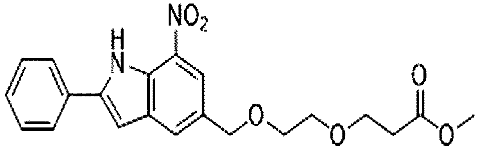

¹H NMR (400MHz, CDCl₃) δ 10.08 (br s, 1H), 8.14 (s, 1H), 7.95 (s, 1H), 7.74 (d, 2H), 7.51 (t, 2H), 7.40 (t, 1H), 6.93 (s, 1H), 4.71 (s, 2H), 3.80 (t, 2H), 3.69 (s, 7H), 2.65 (t, 2H; MS (ESI): m/z 399.45 [M+H]+)

### Preparation Example A63: 3-(2-((7-nitro-2-phenyl-1H-indol-5-yl)methoxy)ethoxy)propan-1-ol

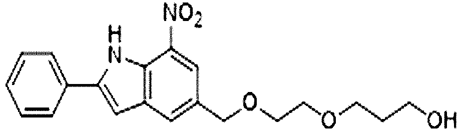

The methyl 3-(2-((7-nitro-2-phenyl-1H-indol-5-yl)methoxy)ethoxy)propanoate (258 mg, 0.647 mmol) obtained in Preparation Example A62 and anhydrous THF (8 mL) were added and cooled to -5 °C, and then 2M LiAlH₄/THF (0.65 mL, 1.30 mmol) was slowly added dropwise. After stirring for 2 hours at 0 °C and confirming the termination of the reaction by TLC, a Ruchell's salt (potassium sodium tartrate) aqueous solution was added at 0 °C to terminate the reaction. The temperature was raised to room temperature, EA and water were added, and the resulting product was dried over MgSO₄ and filtered. The filtrate was concentrated under reduced pressure and purified through silica gel column chromatography, thereby obtaining the title compound (89 mg, 38%).
¹H NMR (400MHz, CDCl₃) δ 10.08 (br s, 1H), 8.15 (s, 1H), 7.96 (s, 1H), 7.75 (d, 2H), 7.51 (t, 2H), 7.43 (t, 1H), 6.93 (s, 1H), 4.71 (s, 2H), 3.81 (t, 2H), 3.71 (t, 2H), 3.68 (s, 4H), 1.87 (m, 2H; MS (ESI): m/z 371.46 [M+H]+)

### Preparation Example A64: 3-(2-((7-amino-2-phenyl-1H-indol-5-yl)methoxy)ethoxy)propan-1-ol

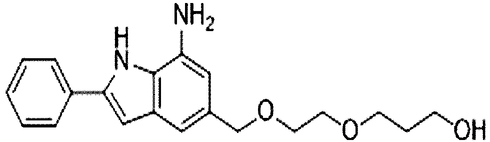

¹H NMR (400MHz, CDCl₃) δ 8.96 (br s, 1H), 7.70 (d, 2H), 7.42 (t, 2H), 7.30 (t, 1H), 7.07 (s, 1H), 6.71 (s, 1H), 6.63 (s, 1H), 4.52 (s, 2H), 3.77 (br s, 2H), 3.68 (br s, 2H), 3.64-3.61 (m, 4H), 1.82 (m, 2H; MS (ESI): m/z 341.18 [M+H]+)

### Preparation Example A65: 4-((7-nitro-2-phenyl-1H-indol-5-yl)methoxy)cyclohexan-1-ol

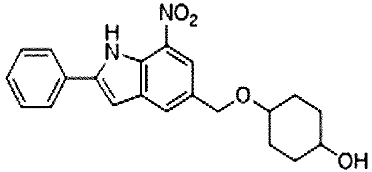

The 5-(bromomethyl)-7-nitro-2-phenyl-1H-indole (300 mg, 0.769 mmol) obtained in Preparation Example A8 and 1,4-cyclohexanediol (6 g) were added and refluxed. After cooling, EA and a saturated NaHCO₃ solution were added to the reaction solution, and the resulting solution was dried over MgSO₄ and filtered. The filtrate was concentrated under reduced pressure and purified through silica gel column chromatography, thereby obtaining the title compound (223 mg, crude; MS (ESI): m/z 376.16 [M+H]+).

### Preparation Example A66: 4-((7-amino-2-phenyl-1H-indol-5-yl)methoxy)cyclohexan-1-ol

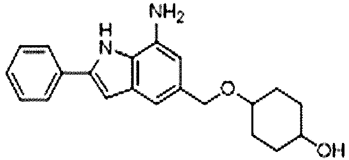

¹H NMR (400MHz, CDCl₃) δ 8.30 (br s, 1H), 7.68 (d, 2H), 7.43 (t, 3H), 7.30 (t, 1H), 7.13 & 7.12 (two s, 1H), 6.78 (s, 1H), 6.66 & 6.63 (two s, 1H), 4.56 & 4.54 (two s, 2H), 3.75-3.41 (m, 2H), 2.08-1.27 (m, 8H).

### Preparation Example A67: 4-hydroxy-4-methylcyclohexan-1-one

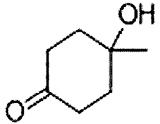

Commercially available 1,4-dioxaspiro[4,5]decan-8-one (10 g, 64.03 mmol) was dissolved in THF (200 mL) and cooled to -5 °C. After adding 3M MeMgBr/ether (27.7 mL, 83.23 mmol) dropwise, the resulting solution was heated to room temperature and stirred for 3 hours. After cooling to 0 °C, the reaction was terminated with a saturated NH₄Cl solution, and EA was added for extraction at room temperature. After drying over MgSO₄ and filtration, the filtrate was concentrated and diluted with THF (200 mL) and water (100 mL), and then stirred 2N HCl (200 mL) was added and then the resulting solution was stirred. After neutralization with a saturated NaHCO₃ solution, the resulting solution was extracted with EA, dried over MgSO₄, and filtered, thereby obtaining a filtrate. The filtrate was concentrated under reduced pressure and purified through silica gel column chromatography, thereby obtaining the title compound (2.5 g, 18%).

¹H NMR (400MHz, CDCl₃) δ 2.72 (m, 2H), 2.26 (d, 2H), 1.97 (m, 2H), 1.86 (m, 2H), 1.38 (s, 3H).

### Preparation Example A68: 1-methylcyclohexane-1,4-diol}

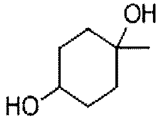

The 4-hydroxy-4-methylcyclohexan-1-one (2.5 g, 19.5 mmol) obtained in Preparation Example A67 was dissolved in MeOH (37 mL) and cooled to 0 °C, NaBH₄ (1.47 g, 39 mmol) was added, and the resulting mixture was heated to room temperature and stirred overnight. Water was added to terminate the reaction, and EA was added to perform extraction 10 or more times. The resulting extract was dried over MgSO₄ and filtered, and then the filtrate was concentrated under reduced pressure and purified through silica gel column chromatography, thereby obtaining the title compound (2.2 g, 86%).

¹H NMR (400MHz, CDCl₃) δ 3.61 (m, 1H), 1.77 (m, 2H), 1.67 (m, 4H), 1.45 (m, 2H), 1.23 (s, 3H).

### Preparation Example A69: 1-methyl-4-((7-nitro-2-phenyl-1H-indol-5-yl)methoxy)cyclohexan-1-ol

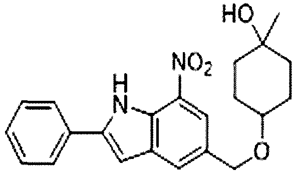

MS (ESI): m/z 381.4 [M+H]+

### Preparation Example A70: 4-((7-amino-2-phenyl-1H-indol-5-yl)methoxy)-1-methylcyclohexan-1-ol

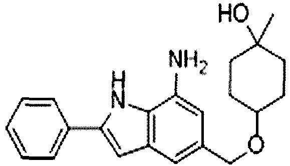

MS (ESI): m/z 351.2 [M+H]+

### Preparation Example A71: 4-(2-((tetrahydro-2H-pyran-2-yl)oxy)ethoxy)cyclohexan-1-ol

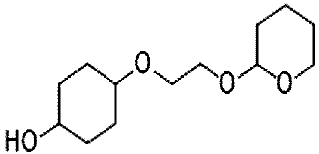

2-(2-bromoethoxy)tetrahydro-2H-pyran (5 g, 23.9 mmol), tetra-n-butylammonium hydrogen sulfate (1.623 g, 4.78 mmol), 1,4-cyclohexanediol (13.88 g, 119.5 mmol), and 50% NaOH/toluene (1/4, 50 mL) were added, heated to 40 °C, and stirred overnight. The resulting mixture was extracted with toluene for approximately 15 times, dried over MgSO₄, and filtered. The filtrate was concentrated under reduced pressure and purified through silica gel column chromatography, thereby obtaining the title compound (2.5 g, 45%; MS (ESI): m/z 245.17 [M+H]+).

### Preparation Example A72: 4-(2-hydroxyethoxy)cyclohexan-1-ol

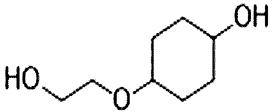

The 4-(2-((tetrahydro-2H-pyran-2-yl)oxy)ethoxy)cyclohexan-1-ol (2.8 g, 11.46 mmol) obtained in Preparation Example A71 was diluted in MeOH (30 mL), 3M HCl in MeOH (4 mL) was added, and then the resulting mixture was stirred for 2 hours at room temperature. The reaction solution was concentrated and purified through silica gel column chromatography, thereby obtaining the title compound (1.3 g, 71%; MS (ESI): m/z 161.11 [M+H]+).

### Preparation Example A73: 4-(2-((7-nitro-2-phenyl-1H-indol-5-yl)methoxy)ethoxy)cyclohexan-1-ol)

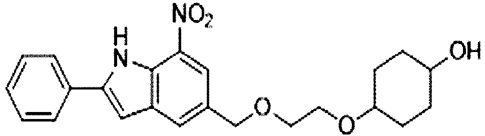

¹H NMR (400MHz, CDCl₃) δ 10.08 (br s, 1H), 8.16 (two s, 1H), 7.96 (two s, 1H), 7.75 (d, 2H), 7.51 (t, 2H), 7.40 (t, 1H), 6.93 (s, 1H), 4.74 & 4.72 (two s, 2H), 3.76-3.34 (m, 6H), 2.04-1.30 (m, 8H; MS (ESI): m/z 411.4 [M+H]+)

### Preparation Example A74: 4-(2-((7-amino-2-phenyl-1H-indol-5-yl)methoxy)ethoxy)cyclohexan-1-ol

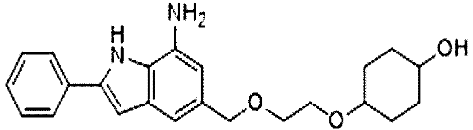

¹H NMR (400MHz, CDCl₃) δ 8.50 (br s, 1H), 7.71 (d, 2H), 7.44 (t, 2H), 7.30 (t, 1H), 7.13 & 7.11 (two s, 1H), 6.77 (s, 1H), 6.65 & 6.61 (two s, 1H), 4.58 & 4.47 (two s, 2H), 4.04 (m, 2H), 3.75-3.67 (m, 2H), 3.53 (t, 2H), 2.13-1.25 (m, 8H; MS (ESI): m/z 381.21 [M+H]+)

### Preparation Example A75: 5-((2-bromoethoxy)methyl)-7-nitro-2-phenyl-1H-indole

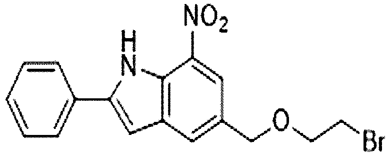

The 2-((7-nitro-2-phenyl-1H-indol-5-yl)methoxy)ethan-1-ol (0.4 g, 1.28 mmol) obtained in Preparation Example A8 was dissolved in THF, and cooled to 0 °C. Triphenyl phosphine (739 mg, 2.8 mmol) and CBr₄ (849 mg, 2.56 mmol) were added and allowed to react for 1 hour at room temperature. The resulting mixture was extracted with EA and water, dried over MgSO₄, filtered, concentrated under reduced pressure, and purified through silica gel column chromatography, thereby obtaining the title compound (340 mg, 71%).

### Preparation Example A76: 5-((2-fluoroethoxy)methyl)-7-nitro-2-phenyl-1H-indole

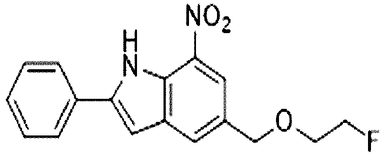

The 5-((2-bromoethoxy)methyl)-7-nitro-2-phenyl-1H-indole (0.3 g, 0.8 mmol) obtained in Preparation Example A75 was put into a sealed flask and ethylene glycol was added. KF (23 mg, 0.4 mmol) was added, and then the reaction was carried out for 15 hours at 110 °C. The resulting reaction product was cooled to room temperature, extracted with EA and water, dried over MgSO₄, filtered and concentrated under reduced pressure, and the concentrated residue was purified through silica gel column chromatography, thereby obtaining the title compound (25 mg, 10%).

### Preparation Example A77: 5-((2-fluoroethoxy)methyl)-2-phenyl-1H-indol-7-amine

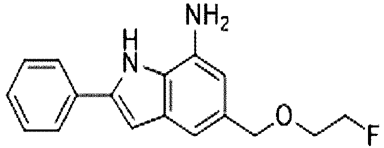

### Preparation Example A78: 5-((2,2-difluoroethoxy)methyl)-7-nitro-2-phenyl-1H-indol

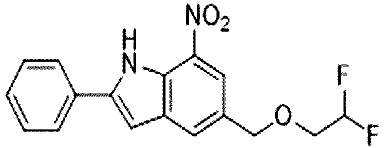

The 5-(bromomethyl)-7-nitro-2-phenyl-1H-indole (0.8 g, 2.4 mmol) obtained in Preparation Example A8 and N,N-dimethylacetate were put into a sealed tube, 2,2-difluoroethanol (1.52 mL, 24.15 mmol) and DIPEA (2.08 mL, 12.08 mmol) were added, and the reaction was carried out for 15 hours at 100 °C. EA and water were extracted, dried over MgSO₄, filtered and concentrated under reduced pressure, and the concentrated residue was purified through silica gel column chromatography to the title compound (40 mg, 5%) as a yellow solid.

### Preparation Example A79: 5-((2,2-difluoroethoxy)methyl)-2-phenyl-1H-indol-7-amine

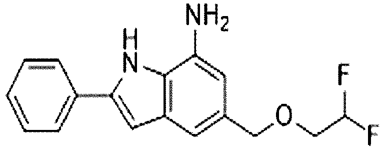

### Preparation Example A80: 2-((7-(cyclopentylamino)-2-phenyl-1H-indol-5-yl)methoxy)ethyl methanesulfonate

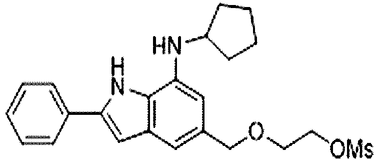

The 2-((7-(cyclopentylamino)-2-phenyl-1H-indol-5-yl)methoxy)ethan-1-ol (40 mg, 0.114 mmol) obtained in Example 26 was added to DCM (0.6 mL), and TEA (0.019 mL, 0.137 mmol) was added. MsCl (0.097 mL, 0.125 mmol) was added dropwise at 0 °C, and the reaction was carried out for 30 minutes at room temperature. Extraction was performed with DCM and water, and an organic layer was washed with a saturated NaCl solution, dried over MgSO₄, filtered, but concentrated under reduced pressure. The concentrated residue was purified through silica gel column chromatography to obtain the title compound (31 mg, 65%; MS (ESI): m/z 429.2 [M+H]+).

### Preparation Example A81: 2-((2-phenyl-7-((tetrahydro-2H-pyran-4-yl)amino)-1H-indol-5-yl)methoxy)ethyl methanesulfonate

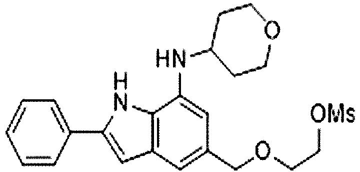

The 2-((2-phenyl-7-((tetrahydro-2H-pyran-4-yl)amino)-1H-indol-5-yl)methoxy)ethan-1-ol (214 mg, 0.585 mmol) obtained in Example 27 and MsCl (0.049 mL, 0.64 mmol) were treated in the same manner as in Preparation Example A80 to obtain the title compound (30 mg, 11%; MS (ESI): m/z 445.2 [M+H]+).

### Preparation Example A82: 5-((2-bromoethoxy)methyl)-N-cyclopentyl-2-phenyl-1H-indol-7-amine

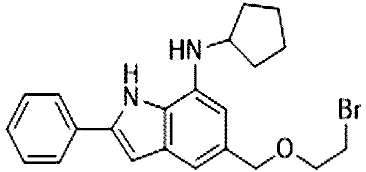

The 2-((7-(cyclopenylamino)-2-phenyl-1H-indol-5-yl)methoxy)ethan-1-ol (70 mg, 0.1997 mmol) obtained in Example 26 was treated in the same manner as in Preparation Example A8, thereby obtaining the title compound (50 mg, 60%; MS (ESI): m/z 414.3 [M+H]+).

### Preparation Example A83: 5-((2-bromoethoxy)methyl)-2-phenyl-N-(tetrahydro-2H-pyran-4-yl)-1H-indol-7-amine

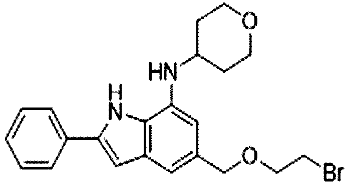

The 2-((2-phenyl-7-((tetrahydro-2H-pyran-4-yl)amino)-1H-indol-5-yl)methoxy)ethan-1-ol (100 mg, 0.273 mmol) obtained in Example 27 was treated in the same manner as in Preparation Example A8, thereby obtaining the title compound (140 mg; MS (ESI): m/z 430.1 [M+H]+).

### Preparation Example A84: Tert-butyl (2-hydroxyethyl)(2-((7-nitro-2-phenyl-1H-indol-5-yl)methoxy)ethyl)carbamate

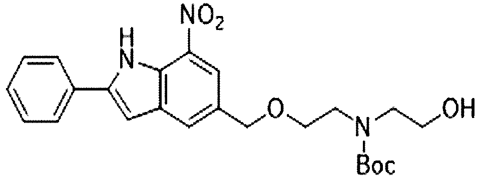

The compound of Preparation Example A8 (500 mg, 1.51 mmol) and tert-butyl bis(2-hydroxyethyl)carbamate (3.1 g, 15.1 mmol) was dissolved in THF (2.86 ml), added to a sealed tube, and the reaction was carried out for 3 days at 70 °C. The resulting mixture was cooled to room temperature, EA and water were added to extract an organic layer, and the organic layer was dried over MgSO₄ and filtered. The filtrate was purified through MPLC to obtain the title compound (330mg, 480 mmol; MS (ESI): m/z 456.2 [M+H]+).

### Preparation Example A85: Tert-butyl (2-((7-amino-2-phenyl-1H-indol-5-yl)methoxy)ethyl)(2-hydroxyethyl)carbamate

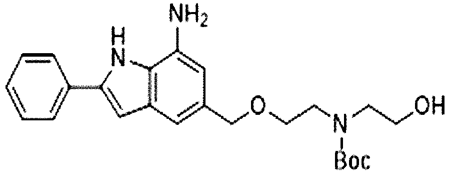

The compound of Preparation Example A84 (330 mg, 0.724 mmol) was dissolved in a mixed solution of EA (10 mL) and MeOH (10 mL), 50% wet 10% Pd/C (300 mg, 0.50 wt%) was added, and then a hydrogen balloon was inserted and stirred at room temperature for 3 hours. After filtering through Celite and washing with EA, the filtrate was concentrated under reduced pressure to obtain the title compound (270 mg, 87%; MS (ESI): m/z 426.2 [M+H]+).

### Preparation Example A86: 2-((2-((7-nitro-2-phenyl-1H-indol-5-yl)methoxy)ethyl)amino)ethan-1-ol

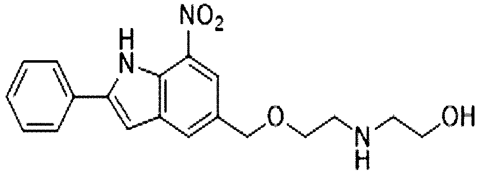

The compound of Preparation Example A84 (200 mg, 0.44 mmol) was dissolved in DCM (10 mL), TFA (2 mL) was added, and the resulting mixture was stirred overnight. The reaction solution was concentrated, EA and saturated Na₂CO₃ were added to extract an organic layer. The organic layer was dried over MgSO₄ and filtered. The filtrated was purified through MPLC to obtain the title compound (60 mg, 38%) as a yellow foam (MS (ESI): m/z 356.1 [M+H]+).

### Preparation Example A87: 2-(methyl(2-((7-nitro-2-phenyl-1H-indol-5-yl)methoxy)ethyl)amino)ethan-1-ol

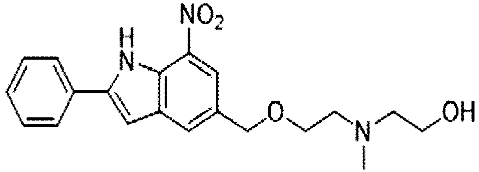

The compound of Preparation Example A86 (60 mg, 0.1689 mmol) was dissolved in ACN (5 mL) and cooled to 0 °C. Formaldehyde 37% in H₂O (2 mL) and NaBH(OAc)₃ (358 mg, 1.689 mmol) were added. The resulting mixture was heated to room temperature and stirred overnight. A saturated NaHCO₃ aqueous solution was added, and the resulting solution was stirred for 30 minutes and extracted with MC. MPLC purification was carried out to obtain the title compound (36 mg, 58%) as a yellow solid (MS (ESI): m/z 370.1 [M+H]+).

### Preparation Example A88: 2-(2-hydroxyethoxy)ethyl 4-methylbenzenesulfonate

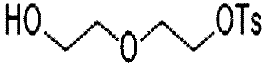

Ethylene glycol (5 g, 47.1 mmol) was diluted in DCM (50 mL), TEA (13.24 g, 94.2 mmol) was added, and the resulting mixture was cooled to 0 °C. p-toluenesulfonyl chloride (9 g, 47.1 mmol) was added, and the resulting mixture was heated to room temperature and stirred overnight. After adding EA and water to the reaction solution for extraction, an aqueous layer was washed with a saturated NaCl solution, dried over MgSO₄, filtered and concentrated under reduced pressure. The concentrated residue was purified through silica gel column chromatography, thereby obtaining the title compound (3.1 g, 25%; MS (ESI): m/z 261.0 [M+H]+).

### Preparation Example A89: 2-(2-(methylamino)ethoxy)ethan-1-ol

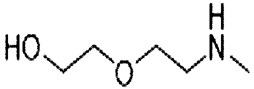

The compound of Preparation Example A88 (1 g, 3.84 mmol) was diluted in ethanol (1 mL), 33% methylamine/ethanol (42.5 mmol) was added, and the resulting mixture was stirred overnight at 80 °C in a sealed tube. After concentration and drying, the resulting product was used in a subsequent reaction without additional purification (MS (ESI): m/z 120.1 [M+H]+).

### Preparation Example A90: 2-(2-methoxyethoxy)-N-methylethan-1-amine

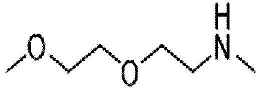

1-bromo-2-(2-methoxyethoxy)ethane (1 g, 5.46 mmol) was reacted and treated in the same manner as in Preparation Example A89 and used in a subsequent reaction without purification (MS (ESI): m/z 134.1 [M+H]+).

### Preparation Example A91: 2-(2-methoxyethoxy)ethan-1-amine

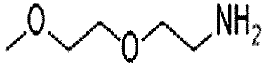

2-(2-methoxyethoxy)ethan-1-ol was reacted and treated in the same manner as in Preparation Examples A88 and A89 to obtain an orange oil, and then used in a subsequent reaction without purification (MS (ESI): m/z 120.1 [M+H]+).

### Preparation Example A92: Ethyl (2-methoxyethyl)glycinate

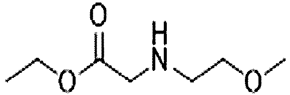

2-methoxyethan-1-amine (10 g, 133 mmol), EA (100 mL), and TEA (11.2 g, 111 mmol) were added and ethyl 2-bromoacetate (18.5 g, 111 mmol) diluted in EA (50 mL) was added dropwise. After stirring for 4 hours at room temperature, the reaction solution was filtered and washed with EA, and the filtrate was concentrated. The concentrated residue was purified through silica gel column chromatography to obtain the title compound (14 g, 79%; MS (ESI): m/z 162.11 [M+H]+).

### Preparation Example A93: Ethyl N-(tert-butoxycarbonyl)-N-(2-methoxyethyl)glycinate

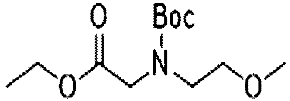

The compound of Preparation Example A92 (1 g, 6.2 mmol) was diluted in DCM (10 mL), TEA (1.88 g, 18.6 mmol) and (Boc)₂O (2.7 g, 12.4 mmol) were added, and the resulting mixture was stirred for 4 hours at room temperature. The reaction solution was concentrated and purified through a silica gel column chromatography, thereby obtaining the title compound (1.6 g, quant.) as a colorless oil (MS (ESI): m/z 262.1 [M+H]+).

### Preparation Example A94: Tert-butyl (2-hydroxyethyl)(2-methoxyethyl)carbamate

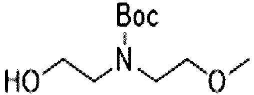

The compound of Preparation Example A93 (1.6 g, 6.12 mmol) was dissolved in THF (16 mL) and dissolved in ethanol (16 mL), and NaBH₄ (1.16 g, 30.6 mmol) was added dropwise. The resulting mixture was stirred overnight, MeOH and water were added to terminate the reaction. The resulting solution was extracted with EA, an organic layer was dried over MgSO₄, filtered and concentrated. The concentrated residue was purified through column chromatography to obtain the title compound (1.2 g, 90%; (MS (ESI): m/z 220.1 [M+H]+).

### Preparation Example A95: Tert-butyl (2-methoxyethyl)(2-((2-phenyl-7-((tetrahydro-2H-pyran-4-yl)amino)-1H-indol-5-yl)methoxy)ethyl)carbamate

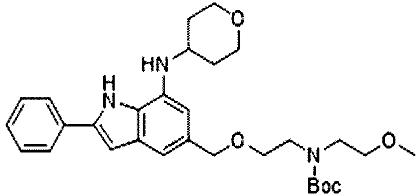

The compound of Preparation Example A94 was treated in the same manner as in Preparation Example A1, and reacted and treated in the same manner as in Preparation Example A2 and Example 2, thereby obtaining the title compound (153 mg) as a white solid (MS (ESI): m/z 524.3 [M+H]+).

### Preparation Example A96: N-(2-hydroxyethyl)methanesulfonamide

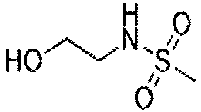

2-aminoethan-1-ol (5 g, 81.86 mmol) was diluted in DCM (70 mL) and cooled to -10 °C, and TEA (3.45 mL, 24.558 mmol) was added. A methanesulfonyl chloride (1.9 mL, 24.558 mmol) solution diluted in DCM (19 mL) was slowly added dropwise at -10 °C, heated to room temperature, and stirred overnight. DCM and water were added to the reaction solution for extraction, and an organic layer was dried over MgSO₄, filtered and concentrated. The concentrated residue was purified through silica gel column chromatography to obtain the title compound (1.58 g, 33%; MS (ESI): m/z 141.0 [M+H]+).

### Preparation Example A97: N-(2-((7-nitro-2-phenyl-1H-indol-5-yl)methoxy)ethyl)methanesulfonamide

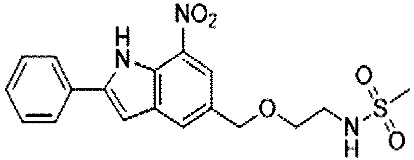

The compound of Preparation Example A96 (780mg, 5.60 mmol) was added to THF (1 mL), the compound of Preparation Example A8 (250 mg, 0.755 mmol) and DIPEA (117 mg, 0.906 mmol) were added, the resulting mixture was put into a sealed tube, heated in a 100 °C bath, and stirred for two days. After cooling, EA and water were added, diluted, and extracted, and an organic layer was dried over MgSO₄, filtered and concentrated. The concentrated residue was purified through silica gel column chromatography to obtain (74 mg, 25%; MS (ESI): m/z 390.1 [M+H]+).

The compounds of Examples 1 to 61 were prepared using the compounds of Preparation Examples.

### Example 1: 7-(cyclopentylamino)-N,N-diethyl-2-phenyl-1H-indol-5-carboxamide

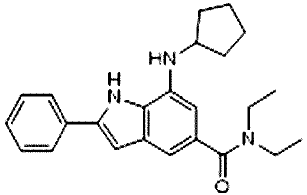

After dissolving cyclopentanone (13.68 mg, 162.66 µmol) in methanol (2 mL), the 7-amino-N,N-diethyl-2-phenyl-1H-indol-5-carboxamide (50 mg, 162.66 µmol) obtained in Preparation Example A2, NaBH₃CN (20.44 mg, 325.32 µmol) and AcOH (9.77 mg, 162.66 µmol) were added and stirred for 2 hours at room temperature. After confirming the termination of the reaction by LCMS, EA and H₂O were added for extraction, and an organic layer was washed with a saturated NaCl solution, dried over MgSO₄, and filtered. The filtrate was concentrated under reduced pressure and purified through silica gel column chromatography, thereby obtaining the title compound (21.7 mg, 34.7%) as a white solid.
¹H NMR (400 MHz, DMSO-d₆) δ 11.07 (br s, 1H), 7.87 - 7.74 (d, J=7.2Hz, 2H), 7.52 - 7.44 (t, J=7.6Hz, 2H), 7.36 - 7.29 (t, J=7.2Hz, 1H), 6.85 - 6.80 (d, J=2Hz, 1H), 6.77 (s, 1H), 6.15 (s, 1H), 5.62 - 5.52 (d, J=6Hz, 1H), 3.94 - 3.84 (m, 1H), 3.37 - 3.34 (m, 4H), 2.08 - 1.97 (m, 2H), 1.81 - 1.71 (m, 2H), 1.67 - 1.51 (m, 4H), 1.17 - 1.04 (t, J=6.8Hz, 6H; MS (ESI): m/z 376.1 [M+H]+)

### Example 2: N,N-diethyl-2-phenyl-7-((tetrahydro-2H-pyran-4-yl)amino)-1H-indol-5-carboxamide

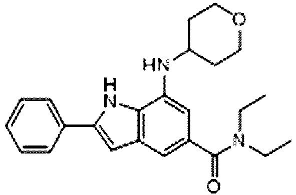

The 7-amino-N,N-diethyl-2-phenyl-1H-indol-5-carboxamide (50 mg, 162.66 µmol) obtained in Preparation Example A2 and tetrahydro-4H-pyran-4-one (16.28 mg, 162.66 µmol) were treated in the same manner as in Example 1, thereby obtaining the title compound (39.3 mg, 58.19%) as a yellow solid.
¹H NMR (400 MHz, DMSO-d₆) δ 11.00 (br s, 1H), 7.85 - 7.78 (d, J=7.6Hz, 2H), 7.53 - 7.45 (t, J=7.6Hz, 2H), 7.37 - 7.29 (t, J=7.2Hz, 1H), 6.84 (s, 1H), 6.78 (s, 1H), 6.24 (s, 1H), 5.55 - 5.48 (d, J=7.6Hz, 1H), 3.97 - 3.88 (m, 2H), 3.73 - 3.61 (m, 1H), 3.54 - 3.45 (m, 4H), 3.36 - 3.35 (m, 2H), 2.08 - 2.00 (m, 2H), 1.53 - 1.41 (m, 2H), 1.17-1.07 (m, 6H; MS (ESI): m/z 392.2 [M+H]+)

The example compounds in Table 2 below were prepared in a similar manner to Examples 1 and 2. The chemical structures, compound names, NMR and LCMS analysis results of each example compound are shown in Table 2.

**[Table 2]**

| **Exam ple No.** | **Structure** | **Compound Name** | **¹H NMR; MS[M+H]+** |
|---|---|---|---|
| 3 | 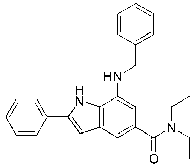 | 7-(benzylamino)-N,N-diethyl-2-phenyl-1H-indole-5-carboxamide | ¹H NMR (400 MHz, DMSO-*d₆*) δ 11.20 (br s, 1H), 7.84 - 7.78 (d, J=7.6Hz, 2H), 7.52 - 7.43 (m, 4H), 7.41 - 7.25 (m, 4H), 6.89 - 6.83 (m, 1H), 6.80 (s, 1H), 6.18 - 6.07 (m, 2H), 4.49 - 4.42 (d, J=5.2Hz, 2H), 3.32 - 3.18 (m, 4H), 1.14 (br s, 6H); MS (ESI): m/z 398.1 [M+H]+ |
| 4 | 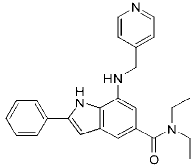 | N,N-diethyl-2-phenyl-7-((pyridin-4-ylmethyl)amino)-1H-indole-5-carboxamide | ¹H NMR (400 MHz, DMSO-*d₆*) δ 11.22 (br s, 1H), 8.58 - 8.42 (d, J=5.6Hz, 2H), 7.90 - 7.74 (d, J=7.6Hz, 2H), 7.53 - 7.46 (t, J=7.6Hz, 2H), 7.46 - 7.41 (d, J=5.6Hz, 2H), 7.36 - 7.29 (t, J=7.2Hz, 1H), 6.91 - 6.84 (m, 1H), 6.81 (s, 1H), 6.36 - 6.30 (t, J=5.2Hz, 1H), 6.01 (s, 1H), 4.61 - 4.53 (d, J=5.6Hz, 2H), 3.28 - 3.09 (m, 4H), 0.94 (br s, 6H); MS (ESI): m/z 399.1 [M+H]+ |
| 5 | 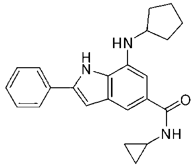 | 7-(cyclopentylamino)-N-cyclopropyl-2-phenyl-1H-indole-5-carboxamide | ¹H NMR (400 MHz, DMSO-*d₆*) δ 11.10 (br s, 1H), 8.21 - 8.11 (d, J=4Hz, 1H), 7.87 - 7.76 (d, J=7.2Hz, 2H), 7.53 - 7.42 (t, J=7.6Hz, 2H), 7.38 - 7.28 (m, 2H), 6.90 - 6.81 (d, J=2Hz, 1H), 6.74 (s, 1H), 5.56 - 5.40 (d, J=6.4Hz, 1H), 4.03 - 3.89 (m, 1H), 2.88 - 2.76 (m, 1H), 2.12 - 2.01 (m, 2H), 1.82 - 1.70 (m, 2H), 1.68 - 1.49 (m, 4H), 0.71 - 0.62 (m, 2H), 0.61 - 0.51 (m, 2H); MS (ESI): m/z 360.1 [M+H]+ |
| 6 | 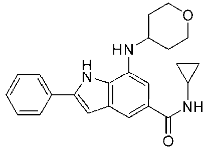 | N-cyclopropyl-2-phenyl-7-((tetrahydro-2H-pyran-4-yl)amino)-1H-indole-5-carboxamide | ¹H NMR (400 MHz, DMSO-*d₆*) δ 11.15 (br s, 1H), 8.23 - 8.17 (d, J=4Hz, 1H), 7.86 - 7.78 (d, J=7.2Hz, 2H), 7.52 - 7.44 (t, J=7.2Hz, 2H), 7.39 - 7.29 (m, 2H), 6.89 - 6.84 (d, J=1.6Hz, 1H), 6.78 (s, 1H), 5.48 - 5.38 (d, J=7.2Hz, 1H), 3.99 - 3.90 (m, 2H), 3.75 - 3.64 (m, 2H), 3.36 - 3.27 (m, 1H), 2.86 - 2.77 (m, 1H), 2.10 - 2.00 (m, 2H), 1.54 - 1.40 (m, 2H), 0.72 - 0.63 (m, 2H), 0.59 - 0.53 (m, 2H); MS (ESI): m/z 376.1 [M+H]+ |
| 7 | 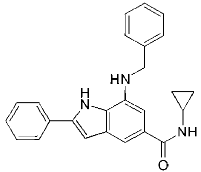 | 7-(benzylamino)-N-cyclopropyl-2-phenyl-1H-indole-5-carboxamide | ¹H NMR (400 MHz, DMSO-*d₆*) δ 11.23 (br s, 1H), 8.19 - 8.12 (d, J=4.4Hz, 1H), 7.85 - 7.78 (d, J=7.6Hz, 2H), 7.51 - 7.44 (t, J=8Hz, 4H), 7.42 - 7.36 (t, J=7.6Hz, 3H), 7.35 - 7.27 (m, 2H), 6.92 - 6.88 (d, J=1.6Hz, 1H), 6.80 (s, 1H), 6.03 - 5.95 (t, J=5.6Hz, 1H), 4.52 - 4.44 (d, J=5.2Hz, 2H), 2.85 - 2.77 (m, 1H), 0.70 - 0.62 (m, 2H), 0.59 - 0.52 (m, 2H); HPLC: 94.19%, MS (ESI): m/z 382.1 [M+H]+ |
| 8 | 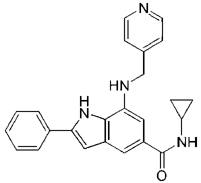 | N-cyclopropyl-2-phenyl-7-((pyridin-4-ylmethyl)amino)-lH-indole-5-carboxamide | ¹H NMR (400 MHz, DMSO-*d₆*) δ 11.60 (br s, 1H), 8.59 - 8.52 (m, 2H), 8.20 - 8.12 (d, J=4Hz, 1H), 7.98 - 7.89 (d, J=4Hz, 2H), 7.54 - 7.46 (m, 4H), 7.42 (s, 1H), 7.38 - 7.32 (t, J=7.6Hz, 1H), 6.96 - 6.91 (d, J=2Hz, 1H), 6.68 (s, 1H), 6.65 - 6.58 (m, 1H), 4.62 - 4.55 (d, J=6Hz, 2H), 2.85 - 2.76 (m, 1H), 0.70 - 0.63 (m, 2H), 0.59 - 0.53 (m, 2H); MS (ESI): m/z 383.1 [M+H]+ |
| 9 | 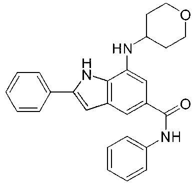 | N,2-diphenyl-7-((tetrahydro-2H-pyran-4-yl)amino)-1H-indole-5-carboxamide | ¹H NMR (400 MHz, DMSO-*d₆*) δ11.25 (br s, 1H), 10.00 (s, 1H), 7.86 (d, J=7.6Hz, 2H), 7.79 (d, J=7.6Hz, 2H), 7.56 (s, 1H), 7.48 - 7.53 (m, 2H), 7.31 - 7.38 (m, 3H), 7.03 - 7.09 (m, 1H), 6.96 (s, 1H), 6.89 (s, 1H), 5.56 (d, J=6.4Hz, 1H), 3.96 (d, J=11.6Hz, 2H), 3.71 - 3.80 (m, 1H), 3.53 (t, J=10.0Hz, 2H), 2.09 (d, J=12.4Hz, 2H), 1.44 - 1.57 (m, 2H); MS (ESI): m/z 412.2 [M+H]+ |
| 10 | 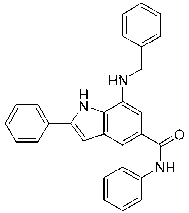 | 7-(benzylamino)-N,2-diphenyl-1H-indole-5-carboxamide | ¹H NMR (400 MHz, DMSO-*d₆*) δ11.35 (br s, 1H), 10.01 (s, 1H), 7.85 (d, J=7.2Hz, 2H), 7.79 (d, J=8.0Hz, 2H), 7.62 (s, 1H), 7.46 - 7.53 (m, 4H), 7.37 - 7.43 (m, 2H), 7.29 - 7.37 (m, 4H), 7.05 (t, J=2.4Hz, 1H), 6.99 (s, 1H), 6.87 - 6.94 (s, 1H), 6.11 (t, J=5.2Hz, 1H), 4.50 - 4.57 (d, t, J=5.2Hz 2H); MS (ESI): m/z 418.0 [M+H]+ |
| 11 | 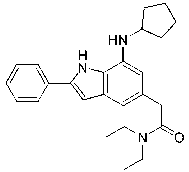 | 2-(7-(cyclopentylamino)-2-phenyl-1H-indol-5-yl)-N,N-diethylacetamide | ¹H NMR (400 MHz, DMSO-*d₆*) δ10.86 (br s, 1H), 7.87 - 7.72 (d, J=7.2Hz, 2H), 7.52 - 7.42 (t, J=8Hz, 2H), 7.33 - 7.26 (t, J=7.2Hz, 1H), 6.77 - 6.70 (d, J=2Hz, 1H), 6.65 (s, 1H), 6.16 (s, 1H), 5.45 - 5.36 (d, J=6.4Hz, 1H), 3.89 - 3.79 (m, 1H), 3.59 (s, 2H), 3.32 - 3.23 (m, 4H), 2.08 - 1.97 (m, 2H), 1.80 - 1.70 (m, 2H), 1.67 - 1.49 (m, 4H), 1.05 - 0.95 (m, 6H); MS (ESI): m/z 390.1 [M+H]+ |
| 12 | 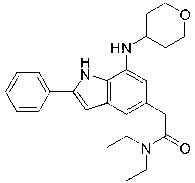 | N,N-diethyl-2-(2-phenyl-7-((tetrahydro-2H-pyran-4-yl)amino)-1H-indol-5-yl)acetamide | ¹H NMR (400 MHz, DMSO-*d₆*) δ10.89 (br s, 1H), 7.83 - 7.75 (d, J=7.2Hz, 2H), 7.51 - 7.43 (t, J=8Hz, 2H), 7.34 - 7.25 (t, J=7.6Hz, 1H), 6.76 - 6.70 (d, J=2Hz, 1H), 6.65 (s, 1H), 6.21 (s, 1H), 5.38 - 5.30 (d, J=7.6Hz, 1H), 3.98 - 3.89 (m, 2H), 3.63 - 3.52 (m, 3H), 3.51 - 3.42 (m, 2H), 3.32 - 3.21 (m, 4H), 2.09 - 2.00 (m, 2H), 1.54 - 1.38 (m, 2H), 1.06 - 0.92 (m, 6H); MS (ESI): m/z 406.1 [M+H]+ |
| 13 | 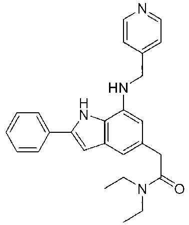 | N,N-diethyl-2-(2-phenyl-7-((pyridin-4-ylmethyl)amino)-1H-indol-5-yl)acetamide | ¹H NMR (400 MHz, DMSO-*d₆*) δ11.00 (s, 1H), 8.56 - 8.46 (m, 2H), 7.85 - 7.74 (m, 2H), 7.50 - 7.43 (m, 2H), 7.43 - 7.37 (m, 2H), 7.34 - 7.26 (m, 1H), 6.79 - 6.74 (m, 1H), 6.73 - 6.65 (m, 1H), 6.18 - 6.09 (m, 1H), 6.07 - 6.00 (m, 1H), 4.55 - 4.41 (m, 2H), 3.57 - 3.44 (m, 2H), 3.24 - 3.07 (m, 4H), 1.01 - 0.77 (m, 6H); MS (ESI): m/z 413.1 [M+H]+ |
| 14 | 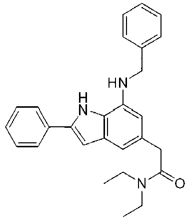 | 2-(7-(benzylamino)-2-phenyl-1H-indol-5-yl)-N,N-diethylacetamide | ¹H NMR (400 MHz, DMSO-*d₆*) δ11.35 (br s, 1H), 7.88 - 7.79 (d, J=7.6Hz, 2H), 7.52 - 7.41 (m, 4H), 7.40 - 7.24 (m, 4H), 6.87 (s, 1H), 6.83 - 6.77 (d, J=2Hz, 1H), 6.40 (s, 1H), 4.46 (s, 2H), 3.58 (s, 2H), 3.28 - 3.19 (m, 4H), 1.04 - 0.89 (m, 6H); MS (ESI): m/z 412.1 [M+H]+ |
| 15 | 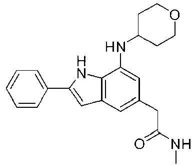 | N-methyl-2-(2-phenyl-7-((tetrahydro-2H-pyran-4-yl)amino)-1H-indol-5-yl)acetamide | ¹H NMR (400MHz, CDCl₃) δ 8.99 (br s, 1H), 7.71 (d, 2H), 7.43 (t, 2H), 7.30 (t, 1H), 6.94 (s, 1H), 6.74 (s, 1H), 6.36 (s, 1H), 5.68 (br, 1H), 4.04 (m, 2H), 3.64 (m, 1H), 3.63 (s, 2H), 3.53 (t, 2H), 2.74 (d, 3H), 2.10 (m, 2H), 1.63 (m, 2H); MS(ESI): m/z 364.2 [M+H]+ |
| 16 | 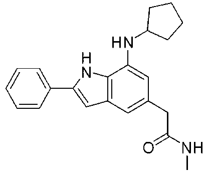 | 2-(7-(cyclopentylamino)-2-phenyl-1H-indol-5-yl)-N-methylacetamide | ¹H NMR (400MHz, CDCl₃) δ 9.23 (br s, 1H), 7.73 (d, 2H), 7.44 (t, 2H), 7.31 (t, 1H), 6.91 (s, 1H), 6.74 (s, 1H), 6.32 (s, 1H), 5.76 (br, 1H), 3.94 (m, 1H), 3.65 (s, 2H), 2.75 (d, 3H), 2.12 (m, 2H), 1.81 (m, 2H), 1.68-1.63 (m, 4H); MS(ESI): m/z 348.2 [M+H]+ |
| 17 | 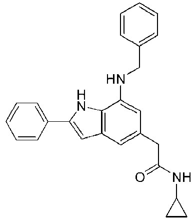 | 2-(7-(benzylamino)-2-phenyl-1H-indol-5-yl)-N-cyclopropylacetamide | ¹H NMR (400 MHz DMSO-*d₆*) δ11.00 (br s, 1H), 7.94 - 7.88 (d, J=4.0Hz, 1H), 7.82 - 7.75 (d, J=7.2Hz, 2H), 7.50 - 7.42 (m, 4H), 7.40 - 7.35 (t, J=7.2Hz, 2H), 7.32 - 7.25 (m, 2H), 6.78 - 6.73 (d, J=2.0Hz, 1H), 6.69 (s, 1H), 6.22 (s, 1H), 5.93 - 5.88 (t, J=5.2Hz, 1H), 4.43 - 4.37 (d, J=5.2Hz, 2H), 3.24 (s, 2H), 2.60 - 2.55 (m, 1H), 0.61 - 0.53 (m, 2H), 0.39 - 0.31 (m, 2H); MS (ESI): m/z 396.1 [M+H]+ |
| 18 | 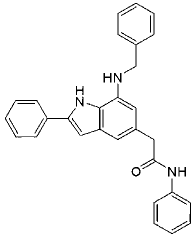 | 2-(7-(benzylamino)-2-phenyl-1H-indol-5-yl)-N-phenylacetamide | ¹H NMR (400 MHz, DMSO-*d₆*) δ10.99 (br s, 1H), 10.00 (s, 1H), 7.78 (d, J=7.6Hz, 2H), 7.58 (d, J=7.6Hz, 2H), 7.50 - 7.41 (m, 4H), 7.38 - 7.24 (m, 6H), 7.02 (t, J=7.6Hz, 1H), 6.81 (s, 1H), 6.74 (d, J=2.0Hz, 1H), 6.36 (s, 1H), 5.94 - 5.86 (m, 1H), 4.41 (d, J=5.6Hz, 2H), 3.53 (s, 2H); MS (ESI): m/z 432.1 [M+H]+ |
| 19 | 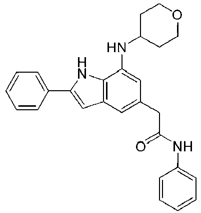 | N-phenyl-2-(2-phenyl-7-((tetrahydro-2H-pyran-4-yl)amino)-1H-indol-5-yl)acetamide | ¹H NMR (400 MHz, DMSO-*d₆*) δ10.89 (br s, 1H), 10.02 (s, 1H), 7.79 (d, J=7.2Hz, 2H), 7.59 (d, J=7.6Hz, 2H), 7.46 (t, J=7.6Hz, 2H), 7.33 - 7.24 (m, 3H), 7.01 (t, J=7.6Hz, 1H), 6.76 (d, J=1.2Hz,1H), 6.36 (s, 1H), 5.34 (d, J=7.6Hz, 1H), 3.94 (m, 2H), 3.67 - 3.58 (m, 1H), 3.54 (s, 2H), 3.51 - 3.43 (m, 2H), 2.10 - 2.02 (m, 2H), 1.51 - 1.39 (m, 2H); MS (ESI): m/z 426.1 [M+H]+ |
| 20 | 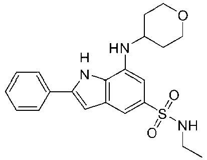 | N-ethyl-2-phenyl-7-((tetrahydro-2H-pyran-4-yl)amino)-1H-indole-5-sulfonamide | ¹H NMR (400 MHz, DMSO-*d₆*) δ11.35 (br s, 1H), 7.83 (d, J=7.6Hz, 2H), 7.49 (t, J=8.0Hz, 2H), 7.33 - 7.39 (m, 1H), 7.31 (s, 1H), 7.21 (t, J=5.6Hz, 1H), 6.95 - 6.99 (m, 1H), 6.67 (s, 1H), 5.76 (d, J=7.2Hz, 1H), 3.93 - 4.00 (m, 2H), 3.59 - 3.73 (m, 1H), 3.50 (t, J=12.0Hz, 2H), 2.64 - 2.76 (m, 2H), 2.04 - 2.13 (m, 2H), 1.43 - 1.57 (m, 2H), 0.93 (t, J=7.2Hz, 3H); MS (ESI): m/z 400.4 [M+H]+ |
| 21 | 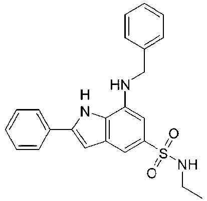 | 7-(benzylamino)-N-ethyl-2-phenyl-1H-indole-5-sulfonamide | ¹H NMR (400 MHz, DMSO-*d₆*) δ11.44 (br s, 1H), 7.82 (d, J=7.6Hz, 2H), 7.45 - 7.52 (m, 4H), 7.26 - 7.42 (m, 5H), 7.09 (t, J=6.0Hz, 1H), 6.97 - 7.00 (m, 1H), 6.64 (s, 1H), 6.32 (t, J=5.6Hz, 1H), 4.47 (d, J=5.2Hz, 2H), 2.56 - 2.66 (m, 2H), 0.89 (t, J=7.2Hz, 3H); MS (ESI): m/z 406.1 [M+H]+ |
| 22 | 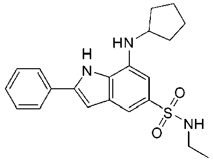 | 7-(cyclopentylamino)-N-ethyl-2-phenyl-1H-indole-5-sulfonamide | ¹H NMR (400 MHz, DMSO-*d₆*) δ11.31 (br s, 1H), 7.82 (d, J=7.6Hz, 2H), 7.50 (t, J=8.0Hz, 2H), 7.35 (t, J=6.0Hz, 1H), 7.30 (s, 1H), 7.18 (t, J=6.0Hz, 1H), 6.95 - 6.98 (m, 1H), 6.62 (s, 1H), 5.80 (d, J=6.4Hz, 1H), 3.84 - 3.96 (m, 1H), 2.68 - 2.77 (m, 2H), 2.04 - 2.15 (m, 2H), 1.73 - 1.83 (m, 2H), 1.54 - 1.70 (m, 4H), 0.95 (t, J=7.2Hz, 3H); MS (ESI): m/z 384.0 [M+H]+ |
| 23 | 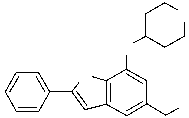 | 2-(2-phenyl-7-((tetrahydro-2H-pyran-4-yl)amino)-1H-indol-5-yl)acetonitrile | ¹H NMR (400MHz, CDCl₃) δ 8.41 (br s, 1H), 7.70 (d, 2H), 7.45 (t, 2H), 7.32 (t, 1H), 7.07 (s, 1H), 6.76 (s, 1H), 6.47 (s, 1H), 4.05 (m, 2H), 3.75 (s, 2H), 3.71 (m, 1H), 3.55 (t, 2H), 2.13 (m, 2H), 1.66 (m, 2H); MS (ESI): m/z 332.2 [M+H]+ |
| 24 | 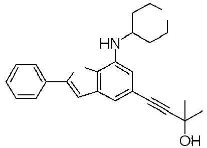 | 2-methyl-4-(2-phenyl-7-((tetrahydro-2H-pyran-4-yl)amino)-1H-indol-5-yl)but-3-yn-2-ol | ¹H NMR (400MHz, CDCl₃) δ 8.99 (br s, 1H), 7.71 (d, 2H), 7.43 (t, 2H), 7.30 (t, 1H), 6.94 (s, 1H), 6.74 (s, 1H), 6.36 (s, 1H), 5.68 (br, 1H), 4.04 (m, 2H), 3.64 (m, 1H), 3.63 (s, 2H), 3.53 (t, 2H), 2.74 (d, 3H), 2.10 (m, 2H), 1.63 (m, 2H); MS (ESI): m/z 375.2 [M+H]+ |
| 25 | 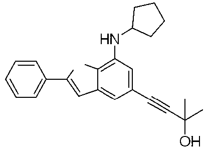 | 4-(7-(cyclopentylamino)-2-phenyl-1H-indol-5-yl)-2-methylbut-3-yn-2-ol | ¹H NMR (400MHz, CDCl₃) δ 8.99 (br s, 1H), 7.71 (d, 2H), 7.43 (t, 2H), 7.30 (t, 1H), 6.94 (s, 1H), 6.74 (s, 1H), 6.36 (s, 1H), 5.68 (br, 1H), 4.04 (m, 2H), 3.64 (m, 1H), 3.63 (s, 2H), 3.53 (t, 2H), 2.74 (d, 3H), 2.10 (m, 2H), 1.63 (m, 2H); MS (ESI): m/z 359.2 [M+H]+ |
| 26 | 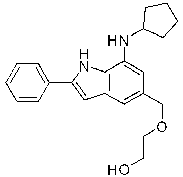 | 2-((7-(cyclopentylamino)-2-phenyl-1H-indol-5-yl)methoxy)ethan-1-ol | ¹H NMR (400MHz, CDCl₃) δ 8.37 (br s, 1H), 7.71 (d, 2H), 7.45 (t, 2H), 7.30 (t, 1H), 7.09 (s, 1H), 6.79 (s, 1H), 6.57 (s, 1H), 4.64 (s, 2H), 3.98 (m, 1H), 3.78 (t, 2H), 3.66 t, 2H), 2.13 (m, 2H), 1.81 (m, 2H), 1.68-1.63 (m, 4H), 1.28 (t, 1H); MS (ESI): m/z 351.4 [M+H]+ |
| 27 | 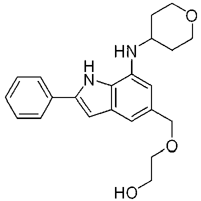 | 2-((2-phenyl-7-((tetrahydro-2H-pyran-4-yl)amino)-1H-indol-5-yl)methoxy)ethan-1-ol | ¹H NMR (400MHz, CDCl₃); d 8.43 (br s, 1H), 7.72 (d, 2H), 7.46 (t, 2H), 7.36 (t, 1H), 7.12 (s, 1H), 6.80 (s, 1H), 6.57 (s, 1H), 4.63 (s, 2H), 4.16 (m, 1H), 4.07 (m, 2H), 3.79 (t, 2H), 3.65 (t, 2H), 3.53 (m, 2H), 2.14 (m, 2H), 1.61 (m, 2H); MS (ESI): m/z 367.4 [M+H]+ |
| 28 | 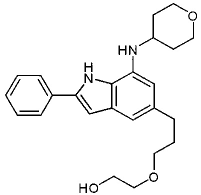 | 2-(3-(2-phenyl-7-((tetrahydro-2H-pyran-4-yl)amino)-1H-indol-5-yl)propoxy)ethan-1-ol | ¹H NMR (400MHz, CDCl₃) δ 8.84 (br s, 1H), 7.75 (d, 2H), 7.45 (t, 2H), 7.32 (t, 1H), 7.08 (s, 1H), 6.77 (s, 1H), 6.58 (s, 1H), 4.05 (m, 2H), 3.77 (m, 2H), 3.68 (m, 1H), 3.58 - 3.48 (m, 6H), 2.76 (t, 2H), 2.15 (m, 2H), 1.97 (m, 2H), 1.74 (m, 2H); MS (ESI): m/z 395.4 [M+H]+ |
| 29 | 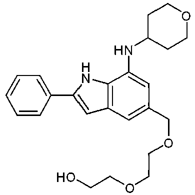 | 2-(2-((2-phenyl-7-((tetrahydro-2H-pyran-4-yl)amino)-1H-indol-5-yl)methoxy)ethoxy)ethan-1-ol | ¹H NMR (400MHz, CDCl₃) δ 8.81 (br s, 1H), 7.76 (d, 2H), 7.46 (t, 2H), 7.36 (t, 1H), 7.12 (s, 1H), 6.80 (s, 1H), 6.52 (s, 1H), 4.60 (s, 2H), 4.06-4.03 (m, 2H), 3.76-3.48 (m, 11H), 2.12 (m, 2H), 1.63 (m, 2H), 1.23 (t, 1H); MS (ESI): m/z 411.23 [M+H]+ |
| 30 | 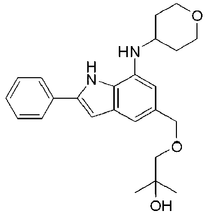 | 2-methyl-1-((2-phenyl-7-((tetrahydro-2H-pyran-4-yl)amino)-1H-indol-5-yl)methoxy)propan-2-ol | ¹H NMR (400 MHz, DMSO-*d₆*) δ10.93 (br s, 1H), 7.68 - 7.91 (m, 2H), 7.41 - 7.53 (m, 2H), 7.26 - 7.36 (m, 1H), 6.76 (s, 2H), 6.31 (s, 1H), 5.27 - 5.43 (m, 1H), 4.46 (s, 2H), 4.29 (s, 1H), 3.93 (d, J=8.0Hz, 2H), 3.59 - 3.67 (m, 2H), 3.13 (s, 2H), 2.06 (d, J=8.0Hz, 2H), 1.37 - 1.56 (m, 2H), 1.08 (s, 6H); MS (ESI): m/z 395.1 [M+H]+ |
| 31 | 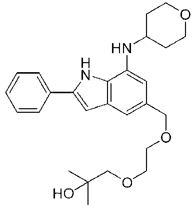 | 2-methyl-1-(2-((2-phenyl-7-((tetrahydro-2H-pyran-4-yl)amino)-1H-indol-5-yl)methoxy)ethoxy)propan-2-ol | ¹H NMR (400 MHz, DMSO-*d₆*) δ10.93 (br s, 1H), 7.80 (d, J=8Hz, 2H), 7.47 (t, J=7.6Hz, 2H), 7.30 (t, J=7.2Hz, 1H), 6.77 (s, 2H), 6.29 (s, 1H), 5.36 (d, J=7.6Hz, 1H), 4.45 (s, 2H), 4.28 (s, 1H), 4.00 - 3.89 (m, 2H), 3.69 - 3.61 (m, 1H), 3.60 - 3.55 (m, 2H), 3.54 - 3.46 (m, 4H), 3.18 (s, 2H), 2.05 (d, J=12.4Hz, 2H), 1.53 - 1.38 (m, 2H), 1.07 (s, 6H); MS (ESI): m/z 439.1 [M+H]+ |
| 32 | 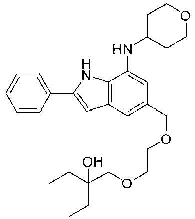 | 3-((2-((2-phenyl-7-((tetrahydro-2H-pyran-4-yl)amino)-1H-indol-5-yl)methoxy)ethoxy)methyl)pentan -3-ol | ¹H NMR (400 MHz, DMSO-*d₆*) δ10.93 (br s, 1H), 7.80 (d, J=8.0Hz, 2H), 7.42 - 7.51 (m, 2H), 7.27 - 7.35 (m, 1H), 6.68 - 6.82 (m, 2H), 6.29 (s, 1H), 5.36 (d, J=7.6Hz, 1H), 4.44 (s, 2H), 3.79 - 4.07 (m, 4H), 3.45 - 3.53 (m, 7H), 2.05 (d, J=12.0Hz, 2H), 1.43 - 1.51 (m, 2H), 1.34 - 1.41 (m, 4H), 0.79 (t, J=7.6Hz, 6H); MS (ESI): m/z 467.1 [M+H]+ |
| 33 | 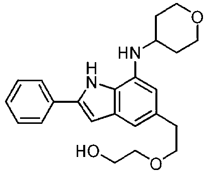 | 2-(2-(2-phenyl-7-((tetrahydro-2H-pyran-4-yl)amino)-1H-indol-5-yl)ethoxy)ethan-1-ol | ¹H NMR (400 MHz, DMSO-*d₆*) δ10.85 (br s, 1H), 7.80 (d, 2H), 7.47 (t, 2H), 7.30 (t, 1H), 6.72 (s, 1H), 6.65 (s, 1H), 6.24 (s, 1H), 5.29 (d, 1H), 4.59 (m, 1H), 3.94 (m, 2H), 3.64 (m, 1H), 3.59 (m, 2H), 3.50 (m, 4H), 3.43 (m, 2H), 2.78 (t, 2H), 2.05 (m, 2H), 1.46 (m, 2H); MS (ESI): m/z 381.2 [M+H]+ |
| 34 | 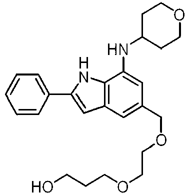 | 3-(2-((2-phenyl-7-((tetrahydro-2H-pyran-4-yl)amino)-1H-indol-5-yl)methoxy)ethoxy)propan-1-ol | ¹H NMR (400 MHz, DMSO-*d₆*) δ10.95 (br s, 1H), 7.81 (d, 2H), 7.48 (t, 2H), 7.30 (t, 1H), 6.78 (two s, 2H), 6.30 (s, 1H), 5.38 (d, 1H), 4.45 (m, 2H), 4.37 (t, 1H), 3.94 (m, 2H), 3.60 (m, 1H), 3.52-3.45 (m, 10H), 2.05 (m, 2H), 1.65 (m, 2H), 1.48 (m, 2H); MS (ESI): m/z 425.2 [M+H]+ |
| 35 | 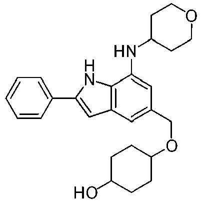 | 4-((2-phenyl-7-((tetrahydro-2H-pyran-4-yl)amino)-1H-indol-5-yl)methoxy)cyclohexan-1-ol | ¹H NMR (400MHz, CDCl₃) δ 8.68 (br s, 1H), 7.71 (d, 2H), 7.44 (t, 2H), 7.33 (t, 1H), 7.15-7.12 (s, 1H), 6.77 (s, 1H), 6.67-6.63 (two s, 1H), 4.57 (s, 2H), 4.03 (m, 2H), 3.75-3.65 (m, 2H), 3.51 (m, 2H), 3.54 (m, 2H), 3.06 (m, 8H), 2.80 (t, 2H), 2.13 (m, 2H), 2.02- 1.25 (m, 10H); MS (ESI): m/z 421.3 [M+H]+ |
| 36 | 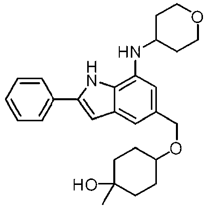 | 1-methyl-4-((2-phenyl-7-((tetrahydro-2H-pyran-4-yl)amino)-1H-indol-5-yl)methoxy)cyclohexan-1-ol | ¹H NMR (400MHz, CDCl₃) δ 8.68 (br s, 1H), 7.71 (d, 2H), 7.45 (t, 2H), 7.32 (t, 1H), 7.15 (s, 1H), 6.78 (s, 1H), 6.66 (s, 1H), 4.69 (s, 2H), 4.05 (m, 2H), 3.69 (m, 1H), 3.54 (m, 2H), 2.17 (s, 1H), 2.15 (m, 2H), 1.58 (m, 10H), 1.25 (s, 3H); MS (ESI): m/z 435.3 [M+H]+ |
| 37 | 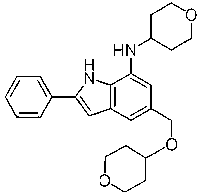 | 2-phenyl-N-(tetrahydro-2H-pyran-4-yl)-5-(((tetrahydro-2H-pyran-4-yl)oxy)methyl)-1H-indol-7-amine | ¹H NMR (400MHz, CDCl₃) δ 8.44 (br s, 1H), 7.70 (d, 2H), 7.45 (m, 3H), 7.34 (t, 1H), 7.13 (s, 1H), 6.78 (s, 1H), 6.63 (s, 1H), 4.60 (s, 2H), 4.06 (m, 2H), 3.95 (m, 2H), 3.63 (m, 1H), 3.61 (m, 1H), 3.55 (m, 2H), 3.41 (m, 2H), 2.12 (m, 2H), 1.93 (m, 2H), 1.65 (m, 6H). |
| 38 | 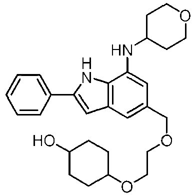 | 4-(2-((2-phenyl-7-((tetrahydro-2H-pyran-4-yl)amino)-1H-indol-5-yl)methoxy)ethoxy)cyclohexan-1-ol | ¹H NMR (400 MHz, DMSO-*d₆*) δ10.94 (br s, 1H), 7.80 (d, 2H), 7.48 (t, 2H), 7.31 (t, 1H), 6.77 (two s, 2H), 6.65 (s, 1H), 5.37 (d, 1H), 4.45 (m, 3H), 3.94 (m, 2H), 3.60 (m, 1H), 3.52-3.45 (m, 6H), 2.05 (m, 2H), 1.78-1.47 (m, 10H); MS (ESI): m/z 465.2 [M+H]+ |
| 39 | 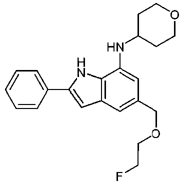 | 5-((2-fluoroethoxy)methyl)-2-phenyl-N-(tetrahydro-2H-pyran-4-yl)-1H-indol-7-amine | ¹H NMR (400MHz, CDCl₃) δ 8.90 (br s, 1H), 7.76 (d, 2H), 7.47 (t, 2H), 7.35 (t, 1H), 7.20 (s, 1H), 6.79 (s, 1H), 6.75 (s, 1H), 4.66 (m, 1H), 4.61 (s, 2H), 4.54 (m, 1H), 4.06 (m, 2H), 3.76-3.74 (m, 3H), 3.67 (3.68 (m, 2H), 3.48 (m, 2H), 2.12 (m, 2H), 1.77 (m, 2H); MS (ESI): m/z 369.2 [M+H]+ |
| 40 | 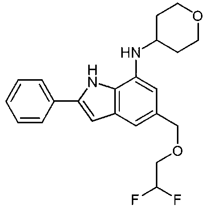 | 5-((2,2-difluoroethoxy)methyl)-2-phenyl-N-(tetrahydro-2H-pyran-4-yl)-1H-indol-7-amine | ¹H NMR (400MHz, CDCl₃) δ 8.34 (br s, 1H), 7.71 (d, 2H), 7.45 (t, 2H), 7.31 (t, 1H), 7.10 (s, 1H), 6.78 (s, 1H), 6.60 (s, 1H), 6.02-5.72 (m, 1H), 4.65 (s, 2H), 4.07 (m, 2H), 3.71-3.53 (m, 5H), 2.15 (m, 2H), 1.62 (m, 2H); MS (ESI): m/z 387.3 [M+H]+ |

### Example 41: N-cyclopentyl-5-((2-morpholinoethoxy)methyl)-2-phenyl-1H-indol-7-amine

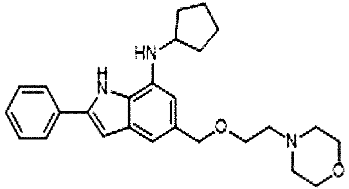

The 2-((7-(cyclopenylamino)-2-phenyl-1H-indol-5-yl)methoxy)ethyl methanesulfonate (31 mg, 0.072 mmol) obtained in Preparation Example A80 was dissolved in morpholine (1.5 mL), heated to 50 °C, and stirred for 2 hours. EA and water were added to the reaction solution and extracted, and an aqueous layer was washed with a saturated NaCl solution, dried over MgSO₄, filtered, and concentrated under reduced pressure. The concentrated residue was purified through silica gel column chromatography, thereby obtaining the title compound (10 mg, 33%) as a green solid.
¹H NMR (400MHz, CDCl₃) δ 8.19 (br s, 1H), 7.71 (d, 2H), 7.46 (t, 2H), 7.32 (t, 1H), 7.07 (s, 1H), 6.79 (s, 1H), 6.58 (s, 1H), 4.63 (s, 2H), 4.00 (m, 1H), 3.75 (m, 4H), 3.61 (t, 2H), 2.64 (t, 2H), 2.53 (br s, 4H), 2.16 (m, 2H), 1.81 (m, 2H), 1.75-1.61 (m, 4H; MS (ESI): m/z 420.3 [M+H]+)

### Example 43: 4-(2-((7-(cyclopentylamino)-2-phenyl-1H-indol-5-yl)methoxy)ethyl)thiomorpholine 1,1-dioxide

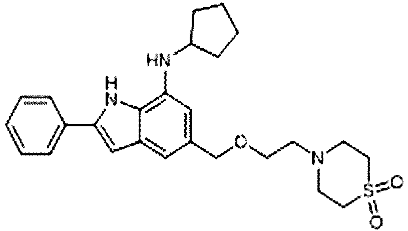

The 5-((2-bromoethoxy)methyl)-N-cyclopentyl-2-phenyl-1H-indol-7-amine (50 mg, 0.1209 mmol) obtained in Preparation Example A82 was dissolved in THF, thiomorpholine 1,1-dioxide (827 mg, 6.12 mmol), KI (200 mg), and DIPEA (100 mL) were added, and then refluxed and stirred for 30 hours. After concentrating the reaction solution, EA and water were added for extraction, the resulting extract was dried over MgSO₄, filtered, and concentrated under reduced pressure. The concentrated residue was purified through silica gel column chromatography, thereby obtaining the title compound (30 mg, 53%) as a white solid.
¹H NMR (400MHz, CDCl₃) δ 8.49 (br s, 1H), 7.71 (d, 2H), 7.45 (t, 2H), 7.31 (t, 1H), 7.07 (s, 1H), 6.78 (s, 1H), 6.56 (s, 1H), 4.60 (s, 2H), 3.97 (m, 1H), 3.58 (br s, 2H), 3.50 (m, 1H), 3.03 (br s, 8H), 2.77 (br s, 2H), 2.12 (m, 2H), 1.81 (m, 2H), 1.66 (m, 4H; MS (ESI): m/z 468.3 [M+H]+)

The following example compounds in Table 3 were prepared in a similar manner to Examples 41 and 43. The chemical structures, compound names, NMR and LCMS analysis results of each example compound are shown in Table 3.

**[Table 3]**

| **Exa mple No.** | **Structure** | **Compound Name** | **¹H NMR; MS[M+H]+** |
|---|---|---|---|
| 42 | 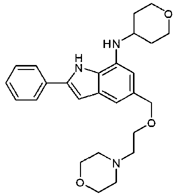 | 5-((2-morpholinoethoxy)methyl)-2-phenyl-N-(tetrahydro-2H-pyran-4-yl)-1H-indol-7-amine | ¹H NMR (400MHz, CDCl₃) δ 8.31 (br s, 1H), 7.71 (d, 2H), 7.46 (t, 2H), 7.32 (t, 1H), 7.09 (s, 1H), 6.80 (s, 1H), 6.59 (s, 1H), 4.62 (s, 2H), 4.07 (m, 2H), 3.73 (m, 4H), 3.65 (m, 1H), 3.64 - 3.52 (m, 4H), 2.64 (t, 2H), 2.51 (br, 4H), 2.14 (m, 2H), 1.61 (m, 2H); MS (ESI): m/z 436.3 [M+H]+ |
| 44 | 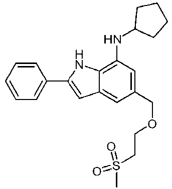 | N-cyclopentyl-5-((2-(methylsulfonyl)ethoxy)methyl) -2-phenyl-1H-indol-7-amine | ¹H NMR (400MHz, CDCl₃) δ 8.53 (br s, 1H), 7.72 (d, 2H), 7.46 (t, 2H), 7.35 (t, 1H), 7.10 (s, 1H), 6.80 (s, 1H), 6.58 (s, 1H), 4.60 (s, 2H), 4.00 (m, 1H), 3.94 (m, 2H), 3.24 (m, 2H), 3.03 (s, 8H), 2.12 (m, 2H), 1.84 (m, 2H), 1.69 (m, 4H); MS (ESI): m/z 414.2 [M+H]+ |
| 45 | 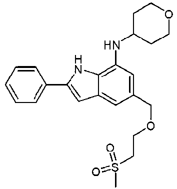 | 5-((2-(methylsulfonyl)ethoxy)methyl) -2-phenyl-N-(tetrahydro-2H-pyran-4-yl)-1H-indol-7-amine | ¹H NMR (400MHz, CDCl₃) δ 8.57 (br s, 1H), 7.73 (d, 2H), 7.47 (t, 2H), 7.36 (t, 1H), 7.13 (s, 1H), 6.80 (s, 1H), 6.60 (s, 1H), 4.61 (s, 2H), 4.07 (m, 2H), 3.94 (m, 2H), 3.70 (m, 1H), 3.57 (m, 2H), 3.25 (t, 2H), 3.02 (s, 3H), 2.15 (m, 2H), 1.69 (m, 2H); MS (ESI): m/z 429.1 [M+H]+ |
| 46 | 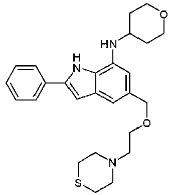 | 2-phenyl-N-(tetrahydro-2H-pyran-4-yl)-5-((2-thiomorpholinoethoxy)methyl)-1H-indol-7-amine | ¹H NMR (400MHz, CDCl₃) δ 8.89 (br s, 1H), 7.73 (d, 2H), 7.41 (t, 2H), 7.30 (t, 1H), 7.05 (s, 1H), 6.78 (s, 1H), 6.53 (s, 1H), 4.59 (s, 2H), 4.04 (m, 2H), 3.65 (m, 1H), 3.61 (m, 2H), 3.54 (m, 2H), 2.82 (br, 4H), 2.69 (br, 6H), 2.10 (m, 2H), 1.57 (m, 2H); MS (ESI): m/z 452.1 [M+H]+ |
| 47 | 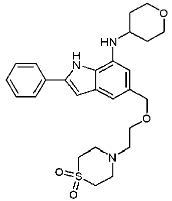 | 4-(2-((2-phenyl-7-((tetrahydro-2H-pyran-4-yl)amino)-1H-indol-5-yl)methoxy)ethyl)thiomorpholin e 1,1-dioxide | ¹H NMR (400MHz, CDCl₃) δ 8.45 (br s, 1H), 7.73 (d, 2H), 7.47 (t, 2H), 7.30 (t, 1H), 7.11 (s, 1H), 6.80 (s, 1H), 6.59 (s, 1H), 4.60 (s, 2H), 4.07 (m, 2H), 3.69 (m, 1H), 3.59 (t, 2H), 3.54 (m, 2H), 3.06 (m, 8H), 2.80 (t, 2H), 2.13 (m, 2H), 1.70 (m, 2H); MS (ESI): m/z 484.3 [M+H]+ |

### Example 48: 2-((2-((2-phenyl-7-((tetrahydro-2H-pyran-4-yl)amino)-1H-indol-5-yl)methoxy)ethyl)amino)ethan-1-ol

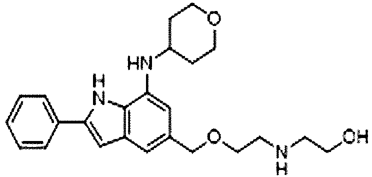

After undergoing the process of Example 1, the compound of Preparation Example A85 was reacted and treated in the same manner as in Preparation Example A56, thereby obtaining the title compound (30 mg, 41%).
¹H NMR (400 MHz, DMSO-d₆) δ 11.34 (br s, 1H), 8.57 (br s, 1H), 8.47 (br s, 1H), 7.84-7.89 (m, 2H), 7.44-7.52 (m, 3H), 7.29-7.34 (m, 1H), 7.00 (s, 1H), 6.85 (br s, 1H), 6.51 (s, 1H), 4.54 (s, 2H), 3.90-3.96 (m, 3H), 3.60-3.70 (m, 4H), 3.13-3.18 (m, 2H), 2.98-3.04 (m, 4H), 1.98-2.04 (m, 2H), 1.55-1.65 (m, 2H; MS (ESI): m/z 410.5 [M+H]+)

### Example 49: N-(2-methoxyethyl)-2-phenyl-7-((tetrahydro-2H-pyran-4-yl)amino)-1H-indol-5-carboxamide

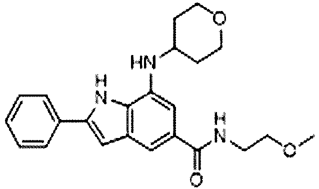

Methoxyethan-1-amine was treated in the same manner as in Preparation Example A1, and then reacted and treated in the same manner as in Preparation Example A2 and Example 2, thereby obtaining the title compound (132 mg) as a white solid.
¹H NMR (400 MHz, DMSO-*d₆*) δ 11.16 (br s, 1H), 8.23 (br s, 1H), 7.84 (d, J = 8.4 Hz, 2H), 7.46 - 7.52 (m, 2H), 7.42 (s, 1H), 7.30 - 7.37 (m, 1H), 6.90 (s, 1H), 6.82 (s, 1H), 5.46 (br s, 1H), 3.93 - 3.98 (m, 2H), 3.70 - 3.74 (m, 1H), 3.50 - 3.53 (m, 2H), 3.42 - 3.48 (m, 2H), 3.37 - 3.41 (m, 2H), 3.28 (s, 3H), 2.04 - 2.11 (m, 2H), 1.45 - 1.52 (m, 2H; MS (ESI): m/z 394.2 [M+H]+)

### Example 50: 5-(((2-methoxyethyl)amino)methyl)-2-phenyl-N-(tetrahydro-2H-pyran-4-yl)-1H-indol-7-amine

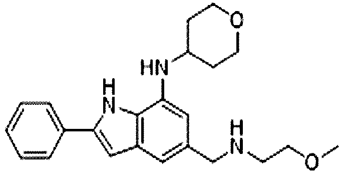

Example 49 (120 mg, 0.305 mmol) was dissolved in THF (10 mL) and then cooled to - 5 °C. 2M LAH in THF (3.05 mL, 6.10 mmol) was added dropwise. After stirring overnight, the resulting mixture was diluted in DCM, and 1N NaOH was dropwise at 5 °C. EA and H₂O were added for extraction, and an organic layer was washed with a saturated NaCl solution, dried over MgSO₄, and filtered. The filtrate was concentrated under reduced pressure and purified through silica gel column chromatography, thereby obtaining the title compound (70 mg, 60%).
¹H NMR (400 MHz, DMSO-*d₆*) δ 10.88 (br s, 1H), 7.81 (d, J = 8.4 Hz, 2H), 7.44 - 7.50 (m, 2H), 7.27 - 7.32 (m, 1H), 6.75 (s, 1H), 6.72 (s, 1H), 6.32 (s, 1H), 5.30 (br s, 1H), 3.92 - 3. 95 (m, 2H), 3.67 (s, 2H), 3.62 - 3.66 (m, 1H), 3.48 - 3.52 (m, 2H), 3.31 - 3.35 (m, 2H), 3.23 (s, 3H), 2.61 - 2.65 (m, 2H), 2.05 - 2.10 (m, 2H), 1.44 - 1.49 (m, 2H; MS (ESI): m/z 380.2 [M+H]+)

The following example compounds in Table 4 below were prepared in a similar manner to Examples 49 and 50. The chemical structures, compound names, NMR and LCMS analysis results of each example compound are shown in Table 4.

**[Table 4]**

| **Exam ple No.** | **Structure** | **Compound Name** | **¹H NMR; MS[M+H]+** |
|---|---|---|---|
| 51 | 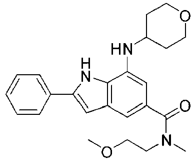 | N-(2-methoxyethyl)-N-methyl-2-phenyl-7-((tetrahydro-2H-pyran-4-yl)amino)-1H-indole-5-carboxamide | ¹H NMR (400 MHz, DMSO-*d₆*) δ 11.13 (br s, 1H), 7.89 (d, J = 8.8 Hz, 2H), 7.46 - 7.50 (m, 2H), 7.31 - 7.35 (m, 1H), 6.86 (s, 1H), 6.83 (s, 1H), 6.31 (s, 1H), 5.53 (br s, 1H), 3.91 - 3.96 (m, 2H), 3.63 - 3.68 (m, 1H), 3.45 - 3.60 (m, 4H), 3.32 (s, 3H), 3.21 - 3.31 (m, 2H), 2.98 (s, 3H), 2.02 - 2.08 (m, 2H), 1.44 - 1.51 (m, 2H); MS (ESI): m/z 408.2 [M+H]+ |
| 52 | 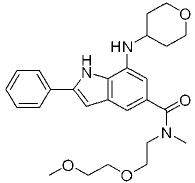 | N-(2-(2-methoxyethoxy)ethyl)-N-methyl-2-phenyl-7-((tetrahydro-2H-pyran-4-yl)amino)-1H-indole-5-carboxamide | ¹H NMR (400 MHz, DMSO-*d₆*) δ 11.18 (br s, 1H), 7.84 (d, J = 8.4 Hz, 2H), 7.46 - 7.51 (m, 2H), 7.31 - 7.35 (m, 1H), 6.85 (s, 2H), 6.29 (s, 1H), 5.55 (br s, 1H), 3.91 - 3.95 (m, 2H), 3.64 - 3.68 (m, 1H), 3.42 - 3.60 (m, 10H), 3.25 (s, 3H), 2.99 (s, 3H), 2.02 - 2.06 (m, 2H), 1.46 - 1.50 (m, 2H); MS (ESI): m/z 452.2 [M+H]+ |
| 53 | 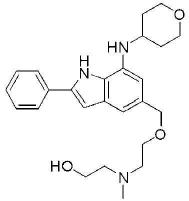 | 2-(methyl(2-((2-phenyl-7-((tetrahydro-2H-pyran-4-yl)amino)-1H-indol-5-yl)methoxy)ethyl)amino)ethan-1-ol | ¹H NMR (400 MHz, DMSO-*d₆*) δ 10.99 (br s, 1H), 7.80 - 7.84 (m, 2H), 7.44 - 7.50 (m, 2H), 7.28 - 7.33 (m, 1H), 6.78 (s, 1H), 6.31 (s, 1H), 5.43 (br s, 1H), 4.45 (s, 2H), 3.92 - 3.96 (m, 2H), 3.62 - 3.67 (m, 1H), 3.44 - 3.52 (m, 6H), 2.49 (s, 3H), 2.01 - 2.04 (m, 2H), 1.45 - 1.51 (m, 2H), 1.22 - 1.26 (m, 2H); MS (ESI): m/z 424.2 [M+H]+ |
| 54 | 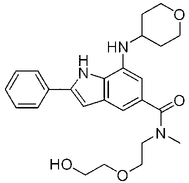 | N-(2-(2-hydroxyethoxy)ethyl)-N-methyl-2-phenyl-7-((tetrahydro-2H-pyran-4-yl)amino)-1H-indole-5-carboxamide | ¹H NMR (400 MHz, DMSO-*d₆*) δ 11.11 (br s, 1H), 7.83 (d, J = 8.4 Hz, 2H), 7.46 - 7.51 (m, 2H), 7.31 - 7.35 (m, 1H), 6.84 (s, 1H), 6.83 (s, 1H), 6.30 (s, 1H), 5.51 (br s, 1H), 4.63 (br s, 1H), 3.91 - 3.95 (m, 2H), 3.65 - 3.70 (m, 1H), 3.30 - 3.45 (m, 10H), 2.99 (s, 3H), 2.03 - 2.07 (m, 2H), 1.43 - 1.49 (m, 2H); MS (ESI): m/z 438.2 [M+H]+ |
| 55 | 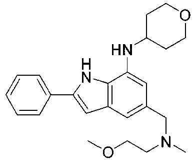 | 5-(((2-methoxyethyl)(methyl)amino)m ethyl)-2-phenyl-N-(tetrahydro-2H-pyran-4-yl)-1H-indol-7-amine | ¹H NMR (400 MHz, DMSO-*d₆*) δ 10.91 (br s, 1H), 7.81 (d, J = 8.4 Hz, 2H), 7.44 - 7.48 (m, 2H), 7.28 - 7.33 (m, 1H), 6.77 (s, 1H), 6.76 (s, 1H), 6.32 (s, 1H), 5.34 (br s, 1H), 3.91 - 3.95 (m, 2H), 3.62 - 3.65 (m, 1H), 3.46 - 3.52 (m, 2H), 3.42 - 3.45 (m, 2H), 3.39 (s, 2H), 3.26 - 3.30 (m, 2H), 3.23 (s, 3H), 2.48 (s, 3H), 2.07 - 2.11 (m, 2H), 1.44 - 1.51 (m, 2H); MS (ESI): m/z 394.2 [M+H]+ |
| 56 | 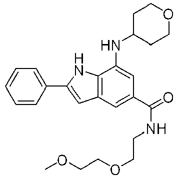 | N-(2-(2-methoxyethoxy)ethyl)-2-phenyl-7-((tetrahydro-2H-pyran-4-yl)amino)-1H-indole-5-carboxamide | ¹H NMR (400 MHz, DMSO-*d₆*) δ 11.15 (br s, 1H), 8.23 (br s, 1H), 7.85 (d, J = 8.8 Hz, 2H), 7.48 - 7.52 (m, 2H), 7.42 (s, 1H), 7.31 - 7.35 (m, 1H), 6.90 (s, 1H), 6.82 (s, 1H), 5.48 (br s, 1H), 3.93 - 3.98 (m, 2H), 3.68 - 3.73 (m, 1H), 3.44 - 3.52 (m, 6H), 3.40 - 3.44 (m, 2H), 3.36 - 3.39 (m, 2H), 3.24 (s, 3H), 2.05 - 2.10 (m, 2H), 1.43 - 1.52 (m, 2H); MS (ESI): m/z 438.2 [M+H]+ |
| 57 | 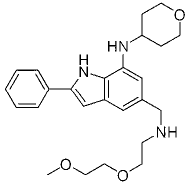 | 5-(((2-(2-methoxyethoxy)ethyl)amino)me thyl)-2-phenyl-N-(tetrahydro-2H-pyran-4-yl)-1H-indol-7-amine | ¹H NMR (400 MHz, DMSO-*d₆*) δ 11.37 (br s, 1H), 8.83 (br s, 1H), 7.88 (d, J = 8.4 Hz, 2H), 7.46 - 7.51 (m, 2H), 7.31 - 7.34 (m, 1H), 6.99 (s, 1H), 6.86 (s, 1H), 6.55 (s, 1H), 4.13 (s, 2H), 3.96 - 3.99 (m, 2H), 3.76 - 3.80 (m, 1H), 3.70 - 3.75 (m, 2H), 3.61 - 3.68 (m, 4H), 3.27 - 3.30 (m, 2H), 3.25 (s, 3H), 3.02 - 3.06 (m, 2H), 2.06 - 2.09 (m, 2H), 1.53 - 1.57 (m, 2H); MS (ESI): m/z 424.2 [M+H]+ |
| 58 | 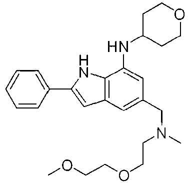 | 5-(((2-(2-methoxyethoxy)ethyl)(methyl)a mino)methyl)-2-phenyl-N-(tetrahydro-2H-pyran-4-yl)-1H-indol-7-amine | ¹H NMR (400 MHz, DMSO-*d₆*) δ 10.91 (br s, 1H), 7.78 - 7.82 (m, 2H), 7.45 - 7.49 (m, 2H), 7.29 - 7.33 (m, 1H), 6.77 (s, 1H), 6.76 (s, 1H), 5.32 (br s, 1H), 3.91 - 3.95 (m, 2H), 3.61 - 3.65 (m, 1H), 3.48 - 3.53 (m, 4H), 3.44 (s, 2H), 3.26 - 3.41 (m, 6H), 3.24 (s, 3H), 2.49 (s, 3H), 2.03 - 2.08 (m, 2H), 1.42 - 1.48 (m, 2H); MS (ESI): m/z 438.2 [M+H]+ |
| 59 | 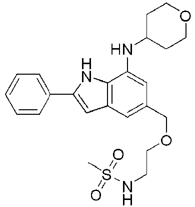 | N-(2-((2-phenyl-7-((tetrahydro-2H-pyran-4-yl)amino)-1H-indol-5-yl)methoxy)ethyl)methanesulfon amide | ¹H NMR (400 MHz, DMSO-*d₆*) δ 10.96 (br s, 1H), 7.82 (d, J = 8.4 Hz, 2H), 7.46 - 7.50 (m, 2H), 7.29 - 7.32 (m, 1H), 6.79 (s, 1H), 6.78 (s, 1H), 6.32 (br s, 1H), 5.38 (br s, 1H), 4.46 (s, 2H), 3.93 - 3.95 (m, 2H), 3.63 - 3.66 (m, 1H), 3.45 - 3.53 (m, 4H), 3.11 - 3.14 (m, 2H), 2.89 (s, 3H), 2.04 - 2.09 (m, 2H), 1.44 - 1.50 (m, 2H); MS (ESI): m/z 444.1 [M+H]+ |

### Example 60: 5-((2-((2-methoxyethyl)amino)ethoxy)methyl)-2-phenyl-N-(tetrahydro-2H-pyran-4-yl)-1H-indol-7-amine

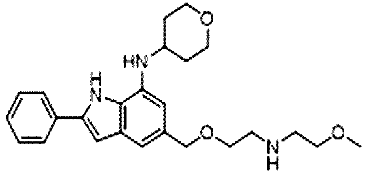

The compound of Preparation Example A95 was reacted and treated in the same manner as in Preparation Example A56, thereby obtaining the title compound (24 mg) as a pink solid.
¹H NMR (400 MHz, DMSO-*d₆*) δ 8.77 (br s, 1H), 7.95 (d, J = 8.4 Hz, 2H), 7.46 - 7.51 (m, 2H), 7.32 - 7.36 (m, 1H), 6.95 (s, 2H), 6.29 (s, 1H), 4.58 (s, 2H), 3.91 - 3.95 (m, 2H), 3.78 - 3.81 (m, 1H), 3.65 - 3.69 (m, 2H), 3.56 - 3.60 (m, 2H), 3.36 - 3.40 (m, 2H), 3.30 (s, 3H), 3.10 - 3.17 (m, 4H), 1.91 - 1.96 (m, 2H), 1.80 - 1.85 (m, 2H; MS (ESI): m/z 424.2 [M+H]+)

### Example 61: 5-((2-((2-methoxyethyl)(methyl)amino)ethoxy)methyl)-2-phenyl-N-(tetrahydro-2H-pyran-4-yl)-1H-indol-7-amine

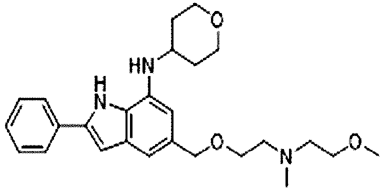

The compound of Preparation Example A95 (100 mg, 0.191 mmol) was dissolved in THF (5 mL), and 2M LAH in THF (0.477 mL, 0.955 mmol) was added to -5 °C. The resulting mixture was heated to 60 °C and stirred for 30 minutes. The mixture was cooled to -5 °C, and the 1N NaOH was added to terminate the reaction. The reaction solution was filtered through Celite, EA and water were added for extraction, and an aqueous layer was washed with a saturated NaCl solution, dried over MgSO₄, filtered, and concentrated under reduced pressure. The concentrated residue was purified through prep-TLC, thereby obtaining the title compound as a beige solid (16 mg, 19%).
¹H NMR (400 MHz, CDCl₃) δ 8.20 (br s, 1H), 7.69 (d, J = 8.4 Hz, 2H), 7.43 - 7.46 (m, 2H), 7.30 - 7.34 (m, 1H), 7.08 (s, 1H), 6.78 (s, 1H), 6.58 (s, 1H), 4.58 (s, 2H), 4.03 - 4.07 (m, 2H), 3.64 - 3.68 (m, 1H), 3.52 - 3.61 (m, 4H), 3.46 - 3.49 (m, 2H), 3.34 (s, 3H), 2.67 - 2.70 (m, 2H), 2.62 - 2.66 (m, 2H), 2.33 (s, 3H), 2.10 - 2.14 (m, 2H), 1.55 - 1.62 (m, 2H; MS (ESI): m/z 438.2 [M+H]+)

### Experimental Example 1: Cell culture

Cell line medium compositions and culture conditions, which were used in the following experimental examples, were prepared as follows. Each cell line was cultured at 37 °C in a 5% CO₂ incubator. In addition, the cell line was cultured without contamination by microorganisms such as Mycoplasma. Table 5 shows the medium compositions used in the culture of the respective cell cultures.

**Table 5: Medium compositions used in culture of respective cell lines**

| **[Table 5]** | | |
|---|---|---|
| **Cell line** | **Cell type** | **Medium compositions** |
| H9C2 | cardiac cells | DMEM, 10% FBS, 1% PS |
| NRK-49F | renal fibroblasts | DMEM, 5% FBS, 1% PS |
| MLE-12 | lung epithelial cells | DMEM:F-12, 2% FBS, 1% PS, 0.1% Insulin-Transferrin-Selenium, 10 nM Hydrocortisone, 10 nM β10 mM HEPES, 2 mM L-GlutaMax |
| ARPE-19 | retinal epithelial cells | DMEM:F-12, 10% FBS, 1% PS |
| PC-12 | adrenal medullary cells | DMEM, 10% FBS, 1% PS |
| HK2 | renal proximal tubular epithelial cells | DMEM:F-12, 10% FBS, 1% PS |
| NIH/3T3 | fibroblasts | DMEM, 10% FBS, 1% PS |
| HT-22 | hippocampal neurons | DMEM, 10% FBS, 1% PS |

### Experimental Example 2: Measurement of protective effect on cardiac cells (H9C2)

To confirm a protective effect on cardiac cells, H9C2 cells were seeded at 1.5 X 10⁴ cells/well in a 96-well plate and cultured for 24 hours. The example compounds were diluted to have the final concentration of 0.001, 0.01, 0.03, 0.1, 0.3, 1, 3, 10, and 30 µM, respectively, and then added to different wells, and the resulting cells were cultured for 15 to 20 minutes. Tert-butyl hydroperoxide (t-BHP) was treated to have the final concentration of 400 µM, and the resulting cells were cultured for 2 hours. To confirm the cell death protection effect of each compound, the degree of extracellular secretion of lactate dehydrogenase (LDH) was measured using a cytotoxicity LDH assay kit [Dojindo; CK12]. Briefly, after 2 hours of t-BHP treatment on the cells, 70 µL of the supernatant was taken from the 96-well plate where the cells were cultured and transferred to a new 96-well plate. The assay buffer included in the kit was added at 70 µL/well (equal to that of the sample) and the plate was covered with foil to block light and allowed to react for 15 minutes at room temperature. While observing a color change of each sample, 35 µL/well of a stop solution was added, an absorbance at 490 nm was measured using an iD3 spectrophotometer, and an EC₅₀ value was calculated. The EC₅₀ of the example compounds is preferably 1.0 µM or less, and more preferably 0.3 µM or less. Table 6 shows the protective effect of the example compounds against the cell death-inducing substance t-BHP.

**Table 6: Protective effect against cell death due to t-BHP induction in cardiac cells (H9C2)**

| **[Table 6]** | | | | | | | |
|---|---|---|---|---|---|---|---|
| **Example** | **EC₅₀** | **Example** | **EC₅₀** | **Example** | **EC₅₀** | **Example** | **EC₅₀** |
| 1 | A | 17 | A | 33 | A | 49 | A |
| 2 | A | 18 | B | 34 | A | 50 | A |
| 3 | A | 19 | A | 35 | A | 51 | A |
| 4 | A | 20 | A | 36 | A | 52 | C |
| 5 | A | 21 | C | 37 | A | 53 | A |
| 6 | A | 22 | B | 38 | C | 54 | B |
| 7 | A | 23 | A | 39 | A | 55 | A |
| 8 | A | 24 | A | 40 | A | 56 | B |
| 9 | B | 25 | A | 41 | A | 57 | A |
| 10 | B | 26 | A | 42 | A | 58 | A |
| 11 | B | 27 | A | 43 | A | 59 | A |
| 12 | A | 28 | A | 44 | A | 60 | A |
| 13 | B | 29 | A | 45 | A | 61 | A |
| 14 | B | 30 | A | 46 | A | - | - |
| 15 | A | 31 | A | 47 | B | - | - |
| 16 | A | 32 | A | 48 | B | - | - |
| A indicates EC₅₀ ≤ 0.3 µM, B indicates 0.3 µM < EC₅₀ ≤ 1 µM, | | | | | | | |
| C indicates EC₅₀ > 1.0 µM | | | | | | | |

### Experimental Example 3: Cell death protection effects on cardiac cells (H9C2), adrenal medullary cells (PC-12), renal fibroblasts (NRK-49F), retinal epithelial cells (ARPE-19), lung epithelial cells (MLE-12), renal proximal tubular epithelial cells (HK2), hippocampal neurons (HT-22), and fibroblasts (NIH/3T3)

To confirm cell death protection effects on cardiac cells, adrenal medullary cells, renal fibroblasts, retinal epithelial cells, lung epithelial cells, renal proximal tubular epithelial cells, hippocampal neurons and fibroblasts, each type of cells were seeded at 4 X 10³ to 1.2 X 10⁴ cells/well in a 96-well plate and cultured for 18 to 24 hours. The example compounds were diluted to have the final concentration of 0.001, 0.003, 0.01, 0.3, 1, 3, and 10 µM, respectively, and then added to different wells and cultured for 15 to 20 minutes. RSL3 was added to be 0.3 to 3 µM for each type of cells as shown in Table 7, and cultured for 24 hours. To confirm the cell death protection effect of the example compounds, the degree of extracellular secretion of lactate dehydrogenase (LDH) was measured using a cytotoxicity LDH assay kit [Dojindo; CK12]. Briefly, at the end of 24-hour treatment of each type of cells with RSL3, 70 µL of the supernatant was taken from the 96-well plate where the cells were cultured and transferred to a new 96-well plate. The assay buffer included in the kit was added at 70 µL/well (equal to that of the sample) and the plate was covered with foil to block light and allowed to react for 15 to 20 minutes at room temperature. While observing a color change of the sample, 35 µL/well of a stop solution was added, an absorbance at 490 nm was measured using an iD3 spectrophotometer, and an EC₅₀ value was calculated. The EC₅₀ of the example compounds is preferably 1.0 µM or less, and more preferably, 0.1 µM or less. Table 7 shows the cell death protection effect of the example compounds on the treatment of each type of cells with a ferroptosis-inducing substance RSL3.

**Table 7: Protective effect against cell death due to RSL3 in cardiac cells (H9C2), adrenal medullary cells (PC-12), renal fibroblasts (NRK-49F), retinal epithelial cells (ARPE-19), lung epithelial cells (MLE-12), renal proximal tubular epithelial cells (HK2), hippocampal neurons (HT-22), and fibroblasts (NIH/3T3)**

| **[Table 7]** | | | | | | | | |
|---|---|---|---|---|---|---|---|---|
| Cell line | H9C2 | NRK-49F | MLE-12 | ARPE-19 | PC-12 | HK2 | HT-22 | NIH/3T3 |
| RSL3 treatment concentration | 0.5 µM | 3 µM | 1 µM | 0.3 µM | 0.3 µM | 0.3 µM | 0.3 µM | 3 µM |

| | EC₅₀ (µM) | | | | | | | |
|---|---|---|---|---|---|---|---|---|
| Fer-1 | A | A | B | A | A | A | B | B |
| Example 8 | B | B | B | A | B | B | C | B |
| Example 14 | A | A | A | A | A | A | B | B |
| Example 22 | A | A | 8 | A | B | A | B | B |
| Example 27 | A | A | 8 | A | A | A | A | A |
| Example 29 | A | A | A | A | B | A | A | B |
| Example 36 | A | A | B | A | B | A | B | B |
| Example 40 | A | A | A | A | A | A | A | B |
| Example 41 | A | A | A | A | A | A | A | B |
| A indicates EC₅₀ ≤ 0.1 µM, B indicates 0.1 µM < EC₅₀ ≤ 1 µM, | | | | | | | | |
| C indicates EC₅₀ > 1 µM | | | | | | | | |

In Table 7, the comparative example compound Fer-1 and the example compounds showed a different ferroptosis inhibitory effect for each type of cells. It can be confirmed that the example compounds exhibited ferroptosis inhibitory effects that are similar to or more effective than the comparative example compound Fer-1 for each type of cells.

### Experimental Example 4: Inhibitory effect on intracellular lipid reactive oxygen species (lipid ROS) in lung epidermal cells

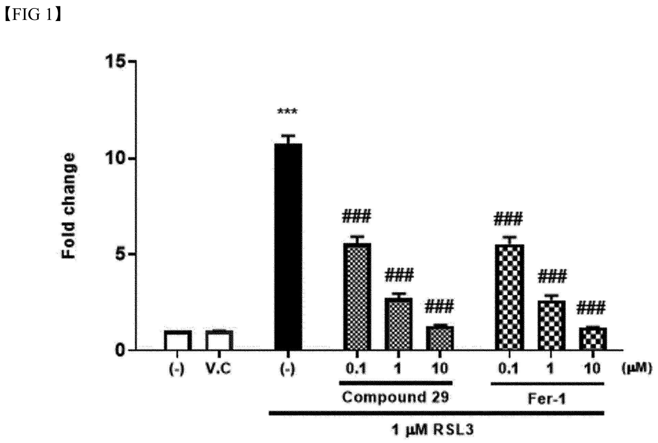

To confirm the protective effect against intracellular lipid ROS in lung epithelial cells, MLE-12 cells were seeded at 2 X 10⁵ cells/well in a 12-well plate and cultured for 18 to 24 hours. Example Compound 29 and Fer-1 were diluted to have the final concentration of 0.01, 0.1, and 1 µM and added to each well, and the cells were cultured for 10 minutes. Afterward, RSL3 was treated to have the final concentration of 1 µM, and the cells were cultured for 2 hours. To confirm the intracellular inhibitory effect on lipid ROS, the lipid peroxidation sensor BODIPY^{™} 581/591 C11 (Invitrogen^{™}; D3861) was used. According to the manufacturer's protocol, the BODIPY^{™} 581/591 C11 was diluted in the culture broth to have the final concentration of 5 µM and treated, and fluorescence staining was performed after a 30-minute reaction. The MLE-12 cells that were fluorescence-stained with BODIPY^{™} 581/591 C11 were detached from the 12-well plate using 0.25% trypsin-EDTA and transferred to a microtube. Fluorescence (Ex/Em = 500~650 nm/510~665 nm) was measured using BD FACSLyric^{™}, and the relative change compared to the control was calculated. The results are shown in FIG. 1.

FIG. 1 shows intracellular lipid ROS levels at various concentrations of Example Compound 29 and Fer-1 for the ferroptosis-inducing substance RSL3. When the MLE-12 cells were treated with 1 µM RSL3, intracellular lipid ROS significantly increased compared to the control (RSL3-untreated group). The lipid ROS increased by RSL3 treatment decreased as the treatment concentration of Example Compound 29 and Fer-1 increased, and significantly decreased at 0.01, 0.1, and 1 µM. Accordingly, it was confirmed that the intracellular lipid ROS increased by the RSL3 treatment was effectively inhibited by the treatment of Example Compound 29.

### Experimental Example 5: Inhibitory effect on mitochondrial lipid reactive oxygen species (mito-lipid ROS) in lung epithelial cells

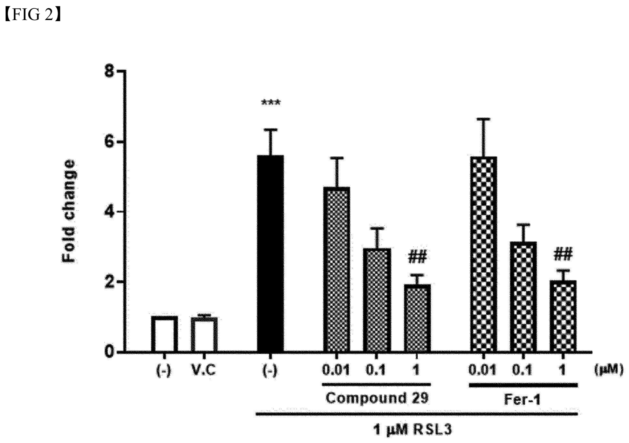

To confirm the protective effect against mito-lipid ROS in lung epithelial cells, MLE-12 cells were seeded at 2 X 10⁵ cells/well in a 12-well plate and cultured for 18 to 24 hours. Example Compound 29 and Fer-1 were diluted to have the final concentration of 0.01, 0.1, and 1 µM and added to each well, and the cells were cultured for 10 minutes. Afterward, RSL3 was treated to have the final concentration of 1 µM, and the cells were cultured for 2 hours. To confirm the inhibitory effect on mito-lipid ROS, the fluorescent mitochondria-targeted lipid peroxidation probe MitoPerOx (abcam; ab146820) was used. According to the manufacturer's protocol, MitoPerOx was diluted in the culture broth to have the final concentration of 1 µM and treated, and fluorescence staining was performed after a 30-minute reaction. The MLE-12 cells that were fluorescence-stained with MitoPerOx were detached from the 12-well plate using 0.25% trypsin-EDTA and transferred to a microtube. Fluorescence (Ex/Em = 495 nm/520-590 nm) was measured using BD FACSLyric^{™}, and the relative change compared to the control was calculated. The results are shown in FIG. 2.

FIG. 2 shows mito-lipid ROS levels at various concentrations of Example Compound 29 and Fer-1 for the ferroptosis-inducing substance RSL3. When the MLE-12 cells were treated with 1 µM RSL3, mito-lipid ROS was significantly increased compared to the control (RSL3-untreated group). The lipid ROS increased by the RSL3 treatment decreased as the treatment concentrations of Example Compound 29 and Fer-1 increased, and significantly decreased at 1 µM. Accordingly, it was confirmed that the mito-lipid ROS increased by RSL3 treatment was effectively inhibited by the treatment of Example Compound 29.

### Experimental Example 6: Inhibitory effect on cytosolic reactive oxygen species (cyto-ROS) in lung epithelial cells

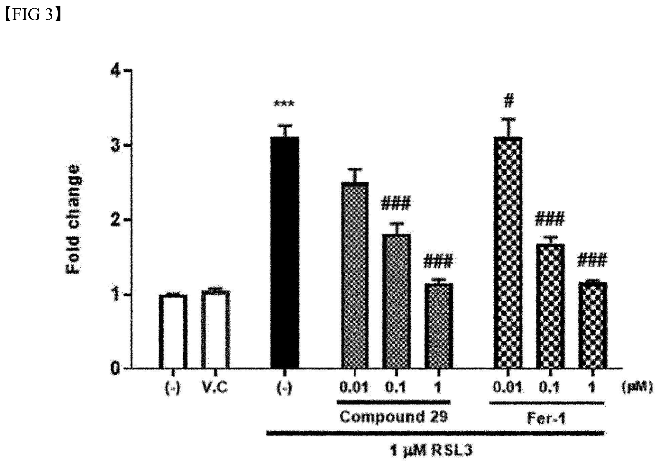

To confirm the protective effect against cyto-ROS in lung epithelial cells, MLE-12 cells were seeded at 2 X 10⁵ cells/well in a 12-well plate, and cultured for 18 to 24 hours. Example Compound 29 and Fer-1 were diluted to have the final concentration of 0.01, 0.1, and 1 µM and added to each well, and the cells were cultured for 10 minutes. Afterward, RSL3 was treated to have the final concentration of 1 µM, and the cells were cultured for 2 hours. To confirm the inhibitory effect on cyto-ROS, the general oxidative stress indicator CM-H2DCFDA (Invitrogen^{™}; C6827) was used. According to the manufacturer's protocol, CM-H2DCFDA was diluted in the culture broth to have the final concentration of 5 µM, and fluorescence staining was performed after a 30-minute reaction. The MLE-12 cells that were fluorescence-stained with CM-H2DCFDA were detached from the 12-well plate using 0.25% trypsin-EDTA and transferred to a microtube. Fluorescence (Ex/Em = 492~495 nm/517~527 nm) was measured using BD FACSLyric^{™}, and the relative change compared to the control (RSL3-untreated group) was calculated. The results are shown in FIG. 3.

FIG. 3 shows intracellular lipid ROS levels at various concentrations of Example Compound 29 and Fer-1 for the ferroptosis-inducing substance RSL3. When the MLE-12 cells were treated with 1 µM RSL3, intracellular lipid ROS was significantly increased compared to the control (RSL3-untreated group). The ROS increased by the RSL3 treatment decreased as the treatment concentrations of Example Compound 29 and Fer-1 increased, and Example Compound 29 significantly reduced lipid ROS levels at 0.1 and 1 µM, and Fer-1 significantly reduced lipid ROS levels at 0.01, 0.1 and 1 µM. Accordingly, it was confirmed that the intracellular lipid ROS increased by the RSL3 treatment was effectively inhibited by the treatment of Example Compound 29.

### Experimental Example 7: Inhibitory effect on mito-ROS in lung epithelial cells

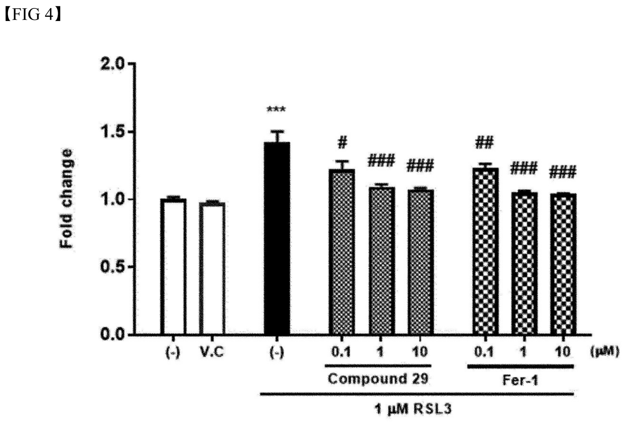

To confirm the protective effect against mito-ROS in lung epithelial cells, MLE-12 cells were seeded at 2 X 10⁵ cells/well in a 12-well plate and cultured for 18 to 24 hours. Example Compound 29 and Fer-1 were diluted to have the final concentration of 0.01, 0.1, 1 and 10 µM and added to each well, and the cells were cultured for 10 minutes. Afterward, RSL3 was treated to have the final concentration of 1 µM, and the cells were cultured for 2 hours. To confirm the inhibitory effect on mito-ROS, the mitochondrial superoxide indicator MitoSOX^{™} (Invitrogen^{™}; C6827) was used. According to the manufacturer's protocol, MitoSOX was diluted in the culture broth to have the final concentration of 5 µM, and fluorescence staining was performed after a 30-minute reaction. The MLE-12 cells fluorescence-strained with MitoSOX were detached from the 12-well plate using 0.25% trypsin-EDTA and transferred to a microtube, and fluorescence (Ex/Em = ^{~} 396 nm/610 nm) was measured using BD FACSLyric^{™}, and the relative change compared to the control (RSL3-untreated group) was calculated. The results are shown in FIG. 4.

FIG. 4 shows mito-ROS levels at various concentrations of Example Compound 29 and Fer-1 for the ferroptosis-inducing substance RSL3. When the MLE-12 cells were treated with 1 µM RSL3, mito-ROS was significantly increased compared to the control (RSL3-untreated group). The ROS increased by the RSL3 treatment decreased as the treatment concentrations of Example Compound 29 and Fer-1 increased, and the ROS significantly decreased at 0.01, 0.1, and 1 µM. Accordingly, it was confirmed that the mito-ROS increased by the RSL3 treatment was effectively inhibited by the treatment of Example Compound 29.

### Experimental Example 8: Inhibitory effect on intracellular accumulation of ferrous ions (Fe²⁺) in lung epithelial cells

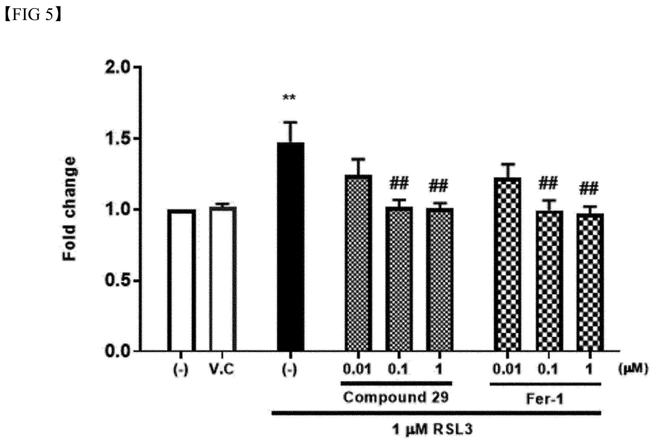

To confirm the inhibitory effect on intracellular accumulation of ferrous ions in lung epithelial cells, MLE-12 cells were seeded at 2 X 10⁵ cells/well in a 12-well plate and cultured for 18 to 24 hours. Example Compound 29 and Fer-1 were diluted to have the final concentration of 0.01, 0.1, 1, and 10 µM and added to each well, and the cells were cultured for 10 minutes. Afterward, RSL3 was treated to have the final concentration of 1 µM, and the cells were cultured for 8 hours. To confirm the inhibitory effect on intracellular accumulation of ferrous ions, FerroOrange (DOJINDO; F374) was used. According to the manufacturer's protocol, FerroOrange was diluted in the culture broth to have the final concentration of 1 µM and added, and fluorescence staining was performed after a 30-minute reaction. The MLE-12 cells that were fluorescence-stained with FerroOrange were detached from the 12-well plate using 0.25% trypsin-EDTA and transferred to a microtube. Fluorescence (Ex/Em = 580 nm/543 nm) was measured using BD FACSLyric^{™}, and the relative change compared to the control (RSL3-untreated group) was calculated. The results are shown in FIG. 5.

FIG. 5 shows the intracellular accumulation of ferrous ions at various concentrations of Example Compound 29 and Fer-1 for the ferroptosis-inducing substance RSL3. When MLE-12 cells were treated with 1 µM RSL3, intracellular ferrous ions were significantly increased compared to the control (RSL3-untreated group). The intracellular ferrous ions increased by the RSL3 treatment decreased as the treatment concentrations of Example Compound 29 and Fer-1 increased, and the intracellular ferrous ions significantly decreased at 0.1 and 1 µM. Accordingly, it was confirmed that the intracellular accumulation of ferrous cells increased by the RSL3 treatment was effectively inhibited by the treatment of Example Compound 29.

### Experimental Example 9: Inhibitory effect on mitochondrial accumulation of ferrous ions in lung epithelial cells

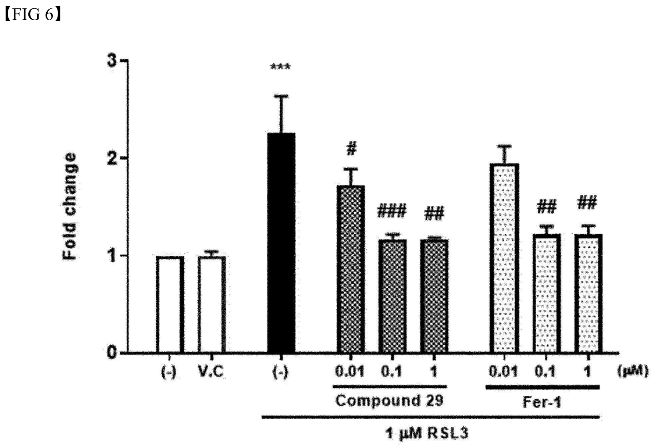

To confirm the inhibitory effect on the mitochondrial accumulation of ferrous ions in lung epithelial cells, MLE-12 cells were seeded at 2 X 10⁵ cells/well in a 12-well plate and cultured for 18 to 24 hours. Example Compound 29 and Fer-1 were diluted to have the final concentration of 0.01, 0.1, and 1 µM and added to each well, and the cells were cultured for 10 minutes. Afterward, RSL3 was treated to have the final concentration of 1 µM, and the cells were cultured for 8 hours. To confirm the inhibitory effect on the mitochondrial accumulation of ferrous ions, Mito-FerroGreen (DOJINDO; M489) was used. According to the manufacturer's protocol, the Mito-FerroGreen was diluted in the culture broth to have the final concentration of 1 µM, and fluorescence staining was performed after a 30-minute reaction. The MLE-12 cells that were fluorescence-stained with Mito-FerroGreen were detached from the 12-well plate using 0.25% trypsin-EDTA and transferred to a microtube. Fluorescence (Ex/Em = 505 nm/535 nm) was measured using, BD FACSLyric^{™}, and the relative change compared to the control was calculated. The results are shown in FIG. 6.

FIG. 6 shows the mitochondrial accumulation of ferrous ions at various concentrations of Example Compound 29 and Fer-1 for the ferroptosis-inducing substance RSL3. When the MLE-12 cells were treated with 1 µM RSL3, mitochondrial ferrous ions significantly increased compared to the control (RSL3-untreated group). The mitochondrial ferrous ions increased by the RSL3 treatment decreased as the treatment concentrations of Example Compound 29 and Fer-1 increased, and Example Compound 29 significantly reduced mitochondrial ferrous ion levels at 0.01, 0.1, and 1 µM, and Fer significantly reduced mitochondrial ferrous ion levels at 0.1 and 1 µM. Accordingly, it was confirmed that the mitochondrial accumulation of ferrous ions that were increased by the RSL3 treatment was significantly inhibited by the treatment of Example Compound 29.

### Experimental Example 10: Pharmacokinetic comparison between Example Compound 27 and comparative example compound in mice

### Pharmacokinetic measurement of Example Compound 27 2-((2-phenyl-7-((tetrahydro-2H-pyran-4-yl)amino)-1H-indol-5-yl)methoxy)ethan-1-ol

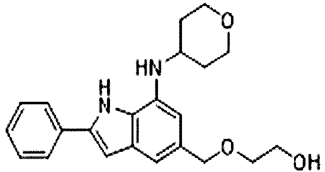

Pharmacokinetic parameters may include the maximum plasma concentration (Cₘₐₓ), the time to reach the maximum plasma concentration (Tₘₐₓ), the area under the plasma concentration-time curve (AUC_{inf}), and a half-life (t_{1/2}).

Example Compound 27 was orally administered to ICR mice at a dose of 10 mg/kg. Drug concentrations in the plasma of mice were measured by HPLC-MS/MS spectroscopy 0.5, 1, 2, 4, 8, or 24 hours after administration. The maximum plasma concentration (Cₘₐₓ) was 4,441 ng/mL, the time to the maximum plasma concentration (Tₘₐₓ) was 1.0 hour, the half-life in plasma (T_{1/2}) was 2.83 hours, and the area under the plasma concentration-time curve (AUC_{inf}) was 25,115 ng/mL.

### Pharmacokinetic measurement of comparative example compound [7-(cyclopentylamino)-2-phenyl-1H-indol-5-yl]methanol

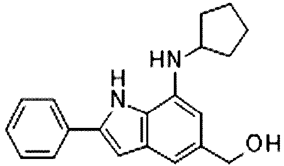

The comparative example compound [7-(cyclopentylamino)-2-phenyl-1H-indol-5-yl]methanol was synthesized according to Korean Patent No. 10-1511771 and used.

The comparative example compound was orally administered to ICR mice at a dose of 10 mg/kg. The drug concentrations in the plasma of the mice were measured by HPLC-MS/MS 0.5, 1, 2, 4, 8, or 24 hours after administration. For the comparative example compound, the maximum plasma concentration (Cₘₐₓ) was 2,113 ng/mL, the time to reach the maximum plasma concentration (Tₘₐₓ) was 1.0 hour, the half-life in plasma (T_{1/2}) was 1.4 hours, and the area under the plasma concentration- time curve (AUC_{inf}) was 5,459 ng/mL.

The maximum plasma concentration, the time to reach the maximum plasma concentration, the half-life in plasma, and the average concentration in plasma over time for oral administration of Example Compound 27 and the comparative example compound are shown in Table 8 below.

**Table 8: Comparison in plasma concentration between Example Compound 27 and comparative example compound**

| [Table 8] | | |
|---|---|---|
| **Test article** | **Example 27** | **Comparative Example** |
| **Dose (mg/kg)** | 10 | 10 |
| **Time (hr)** | plasma concentration (ng/mL) | |
| **0.5** | 3,828 | 1,642 |
| **1** | 4,441 | 2,113 |
| **2** | 3,781 | 1,122 |
| **4** | 2,801 | 389 |
| **8** | 1,075 | 51 |
| **24** | 1 | NA |
| **AUC_{inf} (ng.hr/mL)** | 25,115 | 5,459 |
| **Cmax (ng/mL)** | 4,441 | 2,113 |
| **Tₘₐₓ (hr)** | 1.0 | 1.0 |
| **T_{1/2} (hr)** | 2.83 | 1.4 |

| | | |
|---|---|---|
| NA: not available | | |

### Analysis of pharmacokinetic results between Example Compound 27 and comparative example compound

As can be seen in Table 8, based on the area under the plasma concentration-time curve (AUC_{inf}), Example Compound 27 and the comparative example compound were measured to be 5,459 ng/mL, compared to 25,115 ng/mL, indicating that Example Compound 27 exhibited an exposure approximately 5 times higher than the drug concentration in plasma, and the maximum plasma concentration (Cₘₐₓ) of Example Compound 27 was 4,441 ng/mL, exhibiting an exposure approximately 2 times higher than the comparative example compound (2,113 ng/mL). In addition, Example Compound 27 exhibited a half-life approximately 2 times longer than the comparative example compound.

Therefore, the compound of the present invention exhibited higher plasma exposure than the compound of the comparative example, indicating improved pharmacokinetic results.

## Claims

1. A compound of Chemical Formula 1 below, and an isomer thereof, a solvate thereof, a hydrate thereof or pharmaceutically acceptable salt thereof: 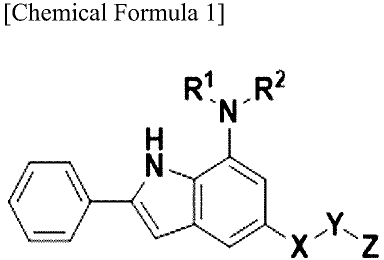 In Chemical Formula 1,
R¹ and R² are each independently hydrogen, -(CH₂)ₘ-C₃₋₁₀ cycloalkyl, -(CH₂)ₘ-C₃₋₁₀ heterocycloalkyl, -(CH₂)ₘ-aryl with 5 and 10 atoms, or -(CH₂)ₘ-heteroaryl with 5 to 10 atoms, wherein m is an integer from 0 to 4,
X is a direct bond, or -C(=O)-NX¹-, -C₁₋₈ alkylene-, C₁₋₈ alkylene-NX¹-, or -C₁₋₈ alkylene-O-,
Y is -(CH₂)ₙ-, -C₁₋₈ alkylene-O-, -C₁₋₈ alkylene-NX¹-, -C₁₋₈ alkynylene-, -C(=O)-, or - S(=O)₂-, wherein n is an integer from 0 to 4,
X¹ is hydrogen or -C₁₋₈ alkyl,
Z is -C₁₋₈ haloalkyl, -C₁₋₈ alkyl, halogen, -CN, -NR³R⁴, -OH, -S(=O)₂-C₁₋₈ alkyl, -C₃₋₁₀ cycloalkyl, or -C₃₋₁₀ heterocycloalkyl, wherein the -C₁₋₈ alkyl, -C₃₋₁₀ cycloalkyl, or -C₃₋₁₀ heterocycloalkyl of Z is substituted with R³ and/or R⁴ or unsubstituted, and
R³ and R⁴ are each independently hydrogen, -OH, -(=O), -C₁₋₈ alkyl, -C₃₋₁₀ cycloalkyl, - C₁₋₈ alkoxy, or -aryl with 5 to 10 atoms, and
provided that a compound of Chemical Formula 1 in which -X-Y-Z is -C₁₋₈ alkylene-OH, -C₁₋₈ alkylene-O-C₁₋₈ alkyl, -C₁₋₈ alkylene-O-C₁₋₈ alkylene-O-C₁₋₈ alkyl, or -C₁₋₈ alkylene-O-C₁₋₈ alkylene-O-C₁₋₈ alkylene-O-C₁₋₈ alkyl is excluded.

2. The compound of claim 1, wherein any one of R¹ and R² is hydrogen, and the other is - (CH₂)ₘ-C₃₋₁₀ cycloalkyl, -(CH₂)ₘ-C₃₋₁₀ heterocycloalkyl, -(CH₂)ₘ-aryl with 5 to 10 atoms, or - (CH₂)ₘ-heteroaryl with 5 to 10 atoms, in which m is an integer from 0 to 4.

3. The compound of any one of claims 1 and 2, wherein Y is -(CH₂)ₙ-, -C₁₋₈ alkylene-NX¹- or -C₁₋₈ alkylene-O-, in which n is an integer from 0 to 4,
X¹ is hydrogen or -C₁₋₈ alkyl,
Z is -C₁₋₈ haloalkyl, -C₁₋₈ alkyl, -CN, -OH, -C₃₋₁₀ cycloalkyl, or -C₃₋₁₀ heterocycloalkyl, in which the -C₁₋₈ alkyl, -C₃₋₁₀ cycloalkyl or -C₃₋₁₀ heterocycloalkyl of Z is substituted with R³ and/or R⁴ or unsubstituted, and
R³ and R⁴ are each independently hydrogen, -OH, -(=O), -C₁₋₈ alkyl, -C₁₋₈ alkoxy, or -C₃₋₁₀ cycloalkyl.

4. The compound of any one of claims 1 and 2, wherein Y is -(CH₂)ₙ-, -C₁₋₈ alkylene-O- or -C₁₋₈ alkynylene-, in which n is an integer from 0 to 4,
Z is -C₁₋₈ alkyl, -OH or -C₃₋₁₀ cycloalkyl, in which the -C₁₋₈ alkyl or -C₃₋₁₀ cycloalkyl of Z is substituted with R³ or unsubstituted, and
R³ is hydrogen, -OH or C₁₋₈ alkyl.

5. The compound of any one of claims 1 and 2, wherein Y is -C(=O)- or -S(=O)₂-,
Z is -NR³R⁴, and
R³ and R⁴ are each independently hydrogen, -C₁₋₈ alkyl, -C₃₋₁₀ cycloalkyl, or aryl with 5 to 10 atoms.

6. The compound of any one of claims 1, 2, and 5, wherein Y is -C(=O)-,
Z is -NR³R⁴, and
R³ and R⁴ are each independently hydrogen, -C₁₋₈ alkyl, -C₃₋₁₀ cycloalkyl, or an aryl with 5 to 10 atoms.

7. The compound of any one of claims 1 and 2, wherein Y is -(CH₂)ₙ- or -C₁₋₈ alkylene-O-, in which n is an integer from 0 to 4,
Z is -halogen, -CN, -S(=O)₂-C₁₋₈ alkyl, -C₁₋₈ alkyl, -C₃₋₁₀ cycloalkyl, or -C₃₋₁₀ heterocycloalkyl, in which the -C₁₋₈ alkyl, -C₃₋₁₀ cycloalkyl, or C₃₋₁₀ heterocycloalkyl of Z is substituted with R³ or unsubstituted, and
R³ is hydrogen, -OH, -(=O), -C₁₋₈ alkyl, or -C₃₋₁₀ cycloalkyl.

8. The compound of any one of claims 1, 2, and 7, wherein Z is -S(=O)₂-C₁₋₈ alkyl, or C₃₋₁₀ heterocycloalkyl, in which the C₃₋₁₀ heterocycloalkyl of Z is substituted with R³ or unsubstituted, and
R³ is hydrogen, -(=O), or -C₁₋₈ alkyl.

9. The compound of any one of claims 1 to 8, wherein, among compounds of Chemical Formula 1,
a compound in which X is -C₁₋₈ alkylene-, Y is -C(=O)-, and Z is -OH or -C₃₋₁₀ heterocycloalkyl, and
a compound in which X is a direct bond or -C₁₋₈ alkylene-, Y is -(CH₂)ₙ-, n is an integer from 1 to 4, and Z is substituted with -(=O) or unsubstituted -C₃₋₁₀ heterocycloalkyl are excluded.

10. The compound of claim 1, wherein the compound of Chemical Formula 1 is any one selected from the group consisting of the following compounds:
<1>7-(cyclopentylamino)-N,N-diethyl-2-phenyl-1H-indol-5-carboxamide; <2>N,N-diethyl-2-phenyl-7-((tetrahydro-2H-pyran-4-yl)amino)-1H-indol-5-carboxamide; <3>7-(benzylamino)-N,N-diethyl-2-phenyl-1H-indol-5-carboxamide; <4>N,N-diethyl-2-phenyl-7-((pyridin-4-ylmethyl)amino)-1H-indol-5-carboxamide; <5>7-(cyclopentylamino)-N-cyclopropyl-2-phenyl-1H-indol-5-carboxamide; <6>N-cyclopropyl-2-phenyl-7-((tetrahydro-2H-pyran-4-yl)amino)-1H-indol-5-carboxamide; <7>7-(benzylamino)-N-cyclopropyl-2-phenyl-1H-indol-5-carboxamide; <8>N-cyclopropyl-2-phenyl-7-((pyridin-4-ylmethyl)amino)-1H-indol-5-carboxamide; <9>N,2-diphenyl-7-((tetrahydro-2H-pyran-4-yl)amino)-1H-indol-5-carboxamide; <10>7-(benzylamino)-N,2-diphenyl-1H-indol-5-carboxamide; <11>2-(7-(cyclopentylamino)-2-phenyl-1H-indol-5-yl)-N,N-diethylacetamide; <12>N,N-diethyl-2-(2-phenyl-7-((tetrahydro-2H-pyran-4-yl)amino)-1H-indol-5-yl)acetamide; <13>N,N-diethyl-2-(2-phenyl-7-((pyridin-4-ylmethyl)amino)-1H-indol-5-yl)acetamide; <14>2-(7-(benzylamino)-2-phenyl-1H-indol-5-yl)-N,N-diethylacetamide; <15>N-methyl-2-(2-phenyl-7-((tetrahydro-2H-pyran-4-yl)amino)-1H-indol-5-yl)acetamide; <16>2-(7-(cyclopentylamino)-2-phenyl-1H-indol-5-yl)-N-methylacetamide; <17>2-(7-(benzylamino)-2-phenyl-1H-indol-5-yl)-N-cyclopropylacetamide; <18>2-(7-(benzylamino)-2-phenyl-1H-indol-5-yl)-N-phenylacetamide; <19>N-phenyl-2-(2-phenyl-7-((tetrahydro-2H-pyran-4-yl)amino)-1H-indol-5-yl)acetamide; <20>N-ethyl-2-phenyl-7-((tetrahydro-2H-pyran-4-yl)amino)-1H-indol-5-sulfonamide; <21>7-(benzylamino)-N-ethyl-2-phenyl-1H-indol-5-sulfonamide; <22>7-(cyclopentylamino)-N-ethyl-2-phenyl-1H-indol-5-sulfonamide; <23>2-(2-phenyl-7-((tetrahydro-2H-pyran-4-yl)amino)-1H-indol-5-yl)acetonitrile; <24>2-methyl-4-(2-phenyl-7-((tetrahydro-2H-pyran-4-yl)amino)-1H-indol-5-yl)but-3-yn-2-ol; <25>4-(7-cyclopentylamino)-2-phenyl-1H-indol-5-yl)-2-methylbut-3-yn-2-ol; <26>2-((7-(cyclopentylamino)-2-phenyl-1H-indol-5-yl)methoxy)ethan-1-ol; <27>2-((2-phenyl-7-((tetrahydro-2H-pyran-4-yl)amino)-1H-indol-5-yl)methoxy)ethan-1-ol; <28>2-(3-(2-phenyl-7-((tetrahydro-2H-pyran-4-yl)amino)-1H-indol-5-yl)propoxy)ethan-1-ol; <29>2-(2-((2-phenyl-7-((tetrahydro-2H-pyran-4-yl)amino)-1H-indol-5-yl)methoxy)ethoxy)ethan-1-ol; <30>2-methyl-1-((2-phenyl-7-((tetrahydro-2H-pyran-4-yl)amino)-1H-indol-5-yl)methoxy)propan-2-ol; <31>2-methyl-1-(2-((2-phenyl-7-((tetrahydro-2H-pyran-4-yl)amino)-1H-indol-5-yl)methoxy)ethoxy)propan-2-ol; <32>3-((2-((2-phenyl-7-((tetrahydro-2H-pyran-4-yl)amino)-1H-indol-5-yl)methoxy)ethoxy)methyl)pentan-3-ol; <33>2-(2-(2-phenyl-7-((tetrahydro-2H-pyran-4-yl)amino)-1H-indol-5-yl)ethoxy)ethan-1-ol; <34>3-(2-((2-phenyl-7-((tetrahydro-2H-pyran-4-yl)amino)-1H-indol-5-yl)methoxy)ethoxy)propan-1-ol; <35>4-((2-phenyl-7-((tetrahydro-2H-pyran-4-yl)amino)-1H-indol-5-yl)methoxy)cyclohexan-1-ol; <36>1-methyl-4-((2-phenyl-7-((tetrahydro-2H-pyran-4-yl)amino)-1H-indol-5-yl)methoxy)cyclohexan-1-ol; <37>2-phenyl-N-(tetrahydro-2H-pyran-4-yl)-5-(((tetrahydro-2H-pyran-4-yl)oxy)methyl)-1H-indol-7-amine; <38>4-(2-((2-phenyl-7-((tetrahydro-2H-pyran-4-yl)amino)-1H-indol-5-yl)methoxy)ethoxy)cyclohexan-1-ol; <39>5-((2-fluoroethoxy)methyl)-2-phenyl-N-(tetrahydro-2H-pyran-4-yl)-1H-indol-7-amine; <40>5-((2,2-difluoroethoxy)methyl)-2-phenyl-N-(tetrahydro-2H-pyran-4-yl)-1H-indol-7-amine; <41>N-cyclopentyl-5-((2-morpholinoethoxy)methyl)-2-phenyl-1H-indo1-7-amine;<42>5-((2-morpholinoethoxy)methyl)-2-phenyl-N-(tetrahydro-2H-pyran-4-yl)-1H-indol-7-amine; <43>4-(2-((7-(cyclopentylamino)-2-phenyl-1H-indol-5-yl)methoxy)ethyl)thiomorpholine 1,1-dioxide; <44>N-cyclopentyl-5-((2-(methylsulfonyl)ethoxy)methyl)-2-phenyl-1H-indol-7-amine; <45>5-((2-(methylsulfonyl)ethoxy)methyl)-2-phenyl-N-(tetrahydro-2H-pyran-4-yl)-1H-indo1-7-amine; <46>2-phenyl-N-(tetrahydro-2H-pyran-4-yl)-5-((2-thiomorpholinoethoxy)methyl)-1H-indol-7-amine; <47>4-(2-((2-phenyl-7-((tetrahydro-2H-pyran-4-yl)amino)-1H-indol-5-yl)methoxy)ethyl)thiomorpholine 1,1-dioxide; <48>2-((2-((2-phenyl-7-((tetrahydro-2H-pyran-4-yl)amino)-1H-indol-5-yl)methoxy)ethyl)amino)ethan-1-ol; <49>N-(2-methoxyethyl)-2-phenyl-7-((tetrahydro-2H-pyran-4-yl)amino)-1H-indol-5-carboxamide; <50>5-(((2-methoxyethyl)amino)methyl)-2-phenyl-N-(tetrahydro-2H-pyran-4-yl)-1H-indo1-7-amine; <51>N-(2-methoxyethyl)-N-methyl-2-phenyl-7-((tetrahydro-2H-pyran-4-yl)amino)-1H-indol-5-carboxamide; <52>N-(2-(2-methoxyethoxy)ethyl)-N-methyl-2-phenyl-7-((tetrahydro-2H-pyran-4-yl)amino)-1H-indol-5-carboxamide; <53>2-(methyl(2-((2-phenyl-7-((tetrahydro-2H-pyran-4-yl)amino)-1H-indol-5-yl)methoxy)ethyl)amino)ethan-1-ol; <54>N-(2-(2-hydroxyethoxy)ethyl)-N-methyl-2-phenyl-7-((tetrahydro-2H-pyran-4-yl)amino)-1H-indol-5-carboxamide; <55>5-(((2-methoxyethyl)(methyl)amino)methyl)-2-phenyl-N-(tetrahydro-2H-pyran-4-yl)-1H-indol-7-amine; <56>N-(2-(2-methoxyethoxy)ethyl)-2-phenyl-7-((tetrahydro-2H-pyran-4-yl)amino)-1H-indol-5-carboxamide; <57>5-(((2-(2-methoxyethoxy)ethyl)amino)methyl)-2-phenyl-N-(tetrahydro-2H-pyran-4-yl)-1H-indol-7-amine; <58>5-(((2-(2-methoxyethoxy)ethyl)(methyl)amino)methyl)-2-phenyl-N-(tetrahydro-2H-pyran-4-yl)-1H-indol-7-amine; <59>N-(2-((2-phenyl-7-((tetrahydro-2H-pyran-4-yl)amino)-1H-indol-5-yl)methoxy)ethyl)methanesulfonamide; <60>5-((2-((2-methoxyethyl)amino)ethoxy)methyl)-2-phenyl-N-(tetrahydro-2H-pyran-4-yl)-1H-indo1-7-amine, and <61>5-((2-((2-methoxyethyl)(methyl)amino)ethoxy)methyl)-2-phenyl-N-(tetrahydro-2H-pyran-4-yl)-1H-indol-7-amine.

11. A pharmaceutical composition for preventing or treating a necrotic cell death- or ferroptosis-related disease, comprising:
the compound of Chemical Formula 1 of claim 1, an isomer thereof, a solvate thereof, a hydrate thereof or pharmaceutically acceptable salt thereof, as an active ingredient; and
a pharmaceutically acceptable carrier.

12. The pharmaceutical composition of claim 11, wherein the necrotic cell death- or ferroptosis-related disease is one or more selected from the group consisting of the following diseases:
an acute or chronic liver disease, such as hepatitis, fibrosis, or cirrhosis;
a degenerative nerve disease such as dementia including Alzheimer's disease and vascular dementia, Parkinson's disease, epilepsy, dementia with Lewy bodies, or Huntington's disease;
an ischemic disease such as ischemic heart disease, reperfusion injury, ischemic stroke, or ischemic injury;
pancreatitis, bacterial or viral sepsis, diabetes or diabetic complications, or a diabetic vascular disease;
necrotizing proctitis, cystic fibrosis, rheumatoid arthritis, degenerative arthritis, nephrotic syndrome, bacterial infections, viral infections such as SARS-CoV, multiple sclerosis, leukemia, lymphoma, neonatal respiratory distress syndrome, asphyxiation, tuberculosis, endometriosis, angiasthenia, frostbite, steroid injection course/complications, vibrio sepsis, tenderness, hemoglobinuria, burns, hyperthermia, celiac disease, compartment syndrome, spinal cord injury, glomerulonephritis, renal failure, metabolic genetic disorders, mycoplasma infections, anthrax, Fabry disease, congenital mitochondrial diseases, phenylketonuria, placental infarction, syphilis, and aseptic necrosis;
alcohol addiction and cocaine addiction;
necrosis associated with exposure to drugs such as antibiotics, anticancer agents, doxorubicin, puromycin, bleomycin, non-steroidal anti-inflammatory drugs (NSAIDs), or cyclosporine, chemical toxins such as carbon tetrachloride, cyanide, methanol, or ethylene glycol, toxic gas, pesticides, or heavy metals such as lead, mercury, or cadmium, or administration thereof, or self-administration;
damage caused by radiation or UV exposure and necrotic cell death associated therewith; acute or chronic kidney disease, traumatic brain injury, necrotizing enterocolitis, or a skin disease such as psoriasis or allergic dermatitis;
organ preservation or organ transplantation;
inflammatory pulmonary diseases such as acute respiratory distress syndrome, acute pulmonary disease, pneumonia, tuberculosis, asthma, chronic obstructive pulmonary disease (COPD), idiopathic pulmonary fibrosis (IPF), and cystic fibrosis;
demyelinating diseases, such as demyelination and amyotrophic lateral sclerosis (ALS); hypertension such as pulmonary hypertension; mental disorders such as stroke, prion disease, epilepsy, ataxia, migraine, memory and cognitive decline, seizures, tremors, or depression;
spinocerebellar degeneration such as Friedreich's ataxia;
insulin resistance, and dyslipidemia such as hyperlipidemia;
atherosclerosis, and inflammatory bowel disease (IBD) such as Crohn's disease and ulcerative colitis;
various types of cancer and metastasis of cancer;
visual impairment-related diseases such as macular degeneration, retinitis pigmentosa, cataracts, and glaucoma;
anemia, cholestasis, hypoparathyroidism, pancytopenia, pancreatic disorder, lactic acidosis, lacticemia, hearing loss, short stature, intestinal obstruction, cardiac conduction defects such as arrhythmia, cardiomyopathy, myocardial infarction, ischemia-reperfusion heart injury, heart failure, endometriosis, infertility, subfertility, and early menopause;
muscular atrophy such as limb-girdle muscular dystrophy (LGMD), Becker muscular dystrophy (BMD), and Duchenne muscular dystrophy (DMD);
aging and aging-related diseases;
neuropathic pain; and
mucositis, such as oral mucositis and gastrointestinal mucositis.

13. The pharmaceutical composition of claim 11, wherein the disease is a degenerative neurological disease, liver disease, kidney disease, stroke, myocardial infarction, ocular disease, or lung disease.

14. The pharmaceutical composition of claim 13, wherein the degenerative neurological disease is one or more selected from the group consisting of Alzheimer's disease, Parkinson's disease, epilepsy, Huntington's disease, amyotrophic lateral sclerosis, Friedreich's ataxia, multiple sclerosis, Charcot-Marie-Tooth (CMT) disease, dementia with Lewy bodies, and traumatic brain injury.

15. A method of preventing or treating a necrotic cell death- or ferroptosis-related disease, comprising:
administering the compound of Chemical Formula 1 of claim 1, an isomer thereof, a hydrate thereof, a solvate thereof, or a pharmaceutically acceptable salt thereof, to a subject in need thereof at a pharmaceutically effective amount.

16. The method of claim 15, wherein the disease involves lipid peroxidation.

17. A method of inhibiting ferroptosis, comprising:
administering the compound of Chemical Formula 1 of claim 1, an isomer thereof, a hydrate thereof, a solvate thereof, or a pharmaceutically acceptable salt thereof, to a subject in need thereof at a pharmaceutically effective amount.

18. A method of reducing reactive oxygen species (ROS) in cells or mitochondria, comprising:
contacting the compound of Chemical Formula 1 of claim 1, an isomer thereof, a hydrate thereof, a solvate thereof, or a pharmaceutically acceptable salt thereof with cells or mitochondria.
